# EUROPEAN PATENT SPECIFICATION

(11) **EP 4 271 702 B1**
(45) Date of publication and mention of the grant of the patent: **06.11.2024**
(21) Application number: 21854871.7
(22) Date of filing: 28.12.2021
(51) Int. Cl.: C07K 14/435, A61K 47/64, C12N 15/62, A61K 39/00, A61K 47/65, A61K 49/00, C12N 5/0783, G01N 33/532, G01N 33/574

(54) **CHLOROTOXIN DERIVATIVES AND USE THEREOF**
CHLOROTOXINDERIVATE UND IHRE VERWENDUNG
DÉRIVÉS DE CHLOROTOXINE ET LEUR UTILISATION

(30) Priority: 30.12.2020 HU 2000455; 12.10.2021 HU 2100353
(43) Date of publication of application: 08.11.2023
(73) Proprietor: VRG Therapeutics Zrt., 1083 Budapest (HU)
(72) Inventor: FARKAS, Sándor, 1103 Budapest (HU); TAKÁCS, Zoltán, 2721 Pilis (HU); NACSA, János, 6720 Szeged (HU); RÁCZ, Gábor, 2045 Törökbálint (HU); HORNYÁK, Péter, 5200 Törökszentmiklós (HU); HUJBER, Zoltán, 1164 Budapest (HU); CIOCA, Daniel, Arad (RO)
(74) Representative: Kovári, Zoltán
(86) International application number: PCT/HU2021/050075
(87) International publication number: WO 2022/144560

(56) References cited:
- CN-A- 101 824 084
- PAOLA G OJEDA ET AL: "Lysine to arginine mutagenesis of chlorotoxin enhances its cellular uptake", BIOPOLYMERS, JOHN WILEY, HOBOKEN, USA, vol. 108, no. 5, 18 September 2017 (2017-09-18), pages n/a, XP071133766, ISSN: 0006-3525, DOI: 10.1002/BIP.23025

## Description

### Field of the Invention

The present invention relates to chlorotoxin derivatives. In particular, the present invention relates to novel chlorotoxin derivatives useful for targeting cancerous cells in mammals, especially in humans. The present invention relates also to methods for targeting cancerous cells using the novel chlorotoxin derivatives.

### Background of the Invention

### Chlorotoxin characteristics

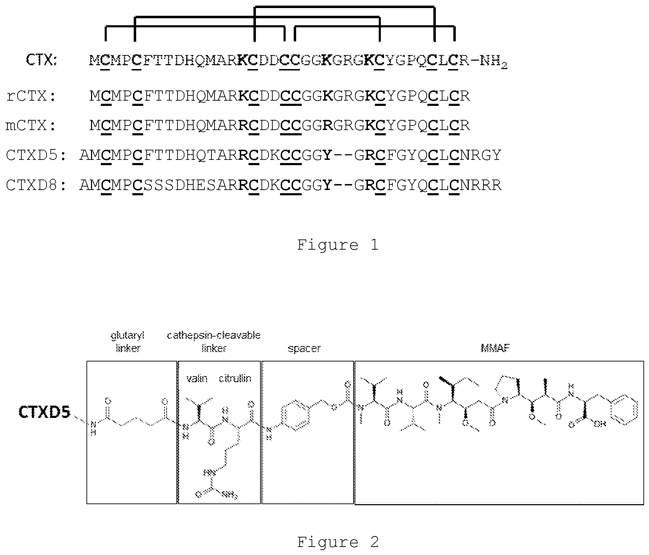

Chlorotoxin (hereinafter referred to as CTX) is a small 36 aminoacid protein, a cystine-knot miniprotein [Moore 2012], having a compact tertiary structure stabilized by four disulfide bonds. CTX was first isolated from the venom of the scorpion *Leiurus quinquestriatus* and named after its ability to block chloride channels. CTX can cause neurotoxicity in arthropods [DeBin 1993]. Under the terms chlorotoxin or CTX we refer to the wild-type protein, the sequence of which is shown under SEQ ID NO: 1. The disulfide pattern of the wild-type CTX and a few CTX variants/derivatives are shown in Figure 1. The disulfide pattern of the wild-type CTX is: C²-C¹⁹, C⁵-C²⁸, C¹⁶-C³³, and C²⁰-C35.

While studying glioma-specific chloride currents, it was discovered that CTX exhibits specific and selective binding to glioma cells *in vitro* and *in vivo* as opposed to cells of normal brain tissue [Soroceanu 1998]. This finding raised the idea of using CTX as a targeting molecule focusing the ¹³¹I iodine radioisotope-based cell killing radiotherapy to the target tumor cells [Mamelak 2006]. It was also shown by immunohistochemical investigations that CTX-like molecules with fluorescent or biotin labels can be successfully used for immunohistochemical labeling of gliomas [Soroceanu 1998]. Subsequently, tumor-specific binding by CTX was more broadly observed in a variety of diverse tumor types classified as tumors of neuroectodermal origin [Lyons 2002]. Besides gliomas comprising glioblastoma multiforme (GBM), anaplastic astrocytomas, astrocytomas and oligodendrogliomas, Lyons et al. described as CTX positive (i.e. specific binders) numerous other primary brain tumors such as gliosarcomas, gangliogliomas, meningiomas, ependymomas, as well as peripheral neuroectodermal tumors such as medulloblastomas, neuroblastomas, ganglioneuromas, melanomas (primary and metastatic), pheochromocytomas, Ewing's sarcomas, small cell lung carcinomas and Schwannomas. Lyons et al. also demonstrated high specific binding of biotinylated CTX to immortalized cell lines of different human tumor origins including seven glioblastoma cell lines and six peripheral neuroectodermal tumor cell lines. It was also shown that in living cells at physiological temperature CTX is taken up into tumor and non-tumor cells by different internalization processes leading to distinct cellular localization patterns [Wiranowska 2011]. In this study, CTX was proved to be taken up by a receptor-mediated (also called clathrine-mediated) endocytosis for processing into lysosomes at the endosomal compartment in glioma cells, which resulted in extended retaining in the cells. On the contrary, in normal cells (astrocytes and fibroblasts) CTX was taken up by non-receptor mediated macropinocytosis, which was followed by rapid release resulting in less net uptake and not extended retaining. These features may play a significant role in selective tumor targeting properties of CTX.

The original venom-derived CTX has a carboxamide at C-terminus (i.e. the native protein denoted as CTX), whereas the recombinantly produced variant of CTX (hereinafter denoted as rCTX) ends with the carboxyl group of the C-terminal arginine.

Tumor selectivity of CTX was originally attributed to binding to chloride ion channels in glioma cells. Subsequently, matrix metalloproteinase 2 (MMP-2) was identified as a receptor for CTX either alone or in a protein complex with MMP-14 (matrix metalloproteinase 14, also known as membrane-type-1 matrix metalloproteinase (MT1-MMP), tissue inhibitor of metalloproteinases 2 (TIMP-2) and αvβ3 integrin [Deshane 2003]. This MMP-TIMP-integrin complex is believed to play a critical role in regulation (maturation, anchoring, activation and inhibition) of cell surface bound and released active MMP-2, which factors in concert may promote tumor invasion, metastasis and angiogenesis [Yosef 2018] .

Nevertheless, in follow up studies a direct and specific interaction between CTX and recombinantly produced MMP-2 could not be established by a pull-down assay [Veiseh 2007]. Prompted by this ambiguity another research group reinvestigated the identity of the glioma CTX receptor using affinity columns, cross-linking reagents and mass spectrometry, and concluded that the direct binding partner protein comprising the CTX receptor is probably annexin 2 (Anx2) [Kesavan 2010]; although they admitted the possibility of other binding partner proteins and did not prove the CTX-Anx2 protein-protein interaction directly with recombinantly produced and purified Anx2 protein.

Yet another research group, identified Neuropilin-1 (NRP1), an endocytic receptor on tumor and endothelial cells, as a novel CTX target [McGonigle 2019]. They have found that only rCTX binds NRP1, the native (i.e. the one with the carboxamide C-terminal) CTX does not bind NRP1 but is metabolized to the carboxyl variant by deamidation in cellular environment.

Thus, in view of controversial proposals, the real target protein(s) of CTX have remained elusive. The target protein domains or sites that specifically bind CTX are often termed as CTX receptors [e.g. Soroceanu 1998; Dardevet 2015] and for the sake of simplicity herein we will also use this terminology.

### CTX-containing constructs and CTX variants in preclinical and clinical trials

Compared to the specific antibody targeting molecules, the small protein CTX can cross the brain blood barrier. This makes CTX a promising starting point to create new targeting molecules.

Some early preclinical studies reported effectiveness of synthetically produced CTX (TM-601) on glioblastoma xenograft tumor growth in mice *in vivo,* according to some patent applications either alone or in combination with temozolomide [US 20100215575 A1 and US 20100210546]. However, these findings were apparently not confirmed later either by scientific publications or by follow-up clinical studies, and the interest turned rather toward utilization of CTX as a targeting molecule.

Despite the lack of clarity on true target of CTX, this molecule has attracted much interest in the field of cancer research for utilizations either as diagnostic or therapeutic tools, or combinations of such utilizations as theranostics. The multiple utilizations reviewed by [Dardevet 2015]) are enabled by that the amino acid sequence of CTX provides several opportunities for useful chemical modifications. Such modifications may include conjugation with a number of compounds, or attachment of further peptide sequences as linkers for conjugation or cyclization, which modifications permit using CTX as a targeting carrier molecule. Targeting utilizations are enabled by the apparent features that such modifications often do not significantly alter the target recognizing (CTX receptor binding) properties of the modified CTX compound. For example, CTX contains a single tyrosine residue at position 29 that can be used for iodination [Soroceanu 1998]; and certain radioactive isotopes of iodine (¹²³I, ¹²⁵I and ¹³¹I) are routinely used either in various preclinical research (e.g. ¹²⁵I in binding studies) or clinical imaging (e.g. gamma-scan or single photon emission computed tomography - SPECT or SPECT/CT) or radiotherapy applications as attached to targeting molecules. Clinical Phase 1/2 studies were initiated with ¹³¹I labeled CTX (in the related publication denoted as TM-601) both with topical (intracavity tumor site) therapeutic administrations and intravenous imaging (low dose) administrations. Phase 1 results of a therapeutic trial were published as showing promising signals for efficacy and safety [Mamelak 2006]; however, the approved Phase 2 therapeutic trial and the diagnostic development was halted without publishing any results. The Phase 1 diagnostic (imaging) study was apparently prematurely terminated [ref. no. NCT00683761 at clinicaltrials.gov].

WO 2011094671 A2 describes N-terminally conjugated polypeptides, wherein the polypeptide can be CTX or its variant. It is described here that CTX can help in targeting the conjugate towards populations of cancerous cell, and that CTX can bind specifically to MMP-2.

Lysine residues of the CTX molecule can also be used to easily perform conjugation of active substances thanks to the availability of a wide range of cross-linking reagents. CTX has three lysine residues at positions 15, 23, and 27 that have been utilized for conjugation to NHS-ester modified Cyanine 5.5 (Cy5.5) and other fluorescent molecules [Akcan 2011; WO2011142858A2]. However, for the sake of high yield and reproducible production of uniform conjugate derivatives at certain position(s) instead of mixtures of various single-site and multiple-site conjugation products some modifications of CTX were deemed reasonable due to the presence of three lysine residues in the wild-type CTX. Although, it is possible to have mixtures approved by the U.S. Food and Drug Administration (FDA) and similar regulatory agencies elsewhere, commercialization is potentially hindered as it is expensive and difficult to match the ratio of mono-, di- and tri-labeled batches. Therefore, Olson and co-workers have presented examples of re-engineering CTX. They showed that substitution of lysine 15 and 23 with either alanine or arginine eliminates mixed bioconjugate species in the resulting batch, while retaining *in situ* tumor staining activity of the mutated CTX proteins as compared to CTX [Akcan 2011; WO2011142858A2]. As a result, the lysine-arginine substitutions at positions 15 and 23 (K15R, K23R) resulted in a monolysine (K27) mutant of CTX (hereinafter referred to as mCTX; SEQ ID NO: 2) . This mCTX protein was conjugated with indocyanine green (ICG) denoted as BLZ-100 (INN name tozuleristide; CAS registry number: 1673565-40-6). It is currently under Phase 2 clinical development [ref. no. NCT03579602 at clinicaltrials.gov] for intra-operative visualisation of human tumors [Dintzis 2019; Patil 2019], i.e. as a "tumor paint". While investigating fluorescent conjugates of CTX and mCTX, it was observed that these conjugates were internalized by HeLa (cervical cancer) cells, and the cellular uptake of Cy5.5- and Alexa Fluor 488-conjugates of mCTX were 1.5- and 2-fold higher than those of similar conjugates of CTX [Ojeda 2017]. However, this difference was characteristic of the fluorophore conjugated but not necessarily of the unconjugated targeting CTX variants. However, for intra-operative visualisation ("fluorescence guided surgery") several other fluorophore-conjugated CTX variants can be used, most preferably near-infrared dyes, including for example IRDye 800 CW, DyLight 750 or VivoTag-S 750 besides the above mentioned Cy5.5, and ICG [Stroud 2011].

WO 2015042202 A1 discloses conjugates and kits comprising CTX variants. According to some embodiments, the CTX variants form conjugates with a fluorescent moiety. The conjugates described here are useful for the treatment and imaging of tumors.

A review article about CTX specific inventions was published in 2014 [Cheng 2014]. It is described here that CTX can specifically inhibit the growth and metastasis of glioma cells, and can accelerate tumor apoptosis. Several references to CTX bioconjugates are provided. It is also described here that CTX specifically binds to MMP-2 receptors.

Cyclization is an often-used method to prolong half-lives of polypeptide drug candidates in blood serum *in vitro* or *in vivo.* Akcan et al. have shown that a cyclic variant of CTX retained the ability to bind to malignant tissues [Akcan 2011]. The cyclic variant was produced by connecting the C-terminal arginine with the N-terminal methionine via a heptapeptide (GAGAAGG) linker. This modification not only prolonged half-life in plasma but also promoted mono-site conjugation. Hence, any cyclization with some extending peptide segments or with built-in extra disulfide bridges might be an obvious modification of a CTX variant parent protein for a skilled person, so that such modification would retain targeting potential of the parent protein.

Another utilization of the CTX as a targeting molecule is applying CTX instead of a specific protein recognizing antibody for chimeric antigen receptor T cell (CAR-T cell) therapy. Chimeric antigen receptors (CARs) are receptor proteins genetically engineered to give T cells the new ability to target a specific protein that is present on the surface of tumor cells. The receptors are chimeric because they combine both antigen-binding and T-cell activating functions into a single receptor. Thus, CARs are composed of an extracellular tumor recognition/targeting domain, an extracellular linker/spacer, a transmembrane domain, and intracellular T cell-activating and co-stimulatory signaling domains. The patent application No. WO 2017/066481 A1 describes CAR-T constructs, in which the extracellular tumor recognition/targeting domain is CTX, or a related toxin, or a CTX variant. Currently a CAR-T cell immunotherapy development using CTX as tumor-targeting domain is in clinical Phase 1 [NCT04214392 at clinicaltrials.gov] for the treatment of patients with MMP-2 positive glioblastoma.

Another utilization of CTX as a targeting molecule is coupling it to an antiproliferative or cytotoxic payload molecule. Various antiproliferative drugs have been attached to antibodies or smaller targeting polypeptides to achieve specificity by the targeting molecules. Initially, the attempts were directed on increasing the specificity of existing chemotherapeutic drugs, such as the vinca alkaloids and doxorubicin. However, antibody-drug conjugates (ADCs) underwent a great evolution in recent years leading to FDA approval of some and also quite a few in clinical development [Lambert 2018]. The cytotoxic compounds used in most of the ADCs in current clinical development are either derivatives of dolastatin 10 (auristatins) or maytansine which are potent antimitotic microtubule-disrupting agents, or derivatives of one of several highly cytotoxic DNA damaging agents: calicheamicin, duocarmycin, pyrrolobenzodiazepine dimers and indolinobenzodiazepine pseudodimers [Lambert 2018]. These carrier-linker-payload constructs are based on the premises that the cytotoxic compound acts on an intracellular target and the targeting molecule promotes its internalization specifically into the cells recognized by the surface presence of cell-specific target proteins. The internalization takes place via the endosome-lysosome pathway, where the linker is cleaved and/or the antibody is degraded to release the payload. Therefore, such ADC constructs have been assembled with either lysosomal enzyme cleavable or non-cleavable linkers [Lambert 2018]. The patent application No. WO 2017/136769 A1 describes conjugates of CTX and cryptophycin derivatives as effective anti-cancer therapeutic drugs. Besides auristatins (e.g. monomethyl auristatin E or F - MMAE or MMAF) and maytansine, cryptophycin is another microtubule-disrupting agent. Salient example of this patent application is a carrier-linker-payload construct of CTX conjugated at lysine 27 to an analogue of cryptophycin via a cleavable dimethyl disulfide linker. This compound possesses superior potent antitumor activity as compared to effectiveness of a similar linker-payload construct without CTX in subcutaneous mouse xenograft models of glioblastoma, pancreas cancer, prostate cancer and breast cancer.

Further proposed or developed diagnostic and therapeutic applications utilizing CTX as targeting molecule have included Platinum(IV)-CTX; conjugates for polymer dot bioconjugates; conjugated upconversion nanoprobes; Ag-In-S/ZnS quantum dots; dendrigraft poly-lysine with gadolinium magnetic nanovectors tailored for combined cancer cell targeting, imaging, and siRNA delivery; nanovector based targeted gene delivery; combined drug delivery and MRI contrast enhancement by CTX-conjugated iron oxide nanoparticles; CTX-modified doxorubicine-loaded liposomes; other nanoparticles and nanoprobes as reviewed by [Dardevet 2015; Cohen 2018].

### Deficiencies of existing CTX constructs and variants

Despite the numerous attempts, to exploit CTX as a diagnostic or therapeutic targeting agent, including some early phase clinical developments, none of these developments resulted in so far authorized introduction (marketing approval) into the medical practice. Two approaches using CTX as targeting agents (CAR-T and Tozuleristide) are apparently in active development these days. Some of the other developments appear to be obviously halted according to published information at mandatory clinical study databases, such as *clinicaltrials.gov.* However, the reasons for failure or stopping were not disclosed, leaving room only for speculations. Besides potential financial or management issues one obvious reason of failure could be the insufficient affinity or potency and/or insufficient selectivity of CTX for its utilizable receptor vs. undesirable binding or effects onto other molecular or cellular targets.

The concentration yielding fifty percent inhibitory effect (IC₅₀) observed with CTX on the invasion and migration of U251MG, D54MG and U87MG glioma cell lines is around 600 nM [Soroceanu 1999], which is in line with the EC₅₀ and IC₅₀ values measured in our MMP-2 binding and displacement experiments ranging approximately 500-700 nM (see below). Such a moderate potency with high submicromolar effective concentration levels means that it is difficult to achieve *in vivo* sufficiently effective therapeutic or labeling systemic concentrations. Nevertheless, selectivity of targeting molecules to bring more payload molecules to the tumor cells than to healthy normal cells is even more important for effective diagnostic and/or therapeutic applications of targeting molecules. Therefore, it is an obvious assumption for a person skilled in the art that that the huge unmet needs in the field of CTX-related research for diagnosis and therapy of malignant tumors might be met better with more potent and/or more selective compounds in terms of either target protein related or cellular binding and functional effects. Furthermore, different tumor cell lines of the same types of tumors exhibit wide variety of marker protein expressions. Therefore, there is a need for a wide variety of new tumor cell marker recognizing molecules with different recognition profiles, and also for coupling cell specific diagnostic tools with matching therapeutic tools connected by the common pattern recognition targeting molecule, i.e. 'theranostics'. Whereas, specific antibodies are widely used for such purposes, smaller size specific targeting molecules may have advantages over antibodies, such as better penetration properties and production cost effectiveness.

### Summary of the Invention

The idea behind the present invention is that the CTX derivatives of the present invention (hereinafter referred to as CTXDs or chlorotoxin derivatives) bind to the matrix metalloproteinase 2 (MMP-2) protein stronger than CTX. Consequently, these compounds are taken up more intensively by numerous neuroectodermal tumor cells, which overexpress MMP-2, including but not restricted to breast cancers, glioblastomas, melanomas and pancreatic cancers. Surprisingly, representative compounds of the present invention (i.e. CTXD5 and CTXD8, see Table II) conjugated with a fluorophore exhibited not only stronger cellular uptake but also higher selectivity towards the tested neuroectodermal tumor cells as compared to non-tumor cells, such as fibroblasts, astrocytes and endothelial cells. In addition, investigating binding to a panel of postulated target proteins in purified form, surprisingly it was found that CTX was binding with approximately similar intensity to another protein besides MMP-2, i.e. NRP-1, and it also binds moderately to MMP-9, TIMP-2, and the chloride channel CLC3, whereas CTXD5 and CTXD8 showed also increased target protein selectivity, exhibiting overt binding only to MMP-2 and very weakly to TIMP-2 and CLC3. Furthermore, affinity of CTXD5 and CTXD8 to their primary target protein, MMP-2, was not significantly altered by conjugation at their lysine residue with relatively large molecules, such as a chemotherapeutic cytostatic compound with designed linker structure or a fluorescent labeling fluorophore compound. These observations altogether lead to the conclusion that compounds of the present invention are useful and better than CTX or mCTX as targeting molecules either alone or as conjugated with other molecules, nano particles, CAR-T cell constructs that provide additional features enabling diagnostic, or therapeutic, or combined diagnostic and therapeutic, also called "theranostic" applications that may be obvious from the prior art related to such postulated utilizations of CTX. The proteins of present invention may be starting points of further modifications or extensions of a CTX derivative for a skilled person, such as cyclization to prolong half-life of the protein drug candidate in blood serum.

In view of the above, the present invention relates to chlorotoxin derivatives comprising the amino acid sequence of the general sequence
X₀X₁CMPCX_{S1}X_{S2}X_{S3}DHX_{S4}X_{S5}ARRCX₂X₃CCGGYGX₄CFGYQCLCX₅X₆X₇X₈
(SEQ ID NO: 43)
wherein
(i) the N-terminal X₀X₁ cluster is selected from the group consisting of AM, 0M, or 00;
(ii) the solubility X_{S1}X_{S2}X_{S3}X_{S4}X_{S5} cluster is selected from the group consisting of FTTQT, FTTES, SSSQT, SSSES, FSSQT, FSSES, or FSSQS;
(iii) the internal X₂X₃X₄ cluster is selected from the group consisting of DKR, RDK, KDR, IKY, HKW, DRK, LKQ, KKK; and
(iv) the C-terminal X₅X₆X₇X₈ cluster is selected from the group consisting of N000, R000, NR00, NRGO, NRGY, NRRR, or RRRR;
where 0 denotes a position where no amino acid is present; wherein said chlorotoxin derivative has a relative human MMP-2 binding that is at least 1.62 times higher than the wild-type chlorotoxin of SEQ ID NO: 1.

According to a preferred embodiment, the present invention relates to chlorotoxin derivatives comprising the amino acid sequence of the general sequence
X₀X₁CMPCFTTDHQTARRCX₂X₃CCGGYGX₄CFGYQCLCX₅X₆X₇X₈
(SEQ ID NO: 35)
wherein
(i) the N-terminal X₀X₁ cluster is selected from the group consisting of AM, 0M, or 00;
(ii) the internal X₂X₃X₄ cluster is selected from the group consisting of DKR, RDK, KDR, IKY, HKW, DRK, LKQ, KKK; and
(iii) the C-terminal X₅X₆X₇X₈ cluster is selected from the group consisting of N000, R000, NR00, NRGO, or NRGY;
where 0 denotes a position where no amino acid is present.

According to a preferred embodiment the internal X₂X₃X₄ cluster of the chlorotoxin derivative is DKR.

According to a more preferred embodiment the chlorotoxin derivative is selected from chlorotoxin derivatives having any of the sequences set forth from SEQ ID NO: 3 to SEQ ID NO: 18 and from SEQ ID NO: 36 to SEQ ID NO: 42. According to an even more preferred embodiment the chlorotoxin derivative has the sequence set forth in SEQ ID NO: 4 or SEQ ID NO: 42.

According to another preferred embodiment the chlorotoxin derivative is cyclic.

The present invention further relates to conjugates, where the conjugate comprises a chlorotoxin derivative of the present invention and a prosthetic group.

The prosthetic group is preferably selected from the following agents:
(i) visualisation agents, preferably selected from fluorescent labels, radiolabels, magnetic resonance imaging labels, and agents enabling indirect labeling by high affinity binding to labeling molecules;
(ii) therapeutic agents, preferably selected from chemotherapeutic agents, and biological therapeutic agents;
(iii) targeting agents, preferably selected from antibodies, polypeptides, polysaccharides, and nucleic acids;
(iv) moieties that increase the circulatory half-life, preferably selected from peg moieties, glycosyl moieties, glycosylpeg moieties and moieties enabling cyclization of the chlorotoxin derivative of any of claims 1 to 5 via a linker extension.

According to a further preferred embodiment of the present invention, the conjugate comprises one or more linker(s) and optionally one or more spacer(s) between the chlorotoxin derivative and the prosthetic group. The linker is preferably selected from the group of peptides, dimethyl disulfide linker, glutaryl linker, cathepsin-cleavable linkers.

The present invention further relates to a theranostic pair of conjugates comprising a first conjugate and a second conjugate, wherein both conjugates are conjugates of the present invention. According to a preferred embodiment of the present invention the chlorotoxin derivative part of said two conjugates have the same amino acid sequence and said two conjugates have different prosthetic groups. According to another preferred embodiment the chlorotoxin derivative part of said first conjugate and said second conjugate have different amino acid sequences and said first conjugate and said second conjugate have the same or different prosthetic groups.

The present invention further relates to a kit comprising a conjugate of the present invention and instructions for use. According to another embodiment, the kit comprises a theranostic pair of the present invention and instructions for use.

The present invention further relates to a pharmaceutical composition comprising a conjugate of the present invention, wherein the prosthetic group of the conjugate is a therapeutic agent or a visualisation agent and the pharmaceutical composition further comprises a pharmaceutically acceptable excipient.

The present invention further relates to a conjugate of the present invention which conjugate is for use in the treatment of cancer, or for use in the diagnosis of cancer. According to a preferred embodiment, the cancer is selected from the group of breast cancers, cervical cancers, colon cancers, ependymomas, Ewing's sarcomas, gangliogliomas, ganglioneuromas, gliomas, glioblastomas, gliosarcomas, medulloblastomas, meningiomas, neuroblastomas, melanomas (primary and metastatic), pancreatic cancers, pheochromocytomas, prostate cancers, Schwannomas and small cell lung carcinomas. According to a most preferred embodiment, the cancer is selected from the group of breast cancers, glioblastomas, melanomas and pancreatic cancers.

The present invention further relates to a conjugate of the present invention which conjugate is for use in the visualisation of cancerous tissues. According to a preferred embodiment, the cancerous tissue is selected from the group of breast cancers, cervical cancers, colon cancers, ependymomas, Ewing's sarcomas, gangliogliomas, ganglioneuromas, gliomas, glioblastomas, gliosarcomas, medulloblastomas, meningiomas, neuroblastomas, melanomas (primary and metastatic), pancreatic cancers, pheochromocytomas, prostate cancers, Schwannomas and small cell lung carcinomas. According to a most preferred embodiment, the cancerous tissue is selected from the group of breast cancers, glioblastomas, melanomas and pancreatic cancers.

The present invention further relates to a pharmaceutical composition according to the present invention for use in the treatment of cancer, wherein the prosthetic group of the conjugate is a therapeutic agent suitable for the treatment of the cancer. According to a preferred embodiment, the cancer is selected from the group of breast cancers, cervical cancers, colon cancers, ependymomas, Ewing's sarcomas, gangliogliomas, ganglioneuromas, gliomas, glioblastomas, gliosarcomas, medulloblastomas, meningiomas, neuroblastomas, melanomas (primary and metastatic), pancreatic cancers, pheochromocytomas, prostate cancers, Schwannomas, small cell lung carcinomas. According to a most preferred embodiment, the cancer is selected from the group of breast cancers, glioblastomas, melanomas and pancretic cancers.

The present invention further relates to a method for the preparation of conjugates, which method comprises the steps of
a) providing a chlorotoxin derivative according to the present invention;
b) optionally binding a linker group to the chlorotoxin derivative of step a);
c) binding a prosthetic group to the linker part of the compound formed in step b) or directly to the protein of step a) in the absence of linker.

The present invention further relates to a method for the visualisation of a cancerous tissue, comprising the step of contacting the tissue to be examined with a conjugate of the present invention, wherein the prosthetic group of said conjugate is a visualisation agent suitable for the visualisation of the cancerous tissue.

The present invention further relates to a nucleic acid molecule encoding a chimeric antigen receptor, wherein the chimeric antigen receptor comprises:
a) a chlorotoxin derivative according to the present invention;
b) optionally a spacer region between the chlorotoxin derivative and the transmembrane domain;
c) a transmembrane domain;
d) one or two costimulatory domains;
e) a signaling domain;
wherein the chlorotoxin derivative enables the chimeric antigen receptor, when expressed on the surface of a T cell, to direct the T cell activity to cancerous cells.

According to a preferred embodiment, the nucleic acid molecule of the present invention encodes a chimeric antigen receptor, wherein the chlorotoxin derivative is selected from chlorotoxin derivatives having any of the sequences set forth from SEQ ID NO: 3 to SEQ ID NO: 18 and from SEQ ID NO: 36 to SEQ ID NO: 42, preferably SEQ ID NO: 4 or SEQ ID NO: 42.

According to another preferred embodiment, said nucleic acid molecule of the present invention encodes a chimeric antigen receptor, wherein the
i) transmembrane domain selected from the group consisting of a CD4 transmembrane domain or variant thereof, a CD8 transmembrane domain or variant thereof, a CD28 transmembrane domain or a variant thereof, and a CD3 zeta transmembrane domain or a variant thereof;
ii) the one or two costimulatory domains selected from the group consisting of a CD28 costimulatory domain or a variant thereof, a 4-1BB costimulatory domain or a variant thereof and an OX40 costimulatory domain or a variant thereof; and
iii) the signaling domain is the CD3 zeta signaling domain or a variant thereof.

The present invention further relates to a vector comprising the nucleic acid molecule of the present invention.

The present invention further relates to a population of isolated human cells
i) transfected by an RNA or DNA vector comprising an expression cassette comprising the nucleic acid of the present invention, wherein transfection is carried out *in vivo* or *ex vivo;* or
ii) transduced by a viral vector comprising an expression cassette comprising the nucleic acid of the present invention, wherein transduction is carried out *in vivo* or ex *vivo,* wherein the viral vector is preferably a retroviral or lentiviral vector; and wherein said human cells are selected from the list consisting of autologous human T cells, autologous human CD4+ helper T cells, autologous human CD8+ cytotoxic T cells, a mixture of autologous human CD4+ helper T cells and CD8+ cytotoxic T cells in any proportion, allogeneic human T cells, allogeneic human CD4+ helper T cells, allogeneic human CD8+ cytotoxic T cells, a mixture of allogeneic human CD4+ helper T cells and CD8+ cytotoxic T cells in any proportion, autologous primary human natural killer (NK) cells, allogeneic primary human natural killer (NK) cells, allogeneic cells of an NK-92 cell line, autologous human monocytes, autologous human macrophages, allogeneic human monocytes, allogeneic human macrophages.

The present invention further relates to one or more population of isolated human cells according to of the present invention for use in the treatment of cancer.

### Brief Description of the Drawings

Figure 1 depicts the sequences of some CTX variants/derivatives showing also the disulfide pattern,
   wherein
   sequence of CTX and rCTX is set forth in SEQ ID NO: 1;
   sequence of mCTX is set forth in SEQ ID NO: 2;
   sequence of CTXD5is set forth in SEQ ID NO: 4;
   sequence of CTXD8 is set forth in SEQ ID NO: 42.
Figure 2 depicts the cleavable cytostatic conjugate of CTXD5 formed with monomethyl auristatin F (MMAF).
Figure 3 depicts the MMP-2 and NRP1 binding intensities of phage-displayed CTX and CTXDsin the Co-bead test.
Figure 4 depicts the MMP-2 binding affinities of CTXD5, CTXD8 and CTX by displacement of CTX-Cy5in the Magnabead test.
Figure 5 depicts the MMP-2 binding of CTXD5-Cy5, CTXD8-Cy5 and CTX-Cy5 in the Magnabead test.
Figure 6 depicts the binding of CTX-Cy5, CTXD5-Cy5 and CTXD8-Cy5 to postulated target proteins in Co-bead test.
Figure 7 depicts the NRP1 binding of CTXD5-Cy5 and CTX-Cy5 in the Magnabead test.
Figure 8 depicts the concentration-uptake relationships for CTXD5-Cy5 and CTX-Cy5 in U251 and Panc-1 cells.
Figure 9 depicts the viability of MDA-HER2 breast cancer-derived cells exposed to CTXD8-CAR and CTX-CAR and non-transduced (NT) control T lymphocytes at 1:1 effector to target cell ratio.
Figure 10 depicts the viability of MDA-HER2 breast cancer-derived cells exposed to CTXD8-CAR and CTX-CAR and control T lymphocytes at different effector cell numbers.
Figure 11 depicts the amino acid sequence of the CTXD8-CAR construct.

### Detailed Description of the Invention

In the context of the present description under the terms "variant", "chlorotoxin variant", or "CTX variant" those proteins are to be understood that differ from the wildtype CTX and are part of the state of the art.

In the context of the present description under the term "derivative", "chlorotoxin derivative", "CTX derivative", or "CTXD" those proteins are to be understood that were developed during the project resulting the present invention.

The term CTXD with a serial number at the end refers to a chlorotoxin derivative (protein) or the phage clone producing said chlorotoxin derivative. Each specific CTXD referred to in the present description has a certain amino acid sequence. For example, CTXD5 in the present description refers to the protein having a sequence according to SEQ ID NO: 4, CTXD1 in the present description refers to the protein having a sequence according to SEQ ID NO: 17.

The term "cancer" is used for diseases in which abnormal cells divide without control and can invade nearby tissues (this definition is based on the description at https://www.cancer.gov/publications/dictionaries/cancer-terms/def/cancer on the filing date of the present patent application). Cancer cells can also spread to other parts of the body through the blood and lymph systems. There are several main types of cancer. Carcinoma is a cancer that begins in the skin or in tissues that line or cover internal organs. Sarcoma is a cancer that begins in bone, cartilage, fat, muscle, blood vessels, or other connective or supportive tissue. Leukemia is a cancer that begins in blood-forming tissue, such as the bone marrow, and causes too many abnormal blood cells to be made. Lymphoma and multiple myeloma are cancers that begin in the cells of the immune system. Central nervous system cancers are cancers that begin in the tissues of the brain and spinal cord. Cancer is also called malignancy.

Under the term "cancerous" a state of a mammalian or human patient is understood, which state involves cancer.

The references to methods of treatment by therapy or in vivo diagnosis methods in this description are to be interpreted as references to compounds and pharmaceutical compositions of the present invention for use in those methods.

The term "theranostics" is derived from a combination of the words therapeutics and diagnostics. The term refers to unique combinations of diagnostic and therapeutic techniques, whereby coupling a specific diagnostic technique with a method or product for treatment provides guidance for identifying the appropriate treatment sensitive patient, tissue or cell population [Jeelani 2014].

The chlorotoxin derivative of the present invention comprises the following general amino acid sequence:
X₀X₁CMPCX_{S1}X_{S2}X_{S3}DHX_{S4}X_{S5}ARRCX₂X₃CCGGYGX₄CFGYQCLCX₅X₆X₇X₈
(SEQ ID NO: 43)
wherein
(i) the N-terminal X₀X₁ cluster is selected from the group consisting of AM, 0M, or 00;
(ii) the solubility X_{S1}X_{S2}X_{S3}X_{S4}X_{S5} cluster is selected from the group consisting of FTTQT, FTTES, SSSQT, SSSES, FSSQT, FSSES, or FSSQS;
(iii) the internal X₂X₃X₄ cluster is selected from the group consisting of DKR, RDK, KDR, IKY, HKW, DRK, LKQ, KKK; and
(iv) the C-terminal X₅X₆X₇X₈ cluster is selected from the group consisting of N000, R000, NR00, NRGO, NRGY, NRRR, or RRRR;
where 0 denotes a position where no amino acid is present; wherein said chlorotoxin derivative has a relative human MMP-2 binding that is at least 1.62 times higher than the wild-type chlorotoxin of SEQ ID NO: 1.

According to a preferred embodiment, the present invention relates to chlorotoxin derivatives comprising the amino acid sequence of the general sequence
X₀X₁CMPCFTTDHQTARRCX₂X₃CCGGYGX₄CFGYQCLCX₅X₆X₇X₈
(SEQ ID NO: 35)
wherein
(i) the N-terminal X₀X₁ cluster is selected from the group consisting of AM, 0M, or 00;
(ii) the internal X₂X₃X₄ cluster is selected from the group consisting of DKR, RDK, KDR, IKY, HKW, DRK, LKQ, KKK; and (iii) the C-terminal X₅X₆X₇X₈ cluster is selected from the group consisting of N000, R000, NR00, NRGO, or NRGY;
where 0 denotes a position where no amino acid is present.

It is to be noted that not all possible variations of SEQ ID NO: 35 and SEQ ID NO: 43 shown in the Sequence Listing fall within the scope of the present invention, but only those which meet the above cluster definitions.

The general sequences SEQ ID NO: 35 and SEQ ID NO: 43 have three and four variable clusters, respectively. There is an N-terminal cluster (X₀X₁ cluster) at the N-terminus, a solubility cluster (X_{S1}X_{S2}X_{S3}X_{S4}X_{S5} cluster, only in SEQ ID NO: 43), an internal cluster (X₂X₃X₄ cluster), and a C-terminal cluster (X₅X₆X₇X₈ cluster) at the C-terminus. All these clusters have a certain, limited composition of amino acids. However, it was found that the composition of the clusters is independent of each other.

The N-terminal cluster consists of two positions, namely positions X₀ and X₁. The composition of the N-terminal cluster can be AM, 0M, or 00. Here, A denotes alanine (Ala), and M denotes methionine (Met). 0 denotes a position where no amino acid is present. Accordingly, the N-terminal cluster can have an Ala-Met dimer, or only a Met monomer, or this N-terminal cluster can remain empty, i.e. with no amino acid in these positions.

The solubility cluster is the X_{S1}X_{S2}X_{S3}X_{S4}X_{S5} cluster (only in SEQ ID NO: 43) close to the N-terminus. It consists of five positions of X_{S1}, X_{S2}, X_{S3}, X_{S4} and X_{S5}., where the "S" in the indices refers to the characteristics of "solubility". It is apparent that the amino acids forming the solubility cluster are not necessarily neighbouring amino acids in the sequence, two sub-clusters are present, namely X_{S1}X_{S2}X_{S3} and X_{S4}X_{S5}. The solubility cluster can have the following compositions: FTTQT, FTTES, SSSQT, SSSES, FSSQT, FSSES, or FSSQS. For example, if the composition of the X_{S1}X_{S2}X_{S3}X_{S4}X_{S5} solubility cluster is FTTQT, it is to be understood that the amino acid in the X_{S1} position is F denoting phenylalanine (Phe), the amino acid in the X_{S2}, X_{S3} and X_{S5} positions is T denoting threonine (Thr), and the amino acid in the X_{S4} position is Q denoting glutamine (Gln). Further amino acids that can be found in this solubility cluster are E denoting glutamic acid (Glu) and S denoting serine (Ser).

The internal cluster is the X₂X₃X₄ cluster in about the middle of the amino acid sequence, consisting of three positions of X₂, X₃, and X₄. It is apparent that the amino acids forming the internal cluster are not necessarily neighbouring amino acids in the sequence. Although, amino acids at positions X₂ and X₃ are neighbouring ones, the amino acid in position X₄ is further away in the C-terminus direction by seven positions. The internal X₂X₃X₄ cluster can have the following compositions: DKR, RDK, KDR, IKY, HKW, DRK, LKQ, KKK. For example, if the composition of the internal X₂X₃X₄ cluster is said to be DKR (which is one of the preferred embodiments of the present invention), it is to be understood that the amino acid in the X₂ position is D denoting aspartic acid (Asp), the amino acid in the X₃ position is K denoting lysine (Lys), and the amino acid in the X₄ position is R denoting arginine (Arg). Further amino acids that can be found in this internal cluster are I denoting isoleucine (Ile), Y denoting tyrosine (Tyr), H denoting histidine (His), W denoting tryptophan (Trp), L denoting leucine (Leu), and Q denoting glutamine (Gln).

The C-terminal cluster consists of four positions, namely positions X₅, X₆, X₇, and X₈. The composition of the C-terminal cluster can be N000, R000, NR00, NRGO, or NRGY. Here, N denotes asparagine (Asn), R denotes arginine (Arg), G denotes glycine (Gly), and Y denotes tyrosine (Tyr). 0 denotes a position where no amino acid is present. It is apparent that this C-terminal cluster can have only one amino acid N or R (i.e. if the meaning of X₅X₆X₇X₈ is N000 or R000), in these cases the chlorotoxin derivative of the present invention ends at the C-terminus with position X₅. Furthermore, the C-terminal cluster can have two amino acids (i.e. if the meaning of X₅X₆X₇X₈ is NR00), or three amino acids (i.e. if the meaning of X₅X₆X₇X₈ is NRG0), or four amino acids (i.e. if the meaning of X₅X₆X₇X₈ is NRGY in the case of SEQ ID NO: 35, and it is NRGY, NRRR, or RRRR in the case of SEQ ID NO: 43).

According to a preferred embodiment of the present invention the chlorotoxin derivative is selected from the chlorotoxin derivatives having any of the sequences set forth from SEQ ID NO: 3 to SEQ ID NO: 18 and from SEQ ID NO: 36 to SEQ ID NO: 42. The chemical space defined by SEQ ID NO: 43 covers all chlorotoxin derivatives set forth from SEQ ID NO: 3 to SEQ ID NO: 18 and from SEQ ID NO: 36 to SEQ ID NO: 42. Apart from the Sequence Listing, the chlorotoxin derivatives corresponding to these sequences are shown in Table I and Table II.

According to a more preferred embodiment of the present invention the chlorotoxin derivative is selected from the chlorotoxin derivatives having any of the sequences set forth from SEQ ID NO: 3 to SEQ ID NO: 18. The chemical space defined by SEQ ID NO: 35 covers all chlorotoxin derivatives set forth from SEQ ID NO: 3 to SEQ ID NO: 18. Apart from the Sequence Listing, the chlorotoxin derivatives corresponding to these sequences are shown in Table I.

According to an even more preferred embodiment of the present invention, the chlorotoxin derivative has the amino acid sequence set forth in SEQ ID NO: 4 or SEQ ID NO: 42. The protein having the amino acid sequence of SEQ ID NO: 4 is referred also to as CTXD5 in the present description. The protein having the amino acid sequence of SEQ ID NO: 42 is referred also to as CTXD8 in the present description.

The chlorotoxin derivatives of the present invention can be cyclic proteins. It is well known for a person skilled in the art that a protein may become more stable if cyclized than in its linear form. As described above, cyclic variant of CTX retained the ability to bind to malignant tissues [Akcan 2011] . Therefore, it is an obvious expectation of a person skilled in the art that any chlorotoxin derivate can be cyclized and thereby increasing its half-life. Protein cyclization methods are well known for a person skilled in the art [Di 2015] . Within the scope of the present invention the term "cyclic" refers to chlorotoxin derivatives made cyclic in themselves or by inserting a linker, provided that the given cyclization does not significantly affect in a negative manner the desired effect of the chlorotoxin derivative, e.g. the affinity of the chlorotoxin derivative to the MMP-2 protein.

The chlorotoxin derivatives of the present invention can effectively recognize and bind cancerous cells. They are therefore suitable for delivering a chemical entity to said cancerous cells, which chemical entity can exert a desired function once bound by a cancerous cell. Within the scope of the present invention, under the term "conjugate" a construct is understood that comprises at least a chlorotoxin derivative and a prosthetic group. According to the present invention said chemical entity is in the form of a prosthetic group. Within the scope of the present invention, under the term "prosthetic group" any chemical entity is to be understood that is bound to the chlorotoxin derivative according to the present invention via a primary bond (i.e. covalent or ionic bond). The range of the prosthetic groups can extend from single atoms to large proteins.

The prosthetic group is preferably a visualisation agent. Namely, in a preferred embodiment of the present invention, a chlorotoxin derivative according to the present invention is forming a conjugate with a prosthetic group, where the prosthetic group is a visualisation agent. Thereby, such a conjugate can be used to visualise that part of a body (e.g. human body) which binds said chlorotoxin derivative. Visualisation agents and method for their chemical binding to certain proteins are known by a person skilled in the art. Preferred visualisation agents in the sense of the present invention are
i) fluorescent labels, like cyanine dyes (e.g. indocyanine green) ;
ii) radiolabels, like iodine isotopes of ¹²³I, ¹²⁵I and ¹³¹I;
iii) magnetic resonance imaging labels, like boron nanoparticles, boron and carbon nanoparticles, boron carbide nanoparticles, boron-containing polymer, boron and carbon containing polymer, boron carbide polymer, any of these nanoparticles or polymers further comprising gadolinium);
iv) agents enabling indirect labeling by high affinity binding to labeling molecules, like biotin, avidin, his-tag.

In case the visualisation agent is a fluorescent label, it can be preferably used for fluorescence guided surgery. In the course of this surgery the conjugate of the present invention visualises those cells or parts of a tissue which bind the conjugate via the chlorotoxin derivative moiety. Thereby these cells or parts of tissue become visible for the doctor carrying out the surgery. Preferably near-infrared dyes including but not restricted to indocyanine green, cyanine 5.5, IRDye 800 CW, DyLight 750 or VivoTag-S 750 can be used for this purpose.

Another preferred example for using the conjugate of the present invention where the prosthetic group is a fluorescent label is when the target cells are analysed in a flow cytometric method or in the course of a histological staining process. In such cases the conjugates of the present invention are used as diagnostic agents.

According to another preferred embodiment the prosthetic group is a therapeutic agent. Namely, in a preferred embodiment of the present invention, a chlorotoxin derivative according to the present invention is forming a conjugate with a prosthetic group, where the prosthetic group is a therapeutic agent. Thereby, such a conjugate can be used for therapy, where the chlorotoxin moiety of the conjugate recognizes a cancerous tissue and binds thereto. The therapeutic moiety of the conjugate becomes thereby into a close proximity with said cancerous tissue or gets internalized in its cells and exerts its therapeutic effect. Therapeutic agents and methods for their chemical binding to certain proteins are known by a person skilled in the art. Preferred therapeutic agents in the sense of the present invention are
i) chemotherapeutic agents, like auristatins, cryptophycin, calicheamicin, duocarmycin, pyrrolobenzodiazepine dimers, indolinobenzodiazepine pseudodimers, maytansine, methotrexate, docetaxel, cisplatin and etoposide;
ii) biological therapeutic agents, like cDNA, siRNA, shRNA, and RNAi.

The conjugates formed with monomethyl auristatin F (MMAF) are examples for conjugates of the present invention suitable for therapeutic purposes (for details see e.g. the "Chemical toolbox", item 3.3. below or Example 4).

According to a further preferred embodiment the prosthetic group is a targeting agent. Namely, in a preferred embodiment of the present invention, a chlorotoxin derivative according to the present invention is forming a conjugate with a prosthetic group, where the prosthetic group is another targeting agent. Thereby, such a conjugate with dual binding site on a target cell provides a targeting molecule with even higher affinity to the targeted cells or tissues. Targeting agents and method for their chemical binding to certain proteins are known by a person skilled in the art. Preferred targeting agents in the sense of the present invention are antibodies, polypeptides, polysaccharides, and nucleic acids.

According to another preferred embodiment the prosthetic group is a moiety that increases the circulatory half-life. Namely, in a preferred embodiment of the present invention, a chlorotoxin derivative according to the present invention is forming a conjugate with a prosthetic group, where the prosthetic group is a moiety that increases the circulatory half-life. Thereby, such a conjugate may mitigate the metabolism of a CTXD. Moieties that increase the circulatory half-life and method for their chemical binding to certain proteins are known by a person skilled in the art. Preferred moieties that increase the circulatory half-life in the sense of the present invention are peg moieties, glycosyl moieties, glycosylpeg moieties and moieties enabling cyclization of the chlorotoxin derivative of the present invention via a linker extension.

The chlorotoxin derivative and the prosthetic group form together a conjugate of the present invention. In some cases, a linker moiety needs to be built between the chlorotoxin derivative and the prosthetic group. For example, a linker must be used when a prosthetic group cannot be chemically bound directly to the chlorotoxin derivative. A specially cleavable linker can be used if the prosthetic group needs to be released once arrived at its target location, like e.g. the cancerous cell. Linkers are known by a person skilled in the art, examples of linkers are peptides, dimethyl disulfide linker, glutaryl linker, cathepsin-cleavable linkers. As apparent for a person skilled in the art, it is possible to use more than one linker in a certain chlorotoxin derivative - prosthetic group construct.

Despite the presence of one or more linkers, some type of prosthetic groups needs to be extended away from the chlorotoxin derivative in order to exert their effect. For this purpose, one or more spacer(s) can be applied between the chlorotoxin derivative and the prosthetic group. The spacer moiety is bound to the prosthetic group, or to the linker or to the chlorotoxin derivative via a primary bond (i.e. covalent or ionic bond).

It is apparent for a person skilled in the art, that sometimes the prosthetic group, the linker and the spacer cannot be strictly distinguished, their borderline is in some cases not exact. For example, some prosthetic groups might have a tail region that can function as a linker.

Theranostic pairs can be selected from the conjugates of the present invention. These two conjugates, i.e. a first conjugate and a second conjugate can be identical or can be different. According to a preferred embodiment of the present invention the chlorotoxin derivative part of said two conjugates have the same amino acid sequence and said two conjugates have different prosthetic groups.

The term "theranostic" refers to the combined diagnostic and therapeutic applications, as known by the person skilled in the art. Briefly, one of the members of a theranostic pair is suitable for diagnostic purposes, the other member of the theranostic pair is suitable for therapeutic purposes.

The members of a theranostic pair can be used either separately in space and/or time, or simultaneously. A theranostic pair of the present invention typically have the same chlorotoxin derivative (i.e. same amino acid sequence), thereby both conjugates bind to the same cancerous tissue. However, the typical theranostic pair of the present invention have different prosthetic groups, one for diagnostic purposes (like e.g. a visualisation agent), and another one for therapeutic purposes (like e.g. a chemotherapeutic agent). As apparent for a person skilled in the art, the same conjugates of the present invention can also form a theranostic pair, where the prosthetic group of said conjugates have both diagnostic and therapeutic features. On the other hand, the members of a theranostic pair of the present invention can have different amino acid sequence in the chlorotoxin derivative part and different prosthetic groups, as well.

The present invention also relates to kits, which comprise a conjugate of the present invention and instructions for use. The conjugate of the present invention can be provided in the kit in the form of a solution or a lyophilizate. The instructions for use describe the use of the conjugate of the kit at a level which can be understood by a person skilled in the art. The instructions for use can be in the form of paper, electronic data carrier or can be available online. The kit can further comprise solutions, buffers, reagents, disposable parts of laboratory devices.

The present invention also relates to kits, which comprise a theranostic pair of the present invention and instructions for use. The theranostic pair of the present invention can be provided in the kit in the form of a solution or a lyophilizate. The instructions for use describe the use of the theranostic pair of the kit at a level which can be understood by a person skilled in the art. The instructions for use can be in the form of paper, electronic data carrier or can be available online. The kit can further comprise solutions, buffers, reagents, disposable parts of laboratory devices.

The present invention further relates to a pharmaceutical composition that comprises a conjugate of the present invention and a pharmaceutically acceptable excipient, where the prosthetic group of said conjugate is a therapeutic agent or a visualisation agent. If the pharmaceutical composition of the present invention is to be used for therapy, the prosthetic group shall be a therapeutic agent. If the pharmaceutical composition of the present invention is to be used for visualisation, the prosthetic group shall be a visualisation agent.

Pharmaceutical compositions of the present invention can be administered via parenteral, subcutaneous, intravenous, intramuscular, intraperitoneal, transdermal or buccal routes. As the pharmaceutical composition is suitable for the treatment of tumors, a preferred way of administration is the microinfusion applied locally to the tumor to be treated. The dosage of the pharmaceutical composition to be administered will be dependent on the type of the tumor, the age, health, and weight of the patient, the kind of an optional concurrent treatment, the frequency of treatment, and the nature of the effect to be achieved. It is the skilled person who can determine the dosage in view of the above data. Typical dosage comprises 1.0 pg/kg body weight to 100 mg/kg body weight. The preferred dosages for systemic administration comprise 100.0 ng/kg body weight to 10.0 mg/kg body weight. The preferred dosages for direct administration to a site via microinfusion comprise 1 ng/kg body weight to 1 mg/kg body weight.

The pharmaceutically acceptable excipient in the pharmaceutical composition of the present invention facilitates the processing of the conjugate into preparations which can be used pharmaceutically for delivery to the site of action. Suitable formulations for parenteral administration include aqueous solutions of the conjugate in water-soluble form (e.g. water-soluble salts). In addition, suspensions of the conjugate (e.g. oily injection suspensions) may be administered. Suitable lipophilic solvents or vehicles include fatty oils (e.g. sesame oil) or synthetic fatty acid esters (e.g. ethyl oleate) or triglycerides. Aqueous injection suspensions may contain substances which increase the viscosity of the suspension include, for example, sodium carboxymethyl cellulose, sorbitol and dextran. Optionally, the suspension may also contain stabilizers. Liposomes can also be used to encapsulate the conjugate for delivery to the tumor cell.

The pharmaceutical formulation for systemic administration according to the invention may be formulated for enteral, parenteral or topical administration. Any common topical formulation such as a solution, suspension, gel, ointment or salve and the like may be employed. Preparation of such topical formulations are described in Remington's Pharmaceutical Sciences (Gennaro ed, 1995, Mack Publishing). For topical application, the pharmaceutical composition could also be administered as a powder or spray, particularly in the form of an aerosol. The pharmaceutical composition may be administered by inhalation. For inhalation therapy the conjugate may be in a solution useful for administration by metered dose inhalers or in a form suitable for a dry powder inhaler. The pharmaceutical composition is also suitable for administration by bronchial lavage.

Suitable formulations for oral administration include hard or soft gelatine capsules, pills, tablets, including coated tablets, elixirs, suspensions, syrups or inhalations and controlled release forms thereof.

The CTXD conjugates of the present invention can be used in the diagnosis of cancer, in the visualisation of cancerous tissues and in the treatment of cancer. All these uses are based on the phenomenon that the conjugates bind selectively to cancerous tissues/cells. Such cells may be those overexpressing the MMP-2 protein. Once a conjugate is bound to a cancerous cell, depending on the function of the prosthetic group of the conjugate the specific binding can be used for diagnostic purposes, visualization or the cell can be killed (i.e. treatment of cancer). The diagnostic use may help either in establishing or localizing the presence of cancerous cells/tissues within the body or on epithelial surfaces (skin or mucous membranes) or in identifying the type of cancerous tissue that might be sensitive to a specific treatment using the CTXD receptors as targeting motifs. If the conjugate has a visualisation agent as the prosthetic group, the cancerous tissue can be visualised. Visualization may help in differentiating the cancerous cells or tissues from surrounding healthy (non-cancerous) cells/tissues, for example in fluorescence guided surgery. Diagnosis, visualisation and treatment of cancerous cells and tissues by conjugates of a specific protein and an appropriate prosthetic group are well known for persons skilled in the art.

The conjugates of the present invention are useful in the diagnosis and treatment of virtually every type of malignant cancer expressing CTX binding sites. These types of cancer include gliomas, astrocytomas, oligodendrogliomas, medulloblastomas, choroid plexus carcinomas, ependymomas, meningiomas, glioblastomas, gangliomas, pheochromocytomas, and metastatic brain tumors, other brain tumors, neuroblastomas, head and neck cancers, small cell lung carcinoma, breast cancers, intestinal cancers, pancreatic cancers, colon cancers, liver cancers, kidney cancers, skin cancers, sarcomas (over 30 types), osteosarcomas, rhabdomyosarcomas, Ewing's sarcomas, carcinomas, melanomas, ovarian cancers, cervical cancers, lymphomas, thyroid cancers, anal cancers, colo-rectal cancers, endometrial cancers, germ cell tumors, laryngeal cancers, multiple myeloma, prostate cancers, retinoblastoma, gastric cancers, testicular cancers and Wilm's tumor. The conjugates of the present invention are especially useful in the treatment and diagnosis of the cancer selected from the following group: breast cancers, cervical cancers, colon cancers, ependymomas, Ewing's sarcomas, gangliogliomas, ganglioneuromas, gliomas, glioblastomas, gliosarcomas, medulloblastomas, meningiomas, neuroblastomas, melanomas (primary and metastatic), pancreatic cancers, pheochromocytomas, prostate cancers, Schwannomas and small cell lung carcinomas. In our experiments we proved particularly high intensity uptake of our CTXD into breast cancer, glioblastoma, melanoma and pancreas carcinoma cells, which are therefore the most preferred cancer types according to the present invention.

The conjugates of the present invention are useful in the visualisation of virtually every type of malignant cancer expressing CTX binding sites. The conjugates of the present invention are especially useful in the visualisation of cancerous tissues, where the tissue is originated from the following cancers: breast cancers, cervical cancers, colon cancers, ependymomas, Ewing's sarcomas, gangliogliomas, ganglioneuromas, gliomas, gliosarcomas, medulloblastomas, meningiomas, neuroblastomas, melanomas (primary and metastatic), pancreatic cancers, pheochromocytomas, prostate cancers, Schwannomas, small cell lung carcinomas; where breast cancers, glioblastomas, melanomas and and pancretic cancers are the most preferred cancerous tissues according to the present invention.

The present invention further relates to methods for the treatment of patients suffering from cancer. In this sense, under the term "patient" any mammal is understood, such as humans, sheep, horses, cattle, pigs, dogs, cats, rats and mice. The invention is particularly useful in the treatment of human patients suffering from cancer. Said method involves administering a conjugate according to the invention to said patient in an effective dose. The conjugate for the purpose of the treatment of cancerous patient has a prosthetic group of a therapeutic agent which is suitable for the treatment of said cancer. In a preferred embodiment the conjugate used for the method of treatment is in the form of a pharmaceutical composition of the present invention.

According to a preferred embodiment of the present invention the patient to be treated with the conjugate or with the pharmaceutical composition of the present invention is suffering from a cancer selected from the group of gliomas, pancreatic cancers, melanomas and breast cancers.

The present invention further relates to a method for the preparation of conjugates of the present invention. This method comprises at least the following steps:
a) providing a chlorotoxin derivative according to any of claims 1 to 5;
b) optionally binding a linker group to the chlorotoxin derivative of step a);
c) binding a prosthetic group to the linker part of the compound formed in step b) or directly to the protein of step a) in the absence of linker.

Briefly, the starting material of the method for preparation is chlorotoxin derivative of the present invention (i.e. step "a)") . As these proteins constitute a simple amino acid sequence consisting of the usual amino acids building up proteins, the synthesis of said chlorotoxin derivatives can be carried out with well-known methods and reagents of the state of the art.

The next step of the method for preparation is optional, as the presence of a linker in the conjugates are optional. Namely, if the conjugate to be prepared is a conjugate having a linker between the chlorotoxin derivate and the prosthetic group, then said step "b" has to be performed. A linker can be attached to a protein with well-known methods of the art.

As the next step "c", the prosthetic group is attached to the construct, which is either the chlorotoxin derivative in the absence of a linker, or the construct that is the result of step "b". The binding of a prosthetic group to a protein strongly depends on the prosthetic group itself. However, building up such a construct is known by a person skilled in the art.

The method for the preparation of the conjugates may comprise further well-known steps, like isolation, purification, analysis etc., all of which are generally known for protein chemists skilled in the art.

The present invention further relates to a method for the visualisation of cancerous tissues, said method comprises the step of contacting the tissue to be examined with a conjugate of the present invention, wherein the prosthetic group of the conjugate is a visualisation agent suitable for the visualisation of the cancerous tissue. Such tissues may be those overexpressing the MMP-2 protein.

As detailed above, if the prosthetic group of the conjugate of the present invention is a visualisation agent, said conjugate can be used to visualise those tissues to which the chlorotoxin derivative part of the conjugate binds. By performing this method, a cancerous tissue can be visualised if the chlorotoxin derivative specifically binds to said cancerous tissue. With this method, a tumor can be visualised that is a great help in the course of a surgery aimed at removing said tumor.

By the above-mentioned visualisation method, a certain tumor can be localized within the body of a patient. This method is helpful, if the localization of a certain tumor type is not certain or needs to be confirmed. Using the visualisation and localisation tools provided by the present invention, the doctor will be supported during the surgical step of removing the cancerous tissue.

A further useful application of the above-mentioned visualisation method is to examine a patient to find out whether there is a certain tumor in the body of said patient. That is, the present invention provides a useful tool for the diagnosis of cancer by detecting a cancerous tissue with the help of the selective binding of the conjugate of the present invention.

Another useful application of the above-mentioned visualisation method is to localize tumor cells whereby the doctor can make sure whether a tumor was fully removed from the patient.

The present invention further relates to chimeric antigen receptors (CAR) that incorporate a chlorotoxin derivative derived extracellular domain according to the present invention. CARs constructed with chlorotoxin or similar toxins are disclosed in WO 2017/066481 A1 international patent application. As to the method of synthesis, features and use of CARs containing a chlorotoxin domain this international patent application is referred to as information known by a person skilled in the art. CARs are expressed on the surface of genetically modified T cells. A CAR T cell can be redirected to specifically recognize antigenically distinct tumor populations.

Briefly, chimeric antigen receptor (CAR) modified T cells can specifically recognize and kill various tumor cell lines by binding to their specific tumor-associated antigens (TAA). CARs are transmembrane chimeric proteins that express an extracellular TAA recognition/targeting domain which is a chlorotoxin derivative according to the present invention that provides specific and efficient binding to molecular targets and an intracellular effector domain (usually TCR zeta (TCRζ) chain) that is capable of efficient activation of T lymphocytes [June 2018]. A T cell can express a CAR if the T cell has CAR encoded by the corresponding nucleic acids following e.g. retro- or lentiviral transduction.

According to an embodiment of the present invention a nucleic acid is provided that encodes a chimeric antigen receptor, i.e. a CAR, which CAR comprises at least the following building blocks:
a) a chlorotoxin derivative according to the present invention;
b) optionally a spacer region between the chlorotoxin derivative and the transmembrane domain;
c) a transmembrane domain;
d) one or two costimulatory domains;
e) a signaling domain;
wherein the chlorotoxin derivative enables the CAR, when expressed on the surface of a T cell, to direct the T cell activity to cancerous cells.

According to this construct, the CAR encoded by the nucleic acid of the present invention has an extracellular chlorotoxin derivative domain which corresponds to the chlorotoxin derivative of the present invention. The chlorotoxin derivative forms or is part of the extracellular domain that serves as the extracellular recognition domain. Its recognition element specifically binds to a molecule present on the cell surface of a target cell. The extracellular recognition domain is connected to the intracellular effector domain by a hinge (also known as linker or spacer) and its associated transmembrane domain. That is, the transmembrane domain anchors the CAR in the membrane of said T cell. The intracellular domains comprise at least one costimulatory domain and a signaling domain, the function of which are well known by a person skilled in the art.

The extracellular domain, in other words the extracellular recognition domain, may comprise one or more same or different chlorotoxin derivatives of the present invention. According to a preferred embodiment the CAR construct comprises one chlorotoxin derivative.

The chlorotoxin derivative is preferably selected from chlorotoxin derivatives having any of the sequences set forth from SEQ ID NO: 3 to SEQ ID NO: 18 and from SEQ ID NO: 36 to SEQ ID NO: 42, preferably SEQ ID NO: 4 or SEQ ID NO: 42.

The CAR construct encoded by the nucleic acid of the present invention is optionally comprising a spacer region between the chlorotoxin derivative and the transmembrane domain. As apparent for a person skilled in the art, the spacer region is needed if the transmembrane domain and chlorotoxin derivative need to have a certain spatial arrangement between each other in order for the CAR construct to properly function. The composition and length of the spacer region determine the mobility of the antigen recognition domain as well as the physical distance between the T cell and the target. The spacer region may comprise amino acids of 5 to 300 residues of length. For example, the spacer region may comprise an IgG (IgG1 or IgG4) or CD8 hinge region.

The transmembrane domain of the CAR construct encoded by the nucleic acid of the present invention is preferably selected from the group consisting of a CD4 transmembrane domain or variant thereof, a CD8 transmembrane domain or variant thereof, a CD28 transmembrane domain or a variant thereof, and a CD3 zeta transmembrane domain or a variant thereof [Fujiwara 2020]. Under the term "variant" in relation to the transmembrane domain a protein is understood that has 1 to 5 amino acid modifications (e.g. substitutions, insertions, deletions) compared to the original protein, provided that cysteine residues are not modified.

The costimulatory domain of the CAR construct encoded by the nucleic acid of the present invention is preferably selected from the group consisting of a CD28 costimulatory domain or a variant thereof, a 4-1BB costimulatory domain or a variant thereof and an OX40 costimulatory domain or a variant thereof [Zhong 2010]. Under the term "variant" in relation to the costimulatory domain a protein is understood that has 1 to 5 amino acid modifications (e.g. substitutions) compared to the original protein, provided that cysteine residues are not modified.

The signaling domain of the CAR construct encoded by the nucleic acid of the present invention is preferably selected from the group consisting of the CD3 zeta signaling domain or a variant thereof. Under the term "variant" in relation to the signaling domain a protein is understood that has 1 to 5 amino acid modifications (e.g. substitutions) compared to the original protein, provided that cysteine residues are not modified.

The costimulatory domain and the signaling domain may have a short (i.e. less than ten amino acids) linker amino acid sequence between them.

The chlorotoxin derivative of the CAR construct encoded by the nucleic acid of the present invention enables the CAR, when expressed on the surface of a T cell, to redirect T cell activity to cancerous cells, preferably to glioblastoma cells.

The nucleic acid of the present invention can be prepared and assembled into a complete coding sequence by standard techniques of molecular cloning known by a person skilled in the art.

The present invention further relates to a vector that comprise the nucleic acid molecule encoding the CAR construct of the present invention. The vector can be a plasmid, a nucleic acid (RNA or DNA) molecule, which is used as a vehicle to carry a foreign genetic material in another cell. In the present case, said foreign genetic material is the nucleic acid of the present invention encoding said CAR construct, and said other cell is a certain immune cell suitable for said purpose (suitable immune cells are the autologous human T cells, autologous human CD4+ helper T cells, autologous human CD8+ cytotoxic T cells, a mixture of autologous human CD4+ helper T cells and CD8+ cytotoxic T cells in any proportion, allogeneic human T cells, allogeneic human CD4+ helper T cells, allogeneic human CD8+ cytotoxic T cells, a mixture of allogeneic human CD4+ helper T cells and CD8+ cytotoxic T cells in any proportion, autologous primary human natural killer (NK) cells, allogeneic primary human natural killer (NK) cells, allogeneic cells of an NK-92 cell line, autologous human monocytes, autologous human macrophages, allogeneic human monocytes, allogeneic human macrophages). This vector is suitable for the transfection of said immune cells and thereby introducing the nucleic acid molecule of the present invention into said immune cell. The transfected immune cell will be able thereafter to express said CAR construct on its surface. Entry of the vector may be facilitated by electroporation or by a transfection reagent, such as lipofectamine. The entry of the foreign genetic material into the host cells and its integration in the host cell's genetic material can also be facilitated by viral vectors, in which case the process is called transduction instead of transfection.

The present invention further relates to a population of isolated human cells, wherein said cells are either i) transfected by an RNA or DNA vector comprising an expression cassette comprising the nucleic acid of the present invention, wherein transfection is carried out *in vivo* or *ex vivo;* or ii) transduced by a viral vector comprising an expression cassette comprising the nucleic acid of the present invention, wherein transduction is carried out *in vivo* or ex *vivo.* In the case of transduction, the viral vector is preferably a retroviral or lentiviral vector, as these types of viral vectors are widely used and are proven to be useful for said transduction purposes. In the case of transduction, the population of human cells of the present invention are transduced by a viral vector, which is preferably a retro- or lentiviral vector, said vector comprises an expression cassette comprising the nucleic acid of the present invention. Here, the vector is a viral vector, in which case the term transduction is used instead of transfection. This virally transduced population of human cells will be able to express said CAR construct on the surface of the transduced cells. The transduction is carried out *in vivo* or *ex vivo.*

As obvious for a person skilled in the art, the term *in vivo* means in this context that the transfection or transduction is carried out on cells that are present in a living human. As obvious for a person skilled in the art, the term *ex vivo* means in this context that the transfection or transduction is carried out on cells that are extracted from a living human, these cells can later be introduced back into said living human or even into another living human. Said human cells shall be selected from the list consisting of the following human cell types: autologous human T cells, autologous human CD4+ helper T cells, autologous human CD8+ cytotoxic T cells, a mixture of autologous human CD4+ helper T cells and CD8+ cytotoxic T cells in any proportion, allogeneic human T cells, allogeneic human CD4+ helper T cells, allogeneic human CD8+ cytotoxic T cells, a mixture of allogeneic human CD4+ helper T cells and CD8+ cytotoxic T cells in any proportion, autologous primary human natural killer (NK) cells, allogeneic primary human natural killer (NK) cells, allogeneic cells of an NK-92 cell line, autologous human monocytes, autologous human macrophages, allogeneic human monocytes, allogeneic human macrophages.

The above listed cells are useful for the purpose of the present invention, as
i) It is proven that the efficacy of CAR T cell therapy is most often attributed to CD8+ cytotoxic T cells, and CD4+ helper T cells are known for their helper function and to intrinsically evoke cytolytic activities by enhancing CD8+ T cells activity through cytokine production [Zhang 2020].
ii) It is proven that chimeric antigen receptor modified natural killer (CAR-NK) cell-based immunotherapy is a promising and advanced alternative in the context of cancer immunotherapy of either solid tumors or hematological malignancies [Marofi 2021].
iii) The NK-92 cell line has been derived from a non-Hodgkin lymphoma patient, and its characteristics is similar to activated primary human NK cells obtained from peripheral blood. However, primary cells are challenging to isolate and expand ex *vivo.* Therefore, the NK-92 cell line provides a valuable alternative since these cells can easily be expanded in an IL-2 supplemented environment.
iv) Furthermore, it is proven that chimeric antigen receptor modified macrophages can infiltrate solid tumor tissues and interact with almost all cellular components in the tumor microenvironment (including tumor cells, immune cells such as T-cells, NK cells, dendritic cells, and other resident non-immune cells) [Mukhopadhyay 2020]. Monocytes are considered precursors of macrophages which reside in the blood.

Said population of human cells are transfected or transduced by said vectors comprising an expression cassette comprising the nucleic acid of the present invention. The population of human cells of the present invention carry a nucleic acid of the present invention in the form of an expression cassette. These human cells can be produced by isolating suitable cells from the human patient to be treated, transfecting or transducing these suitable human cells with a vector comprising an expression cassette which comprises said nucleic acid. The transfection or transduction is carried out *in vivo* or *ex vivo.*

The present invention further relates to a method of treating cancer in a patient. The method involves the step of administering the population of isolated human cells of the present invention. According to the present invention, also the combination of the different populations of human cells of the present invention can be administered for the purpose of treating cancer in a patient.

The method of treating cancer involves for example the step of administering a population of autologous or allogeneic human T cells transduced by a vector comprising an expression cassette comprising the nucleic acid of the present invention.

The method of treating cancer involves for example the step of administering a population of autologous or allogeneic human natural killer (NK) cells or an NK-92 cell line transduced by a vector comprising an expression cassette comprising the nucleic acid of the present invention.

The method involves the step of administering a population of autologous or allogeneic human monocytes or macrophages transduced by a vector comprising an expression cassette comprising the nucleic acid of the present invention.

### Our approach (Process to the invention)

Our goal was to find new CTX derivatives with improved affinity and selectivity for MMP-2 to be used for distinguishing tumor cells from cells of healthy tissues. The background science strongly suggested that MMP-2 is a real binding protein target of CTX, although this finding was previously not confirmed by a pull-down assay with recombinantly produced MMP-2 [Veiseh 2007]. Therefore, first we established a quantitative flow cytometry method, the 'Magnabead test', for assessing the binding of CTX to recombinantly produced purified MMP-2 protein. Using this novel test, we confirmed that fluorophore-labeled CTX specifically binds to beads covered with MMP-2 anchored to the beads by an anti-MMP-2 antibody. This binding, detected by measurement of the fluorescent intensities accumulated on the beads, could be saturated by increasing incubation concentrations with labeled CTX and was completely eliminated ('displaced') by incubation with excess amounts of unlabeled CTX, thus indicating that it is a specific binding to a CTX receptor on the MMP-2 protein.

Having established that purified MMP-2 alone, i.e. not necessarily in a protein complex, specifically binds CTX, we initiated a screening program for more potent MMP-2 binding CTXDs. For this purpose, we applied the designer toxin platform technology [Takacs et al., 2009]. This technology utilizes existing toxin protein sequences to create toxin protein libraries displayed on the surface of the filamentous bacteriophage M13 to identify high affinity binders by iterative rounds of selection, using a method called phage-display, invented by G.P. Smith in 1985 [Smith, 1985]. We designed and constructed a library of 1,376,254 permutational derivatives derived from 32 scorpion species preserving the pattern of disulfide bridges of CTX. The displayed proteins were fused to P3 coat protein at their C terminus via a GSASSA (peptide sequence) linker. We screened this library on human recombinant MMP-2 and identified a novel compound (hereinafter referred to as CTXD1 or SEQ ID NO: 17), which binds stronger to MMP-2 than CTX.

In order to quantitatively characterize relative intensity of binding of proteins displayed on phages we developed a new flow cytometry-based assay entitled 'Cobalt bead test' or briefly 'Co-bead test' (not yet published). This assay is based on the use of factory prefabricated cobalt coated beads (Dynabeads - His-Tag Isolation & Pulldown from ThermoFisher Scientific) aimed for his-tagged protein isolation according to principles of immobilized metal affinity chromatography (IMAC). These beads enabled immobilizing different his-tagged target proteins on their surface and thereby assessing the amount of ligand peptide displaying phage particles bound to the target protein, after fluorescent staining directed on M13 antigen of the phages. This method enabled also investigation of the binding of synthesized or recombinantly produced purified and fluorophore-labeled ligand peptides to various his-tagged target proteins. Details of this novel flow cytometry-based method are described in Example 5; preparation of phages tested in the Co-bead test are described in Example 7.

With the aid of the Co-bead test, in order to reveal structure activity relationships (SAR), we carried out conscious mutagenesis studies starting from the sequence of CTXD1 and quantified the MMP-2 binding superiority or inferiority (`relative binding') of different CTXD displaying phages compared to CTX displaying phages as reference. Relative binding was assessed by measurement of relative amount of M13-related fluorescence increase on MMP-2 coated beads relative to fluorescence signal from beads not coated with MMP-2 after incubation with equal phage concentrations of CTXD and CTX expressing phages and staining for M13 antigen detection. The amino acid sequences studied in these SAR studies and their results are presented in Table I.

**Table I: Amino acid sequences and relative binding intensities of CTX (SEQ ID NO: 1), mCTX (SEQ ID NO: 2) and various CTXDs (from SEQ ID NO: 3 to SEQ ID NO: 34) displaying phages in the Co-bead test.**

| **SEQ ID NO** | **Sequence** | **Relative MMP-2 binding** | **N** |
|---|---|---|---|
| 1 | -MCMPCFTTDHQMARKCDDCCGGKGRGKCYGPQCLCR | 1.00 | - |
| 2 | -MCMPCFTTDHQMARRCDDCCGGRGRGKCYGPQCLCR | 1.01 | 3 |
| 3 | AMCMPCFTTDHQTARRCDKCCGGY--GRCFGYQCLCNR | 3.26 | 4 |
| 4 | AMCMPCFTTDHQTARRCDKCCGGY--GRCFGYQCLCNRGY | 3.12 | 11 |
| 5 | AMCMPCFTTDHQTARRCDKCCGGY--GRCFGYQCLCNRG | 3.08 | 4 |
| 6 | -MCMPCFTTDHQTARRCDKCCGGY--GRCFGYQCLCNRGY | 3.08 | 3 |
| 7 | AMCMPCFTTDHQTARRCDKCCGGY--GRCFGYQCLCNR | 3.04 | 3 |
| 8 | --CMPCFTTDHQTARRCDKCCGGY--GRCFGYQCLCNRGY | 2.98 | 3 |
| 9 | AMCMPCFTTDHQTARRCDKCCGGY--GRCFGYQCLCN | 2.95 | 4 |
| 10 | AMCMPCFTTDHQTARRCRDCCGGY--GKCFGYQCLCNRGY | 2.95 | 6 |
| 11 | AMCMPCFTTDHQTARRCIKCCGGY--GYCFGYQCLCNRGY | 2.73 | 5 |
| 12 | AMCMPCFTTDHQTARRCHKCCGGY--GWCFGYQCLCNRGY | 2.63 | 4 |
| 13 | AMCMPCFTTDHQTARRCDRCCGGY--GKCFGYQCLCNRGY | 2.35 | 6 |
| 14 | AMCMPCFTTDHQTARRCLKCCGGY--GQCFGYQCLCNRGY | 1.99 | 5 |
| 15 | AMCMPCFTTDHQTARRCDKCCGGY--GRCFGYQCLCR | 1.78 | 4 |
| 16 | AMCMPCFTTDHQTARRCKDCCGGY--GRCFGYQCLCNRGY | 1.78 | 4 |
| 17 | AMCMPCFTTDHQTARRCKKCCGGY--GKCFGYQCLCNRGY | 1.63 | 10 |
| 18 | -MCMPCFTTDHQTARRCKKCCGGY--GKCFGYQCLCNRGY | 1.62 | 3 |
| 19 | AMCMPCFTTDHQTARRCKWCCGGY--GICFGYQCLCNRGY | 1.51 | 1 |
| 20 | AMCMPCFTTDHQTARRCVKCCGGY--GFCFGYQCLCNRGY | 1.45 | 1 |
| 21 | AMCMPCFTTDHQTARRCKHCCGGY--GVCFGYQCLCNRGY | 1.43 | 1 |
| 22 | AMCMPCFTTDHQTARRCKFCCGGY--GRCFGYQCLCNRGY | 1.39 | 2 |
| 23 | AMCMPCFTTDHQTARRCKWCCGGY--GWCFGYQCLCNRGY | 0.90 | 1 |
| 24 | AMCMPCFTTDHQTARRCDKCCGGY--GRCFGYQCLC | 0.89 | 1 |
| 25 | AMCMPCFTTDHQTARRCKSCCGGY--GFCFGYQCLCNRGY | 0.83 | 1 |
| 26 | AMCMPCFTTDHQTARRCKRCCGGY--GRCFGYQCLCNRGY | 0.69 | 1 |
| 27 | AMCMPCFTTDHQTARRCRFCCGGY--GKCFGYQCLCNRGY | 0.52 | 1 |
| 28 | AMCMPCFTTDHQTARRCVWCCGGY--GKCFGYQCLCNRGY | 0.37 | 1 |
| 29 | AMCMPCFTTDHQTARRCDDCCGGY--GKCFGYQCLCNRGY | 0.36 | 4 |
| 30 | AMCMPCFTTDHQTARRCRKCCGGY--GRCFGYQCLCNRGY | 0.32 | 1 |
| 31 | AMCMPCFTTDHQTARRCWKCCGGY--GWCFGYQCLCNRGY | 0.32 | 1 |
| 32 | AMCMPCFTTDHQTARRCRRCCGGY--GKCFGYQCLCNRGY | 0.31 | 1 |
| 33 | AMCMPCFTTDHQTARRCRWCCGGY--GKCFGYQCLCNRGY | 0.29 | 1 |
| 34 | AMCMPCFTTDHQTARRCLKCCGGY--GSCFGYQCLCNRGY | 0.26 | 1 |

The CTXD proteins that are not substantially stronger binders than the CTX itself are listed from SEQ ID NO: 19 to SEQ ID NO: 34. "--" in the middle of the sequences are only apparent gaps to have the sequences aligned to each other. "N=" refers to the number of measurements of the relative MMP-2 binding, the "Relative MMP-2 binding" is the average based on the experiments done N times. The protein encoded by SEQ ID NO: 4 is referred to in the present description as CTXD5 and the protein encoded by SEQ ID NO: 17 as CTXD1.

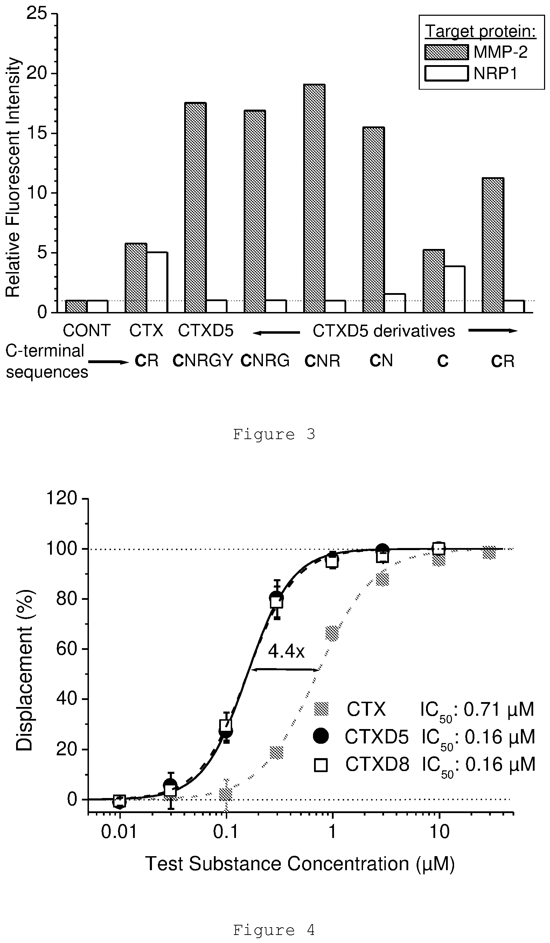

Since a monolysine CTXD protein might be advantageous for the purpose of conjugated products, for the sake of labeling or targeted delivery, first we investigated various monolysine derivatives of CTXD1 by replacing two of the three lysine residues. Surprisingly, among the monolysine derivatives of CTXD1 we have found seven derivatives (SEQ ID NO: 4, SEQ ID NO: 10, SEQ ID NO: 11, SEQ ID NO: 12, SEQ ID NO: 13, SEQ ID NO: 14, SEQ ID NO: 16) that were substantially stronger MMP-2 binders than CTXD1. Next, we aimed at determining the minimum protein length essential for the observed superior MMP-2 binding intensity, while preserving the disulfide bridge pattern. Therefore, we selected CTXD5 for further modifications and investigated the impact of N-terminal and C-terminal truncations (deletion mutations) until the first cysteine residue. The C-terminal step-by-step truncations apparently did not eliminate superior binding potential of the CTX derivative until removal of glutamine following the last cysteine, which is demonstrated in Figure 3. In this figure, a bar graph shows different binding intensities and selectivities of CTX and different CTXD5 derivatives to MMP-2 and NRP1 target proteins as indicated by a phage-display examination using the Co-bead test. The experiment was aimed at investigating the impact of C-terminal modifications of CTXD5, therefore, the different C-terminal sequences starting with the last cysteine residues of the tested CTX and CTXD5 derived proteins are also indicated. Control beads (CONT) were not exposed to phages but were coated with target proteins and were subjected to the whole staining procedure. Other beads were exposed to the indicated peptide displaying phages at uniform phage concentration of 2.6 × 10¹⁴ phage particles/mL.

Our quantitative phage binding study with the C-terminal truncation mutants was extended also to measurement of binding to NRP1 protein as well, since the article by McGonigle et al. [McGonigle 2019] suggested that C-terminal part of rCTX plays a pivotal role in binding to NRP1. The results showed that phage-displayed CTX exhibited approximately equivalent binding to both MMP-2 and NRP1, but CTXD5 and its truncation mutants were selective for MMP-2 and did not bind to NRP1, except when the C-terminal part was truncated until the cysteine residue. However, when all the amino acids after cysteine were deleted, the NRP1 binding reappeared and reached almost equivalent level with the relatively reduced MMP-2 binding (Figure 3) or with NRP1 binding of CTX. Thus, these phage-display results indicated that CTXD5 and its truncated derivatives, except the one ending with cysteine, were not only stronger binders to MMP-2 than CTX but were also selective for MMP-2 as compared to binding to NRP1. This prediction from phage-display studies was also confirmed by investigating the recombinantly produced pure CTXD5 protein substance (see below).

Deletion of one or two N-terminal amino acids of CTXD5 (i.e. SEQ ID NO: 4) resulted in chlorotoxin derivatives of SEQ ID NO: 6 and SEQ ID NO: 8, respectively (see Table I). These mutations, however, did not significantly alter the binding intensity of the resultant chlorotoxin derivatives.

Deletion of the N-terminal alanine of CTXD1 (i.e. SEQ ID NO: 17) resulted in the chlorotoxin derivative of SEQ ID NO: 18. This mutation also did not significantly alter the binding intensity of the resultant protein.

Based on the SAR results obtained from quantitative evaluation of the target protein binding of phage-displayed compounds, a set of chlorotoxin derivatives were identified, which are substantially more potent in terms of binding to MMP-2 than CTX or mCTX and are also more selective in terms of target protein selectivity, which may bring about enhanced selectivity for tumor cells/tissues compared to normal cells/tissues.

In order to confirm the features of proteins predicted from phage-display studies, CTXD5 was recombinantly produced and obtained as a purified chlorotoxin derivative. Using CTXD5 as a lead compound we characterized its target protein- and cellular binding properties in comparison with CTX and/or mCTX either alone or in conjugate forms. However, this lead compound had moderate aqueous solubility, which can be further reduced by various conjugations and thus might be a hindering factor for formulation of diagnostic or therapeutic drug formulations for parenteral use.

Therefore, we investigated the impact of deliberate solubility enhancing mutations of lead compounds SEQ ID NO: 4 (CTXD5) and SEQ ID NO: 10. Based on published scientific literature data [Trevino 2007] we assumed that replacing the FTT and QT sequences starting at amino acid positions of #7 and #12 with SSS and ES sequences, respectively, as well as applying a polyarginine sequence in the C-terminal region might substantially improve solubility of the resultant CTXD proteins while retaining enhanced MMP-2 affinity of the lead compounds. In order to assess MMP-2 affinity of the new solubility enhancing mutants, we quantified the MMP-2 binding superiority of different solubility enhancing CTXD displaying phages relative to CTX displaying phages ("Relative MMP-2 binding") as described above. The results of this solubility enhancing mutant phage-display studies on relative MMP-2 binding are shown in Table II.

**Table II: Amino acid sequences and relative binding intensities of phage-displayed CTXD variants derived from CTXDs SEQ ID NO: 4 and SEQ ID NO: 10 by mutating the underlined amino acids (i.e., the solubility cluster, and the C-terminal cluster).**

| **SEQ ID NO** | **Sequence** | **Relative MMP-2 binding** | **test substance code** |
|---|---|---|---|
| 4 | AMCMPC**FTT**DH**QT**ARRCDKCCGGYGRCFGYQCLCN**RGY** | 3.12 | CTXD5 |
| 36 | AMCMPCSSSDHESARRCDKCCGGYGRCFGYQCLCNRGY | 3.32 | |
| 37 | AMCMPCFTTDHQTARRCDKCCGGYGRCFGYQCLCNRRR | 2.73 | |
| 38 | AMCMPCSSSDHESARRCDKCCGGYGRCFGYQCLCNRRR | 2.96 | |
| 39 | AMCMPCSSSDHQTARRCDKCCGGYGRCFGYQCLCNRGY | 3.25 | |
| 10 | AMCMPC**FTT**DH**QT**ARRCRDCCGGYGKCFGYQCLCN**RGY** | 2.95 | |
| 40 | AMCMPCSSSDHESARRCRDCCGGYGKCFGYQCLCNRGY | 3.70 | |
| 41 | AMCMPCFTTDHQTARRCRDCCGGYGKCFGYQCLCNRRR | 3.51 | |
| 42 | AMCMPCSSSDHESARRCRDCCGGYGKCFGYQCLCNRRR | 2.83 | CTXD8 |

The results showed that all the designed solubility enhancing mutants retained the high MMP-2 binding, as they all exhibited approximately 3-fold or somewhat higher binding intensity than CTX. Therefore, we selected a protein comprising all the tested solubility enhancing mutations (SEQ ID NO: 42) for test substance production as a secondary lead compound and denoted it as CTXD8. Using CTXD5 as a primary lead compound and CTXD8 as a secondary lead compound we characterized their target protein- and cellular binding properties in comparison with CTX and/or mCTX either alone or in conjugate forms.

The chemical toolbox for these investigations included the following compounds.

### Chemical toolbox

### 1. Non-conjugated CTX derivatives:

Some non-conjugated CTX variants/derivatives are shown in Figure 1.

Synthetic CTX was purchased from Iris Biotech GMBH (Germany). rCTX and mCTX were recombinantly produced as described in Example 1.

### 2. Non-conjugated CTXD derivatives:

CTXD5 and CTXD8 were recombinantly produced as described in Example 1.

### 3. Conjugated CTX and CTXD derivatives

The following conjugation products were purchased or produced by conjugating chemical reactions with the above described non-conjugated CTX and chlorotoxin derivatives.

### 3.1. Cyanine-5 (-Cy5) conjugates

We used CTX-Cy5, rCTX-Cy5, mCTX-Cy5, CTXD5-Cy5 and CTXD8-Cy5 in our studies. All of them were monoconjugate CTX derivatives as selected by HPLC separation. CTX-Cy5 and rCTX-Cy5 was a mixture of molecules randomly monoconjugated at one of the three lysine residues of CTX. In the case of CTXD5, CTXD8 and mCTX, the conjugates linked at the only one lysine residue were separated and used. The conjugation reaction applied Cyanine 5 NHS ester as described in Example 2.

### 3.2. Alexa 488 (-A488) conjugate

CTX-A488 conjugate of CTX was applied as a fluorescently labeled ligand working at sufficiently distinct excitation / emission wavelengths to enable comparative assessment of displacing potencies (affinities) of different Cy5-labeled and unlabeled CTX and CTXD ligands without interference with the other (Cy5) fluorophore. Excitation / emission maxima of A488 and Cy5 are 495/519 and 647/665, respectively.

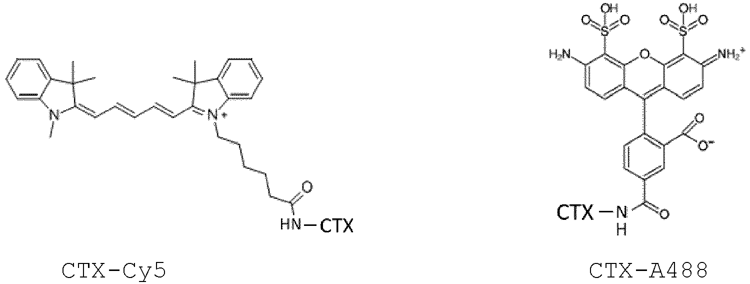

### 3.3. Cleavable cytostatic conjugates with monomethyl auristatin F (MMAF) as payload

The widely used valine-citrullin-p-aminobenzyloxycarbonyl (VC-PABC) dipeptide linker has been popularized as a way to maintain a stable covalent attachment of payload drugs to specific targeting antibodies that could be preferentially cleaved by the intracellular protease cathepsin B of the lysosomal degradation pathway [Doronina 2008]. To demonstrate this kind of targeting utility of CTXD5 in comparison with mCTX we have got synthesized cleavable cytostatic conjugates attached to the targeting protein via a glutaryl linker. Both mCTX-glutaryl-valine-citrullin-p-aminobenzyloxycarbonyl-MMAF (mCTX-G-VC-PAB-MMAF) and CTXD5-glutaryl-valine-citrullin-p-aminobenzyloxycarbonyl-MMAF (CTXD5-G-VC-PAB-MMAF) were synthesized in a way that both 1× conjugated and 2× conjugated forms, i.e. mCTX(-G-VC-PAB-MMAF)₂ and CTXD5(-G-VC-PAB-MMAF)₂, were collected at the final purification step. The second conjugation site is at the N-terminal amine. The synthesis of G-VC-PAB-MMAF conjugates is described in Example 4.

The structure of the CTXD5-G-VC-PAB-MMAF conjugate is shown in Figure 2. The construct is a preferred example of a conjugate of the present invention. Namely, the chlorotoxin derivative moiety is the CTXD5 (SEQ ID NO: 4). The conjugate has two linkers, namely a glutaryl linker and a cathepsin-cleavable linker. It further has a spacer. The prosthetic group of this conjugate is the monomethyl auristatin F (MMAF).

### 3.4. Biotin conjugated CTX and CTX derivatives

Biotinylated CTX and CTXD5 were used for cytochemistry and histochemistry applications. Randomly monobiotinylated CTX was purchased from Iris Biotech GMBH (Germany). Monobiotinylated CTXD5 was produced by a routine biotinylation procedure and the monoconjugate at the only lysine residue was purified by HPLC and lyophilized as described in Example 3.

### Profiling of CTXD5 in comparison with CTX as a targeting molecule

Using the compounds of the above detailed chemical toolbox we investigated key features of CTXD5, CTXD8 and relevant example conjugates and compared to CTX and in some aspects to mCTX and their conjugates.

For investigation of binding affinities of the test substances to selected target proteins, besides the Co-bead test we established another flow cytometry-based assay, the 'Magnabead test'. This test, which uses specific antibodies for immobilizing target proteins on the surface of beads has advantages and disadvantages compared with the Co-bead test. The advantages include higher sensitivity, i.e. working at lower effective concentrations, and insignificant level of nonspecific binding without any blocking incubation step. The disadvantages were that it could not be applied for phage-displayed protein binding studies or for uniform investigation of binding of labeled ligands to a broad panel of immobilized target proteins. The Magnabead test was used for investigating affinities of ligands in two different test modes, i.e. binding and displacement. For assessing affinities of fluorophore labeled ligands to CTX receptors the 'binding mode' was applied, whereby the amount of bound ligand was estimated directly from fluorescence intensities after incubation with various labeled ligand concentrations. For assessing affinities of unlabeled ligands to CTX receptors the 'displacement mode' was applied, whereby the beads were exposed to a fixed concentration of a labeled ligand (1 µM CTX-Cy5) after pre-incubation and co-incubation with the tested unlabeled ligand, and the bound fluorescence was measured. Relative affinities of unlabeled ligands were estimated by concentration-displacement studies characterizing the fifty percent displacing concentrations (IC₅₀ values) of the test ligands. Detailed protocols of the Magnabead test for comparative measurement of binding affinities of labeled and unlabeled CTX and CTXDs to MMP-2 and NRP1 proteins are described in Example 6.

### MMP-2 binding affinity of CTXD5 and CTXD8 compared to CTX, rCTX and mCTX

Binding affinities of CTXD5, CTXD8 and CTX were compared by concentration-response study of the unlabeled ligands obtained by displacement of 1 µM labeled ligand, CTX-Cy5 (Figure 4). Figure 4 shows the concentration-displacement relationships for CTX, CTXD5 and CTXD8 against 1 µM CTX-Cy5 as displaced ligand in the Magnabead test, indicating the difference between their affinities to MMP-2 protein. The results are presented as mean±SD of 2 independent experiments comparing the two compounds, and sigmoidal curve fitting on the mean values.

The binding affinities of CTXD5, CTXD8 and CTX were also compared by displacement of 1 µM CTX-A488 from MMP-2 in the Magnabead test. The results are shown in Table III.

**Table III: Affinities of unlabeled and labeled CTXD5 and CTX derivatives as measured by displacement of CTX-A488 (1 µM) from MMP-2 in the Magnabead test**

| **Displacer compound** | **IC₅₀ (µM)** |
|---|---|
| CTXD5 | 0.22 |
| CTXD5-Cy5 | 0.18 |
| CTX | 0.68 |
| CTX-Cy5 | 0.57 |
| rCTX | 0.68 |
| rCTX-Cy5 | 0.42 |
| mCTX | 0.77 |
| mCTX-Cy5 | 0.40 |
| CTXD8 | 0.18 |
| CTXD8-Cy5 | 0.18 |

In the latter experiments, comparisons were made to rCTX and mCTX, as well. The intensity of labeled CTX binding was displaced by all the five ligands in a concentration dependent manner. The inhibitions followed a sigmoidal concentration-response relationship and reached complete displacement suggesting a common binding site for the five protein ligands. However, CTXD5 and CTXD8 displaced the labeled ligand CTX-Cy5 4.4-times more potently than CTX with IC₅₀ values of 0.16 µM, 0.16 µM and 0.71 µM, respectively (see Figure 4). According to the CTX-A488 displacement test, IC₅₀ values of rCTX and mCTX were not substantially different from that of CTX with IC₅₀ values of 0.68 µM, 0.77 µM and 0.68 µM, respectively. Thus, the results confirmed the almost equally higher MMP-2 binding affinity of CTXD5 and CTXD8 compared to CTX, rCTX and mCTX, as predicted from the phage-display studies.

### MMP-2 binding affinities of conjugated derivatives CTXD5, CTX and mCTX

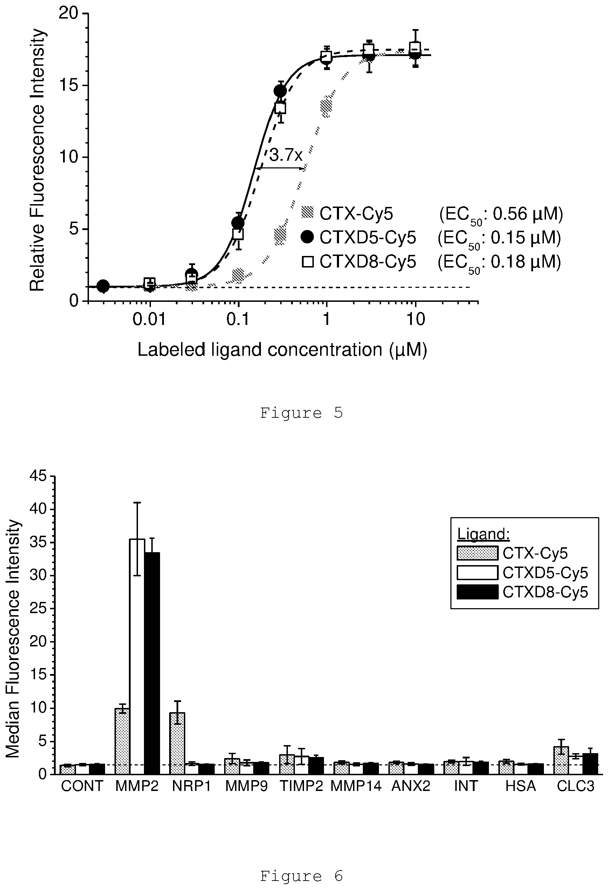

Binding affinities of CTXD5-Cy5, CTXD8-Cy5 and CTX-Cy5 were compared by concentration-response studies of binding of these labeled ligands to MMP-2 in the Magnabead test (Figure 5). This figure shows the MMP-2 binding concentration-fluorescence relationships for CTX-Cy5, CTXD5-Cy5 and CTXD8 fluorophore labeled ligands in the Magnabead test, indicating the difference between the affinities of these fluorophore labeled ligands to MMP-2 protein. The results are presented as mean±SD of three independent experiments comparing the three compounds, and sigmoidal curve fitting on the mean values. Concentration-response relationships fitted well on a sigmoidal curve and reached saturation at similar level, suggesting that they bind to approximately equal number of binding sites on MMP-2. CTXD5-Cy5 and CTXD8-Cy5 were 3.7- and 3.1-times more potent than CTX-Cy5 with EC₅₀ values of 0.15 µM, 0,18 µM and 0.56 µM, respectively. These results indicate that the conjugated ligands of CTXD5, CTXD8 and CTX all retain the affinities of the non-labeled proteins. This conclusion was also confirmed in a displacement study where CTX-A488 (1 µM) was the labeled ligand and the IC₅₀ values of CTXD5, CTXD8, CTXD5-Cy5 and CTXD8-Cy5 were 22 µM, 18 µM, 18µM and 18 µM, respectively. For comparison, unlabeled and Cy5-labeled derivatives of CTX, rCTX and mCTX were also investigated (see Table III). The results showed that MMP-2 affinities of CTX, rCTX and mCTX were not substantially different and their Cy5 conjugates exhibited similar or slightly higher affinities, with largest difference seen between the labeled and unlabeled protein with mCTX-Cy5 having an IC₅₀ value of 0.40 µM compared with 0.77 µM for mCTX.

Retaining of the MMP-2 binding affinity of the cytostatic conjugates of CTXD5 i.e. CTXD5-G-VC-PAB-MMAF and CTXD5(-G-VC-PAB-MMAF)₂ was also confirmed in a displacement study using CTX-Cy5 as displaced ligand. The IC₅₀ values of CTXD5, CTXD5-G-VC-PAB-MMAF and CTXD5(-G-VC-PAB-MMAF)₂ were similar, i.e. 0.20 µM, 0.23 µM and 0.22 µM, respectively.

### Changes in target protein selectivity of CTXD5 and CTXD8 as compared to CTX

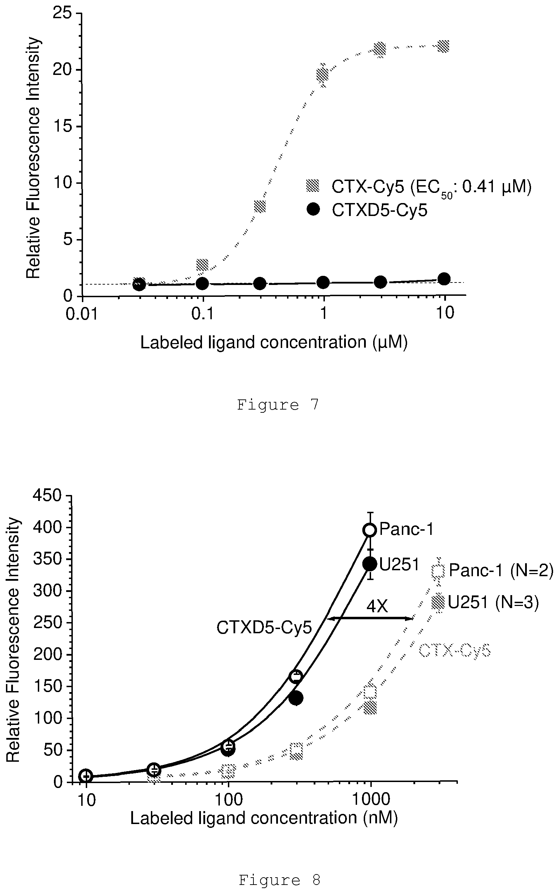

Using the Co-bead test, we investigated the binding of CTX-Cy5, CTXD5-Cy5 and CTXD8-Cy5 to a panel of target proteins that have been suggested as direct or indirect binding partners of CTX. The panel included his-tagged proteins MMP-2, MMP-9, MMP-14, TIMP-2, αᵥβ₃ integrin, Anx2, NRP1, CLC3 (chloride channel) and human serum albumin (HSA), the latter as a kind of negative control. The results are presented in Figure 6. Here, a bar graph shows different binding intensities of CTX-Cy5, CTXD5-Cy5 and CTXD8-Cy5 labeled test substances (all at 1 µM) to various postulated target proteins as measured by bead-bound fluorescent intensities in the Co-bead test. The column triplets represent control beads (CONT) that were not coated with any target protein but blocked with casein as all other target protein-coated beads coated with MMP-2, NRP1, MMP-9, TIMP-2, MMP-14, Anx2, αvβ3 integrin (INT), human serum albumin (HSA), and CLC3 chloride channel. The results are presented as mean±SD of three experiments. The results depicted in Figure 6 showed that CTX-Cy5, at a test concentration of 1 µM was binding to MMP-2 and NRP1 with approximately equal intensity and it also exhibited weaker but overt binding to MMP-9, TIMP-2 and CLC3. Nevertheless, binding of CTX-Cy5 to MMP-14, αᵥβ₃ integrin, Anx2 and HSA was negligible. In contrast, CTXD5-Cy5 and CTXD8-Cy5 were binding to MMP-2 at least 3-fold more intensively than CTX-Cy5, but their binding was negligible to all other proteins in the tested panel, except CLC3 and TIMP-2. Even their minor binding to CLC3 and TIMP-2 was apparently weaker than that of CTX-Cy5. The target protein panel binding and selectivity profiles of the two lead compounds appeared to be qualitatively and quantitatively identical. Since CTX-Cy5 had remarkable affinity to NRP-1, we compared binding concentration-response relationships of CTX-Cy5 and CTXD5-Cy5 in the Magnabead test. The results are presented in Figure 7. Here, the figure shows the NRP1 binding concentration-fluorescence relationships for CTX-Cy5 fluorophore labeled ligand and the lack of any overt binding of CTXD5-Cy5 to NRP1 in the Magnabead test. The results are presented as mean±SD of two independent experiments comparing the two compounds, and sigmoidal curve fitting on the mean values of CTX-Cy5 relative fluorescence intensities with the calculated EC₅₀ value characterizing affinity of CTX-Cy5 to NRP1. The results depicted in Figure 7 showed that CTX-Cy5 was binding to NRP1 in a concentration dependent manner with an EC₅₀ value of 0.41 µM, whereas CTXD5 showed no NRP1 binding at all, up to 10 µM test concentration. Lack of binding of unlabeled CTXD5 and CTXD8 to NRP1 was also confirmed in CTX-Cy5 displacement studies (not shown).

### Utility of CTXD5-Cy5 and CTXD8-Cy5 for selective staining of tumor cells as compared to CTX-Cy5 and mCTX-Cy5

We investigated how the higher MMP-2 binding affinity and selectivity translates into cellular uptake potency and selectivity for tumor cells versus non-tumor cells. For this purpose, we investigated a few representative tumor cell lines for qualitative and semiquantitative characterization by fluorescent microscopy and image analysis; and for quantitative characterization by flow cytometry.

### Cell culture image results

Fluorescent microscopic images of human U251MG glioblastoma cells and HDFa fibroblast cells in adherent cultures, incubated for 1 h at 37 °C with mCTX-Cy5 or CTXD5-Cy5 (2 µM) were investigated using ImageXpress Nano Automated Imaging System and analysed by MetaXpress software (Molecular Devices). For visualisation of cell bodies and nuclei the cultures were stained with Calcein AM (Invitrogen #C1430) and Hoechst 33342 (Life Technologies #H3570), respectively. The results showed that both mCTX-Cy5 and CTXD5-Cy5 were taken up by the cells and exhibited the characteristic intracellular distribution described by Wiranowska et al. [Wiranowska 2011], that is perinuclear macrogranular localization consistent with clathrin mediated endocytosis with trans-Golgi accumulation in glioblastoma cells, but diffuse cytoplasmic distribution consistent micropinocytosis in fibroblasts. The semiquantitative analysis showed higher uptake of both labeled CTXDs into the glioblastoma cells than into fibroblasts, higher uptake of CTXD5-Cy5 than mCTX-Cy5 in both cell types, and also higher CTXD5/mCTX uptake ratios in the glioblastoma than in the fibroblast cells.

### Analysis by flow cytometry

Detailed protocol of the flow cytometry investigations of cellular uptake is presented in Example 8. Briefly, the tested cells were incubated (double stained) with a viability marker (PO-PRO-1 dye) and Cy5-labeled CTX, mCTX, CTXD5, or CTXD8. The Cy5-conjugated protein uptake was measured as the increase in median fluorescence intensity (ΔMFI) relative to Cy5-protein unstained cells, after gating out non-viable cells based on high PO-PRO-1 uptake. A set of representative tumor cell lines were included in this study, comprising human glioblastoma (U251 MG and U87 MG), pancreas carcinoma (Panc-1), melanoma (A375) and breast cancer (SK-Br) cells, which have been classified as 'CTX positive' in previous studies [Lyons 2002]. In addition, a reportedly 'CTX negative' malignant tumor cell-line TE 671 human rhabdomyosarcoma [Soroceanu 1998], and normal non-tumor cells, i.e. rat astrocytes, human dermal fibroblasts (HDFa) and human umbilical vein cells (HUVEC) were also investigated to reveal alterations in tumor selectivity. First, we performed comparative concentration-uptake studies with the fluorophore labeled CTXD5 and CTX on U251 MG and Panc-1 cells. The results showed that CTXD5-Cy5 was taken up four times more potently, i.e. at 4-fold lower equally effective concentrations than CTX-Cy5 in both cell-lines (Figure 8). This figure shows the cellular uptake concentration-fluorescence relationships for CTX-Cy5 and CTXD5-Cy5 fluorophore labeled ligands in the cell staining intensity test as measured by flow cytometry. The data are presented as mean±SD of N=2 and N=3 experiments comparing the staining intensity of the two compounds in two malignant tumor cell lines: U251 and Panc-1. Based on the concentration-uptake relationships 300 nM was selected as a fixed test concentration and the conjugated protein uptake of CTXD5-Cy5, CTX-Cy5 and mCTX-Cy5 was investigated in simultaneous comparative experiments in the set of selected cell-lines. The results showed that relatively large quantities of labeled CTX, mCTX and CTXD5 were taken up by the 'CTX positive' tumor cells: glioblastomas, melanoma, pancreas cancer and breast cancer cells; and much less by the 'normal cells' and the 'CTX negative' rhabdomyosarcoma cell-line (see Table IV).

**Table IV: Cellular uptake of CTXD5-Cy5 and CTX-Cy5 into various tumor and normal cells**

| **Cell line** | **CTXD5-Cy5 uptake** | **CTX-Cy5 uptake** | **mCTX-Cy5 uptake** | **CTXD5/CTX uptake ratio** |
|---|---|---|---|---|
| U87 glioblastoma | 85.09 | 24.48 | 22.81 | 3.48 |
| U251 glioblastoma | 53.15 | 15.69 | 17.79 | 3.39 |
| A375 melanoma | 71.75 | 29.76 | 24.37 | 2.41 |
| PANC-1 pancreas cancer | 66.64 | 27.12 | 26.12 | 2.46 |
| SK-Br breast cancer | 36.82 | 12.24 | 11.67 | 3.01 |
| TE 671 rhabdomyosarcoma | 5.53 | 1.76 | 1.34 | 3.14 |
| Rat astrocyte | 9.91 | 5.37 | 5.18 | 1.85 |
| HDFa fibroblast | 5.65 | 5.19 | 5.44 | 1.09 |
| HUVEC endothel | 9.96 | 5.13 | 3.61 | 1.94 |

| | | | | |
|---|---|---|---|---|
| Uptake is ΔMFI - delta median fluorescence intensity - relative to fluorescence of Cy5-protein unstained cells. The rows of tumor cells are the cell lines U87, U251, A375, PANC-1 and SK-Br. The tumor cell line that was classified as CTX negative by Lyons et al. [Lyons 2002] is the cell line TE 671. | | | | |

The uptake of CTXD5-Cy5 was higher into all cell types than that of CTX-Cy5. However, the CTXD5/CTX uptake ratios indicated that CTXD5 was taken up 2.4-3.5-fold more intensively into the tumor cells but only 1.1-1.9-fold more intensively into normal cells than CTX, which clearly indicates that fluorophore labeled CTXD5 stains not only more intensively the tumor cells than labeled CTX, but it is also more selective for the investigated tumor cells *versus* normal cells. Cellular uptake intensities of mCTX-Cy5 were not substantially different from those of CTX-Cy5 in all tested cell lines.

To compare cellular uptake of the two labeled lead compounds CTXD8-Cy5 and CTXD5-Cy5, we performed a second series of experiments. The results of this comparative study are shown in Table V.

**Table V: Comparison of cellular uptake of CTXD8-Cy5 and CTXD5-Cy5 into various tumor and normal cells**

| **Cell line** | **CTXD8-Cy5 uptake** | **CTXD5-Cy5 uptake** |
|---|---|---|
| U87 glioblastoma | 81.20 | 83.91 |
| U251 glioblastoma | 52.08 | 53.08 |
| PANC-1 pancreas cancer | 62.56 | 65.11 |
| SK-Br breast cancer | 33.64 | 34.90 |
| HDFa fibroblast | 5.50 | 5.56 |

| | | |
|---|---|---|
| Uptake is ΔMFI - delta median fluorescence intensity - relative to fluorescence of unstained cells. | | |

The results showed that the uptake intensities for CTXD5-Cy5 in this newer study were very similar to those in the previous study (cf. Table IV). Furthermore, uptake intensities for CTXD8-Cy5 were almost identical to those for CTXD5-Cy5, which indicate a very similar cellular uptake- and tumor *versus* non-tumor selectivity profile.

### Use of CTXD5 for targeted intracellular delivery of chemotherapeutic drugs

In order to investigate improved targeting potency of CTXD5 over mCTX conjugated with a chemotherapeutic drug, we compared proliferation inhibiting potency of intracellularly cleavable MMAF conjugates of the two targeting proteins CTXD5 and mCTX. Both monoconjugate and biconjugate forms of CTXD5 and mCTX with -GVC-PAB-MMAF were tested. The protocol of the investigation is described in Example 9. The results showed that monoconjugated CTXD5-G-VC-PAB-MMAF with IC₅₀ of 585 nM was 2.4 times more potent inhibitor of U251 MG cell proliferation than monoconjugated mCTX-GVC-PAB-MMAF. The biconjugated CTXD5-(GVC-PAB-MMAF)₂ with IC₅₀ of 235 nM was 9.7 times more potent than biconjugated mCTX-(GVC-PAB-MMAF)₂. Difference between the two targeting proteins was more prominent in comparison of minimum effective concentrations, since the CTXD5 biconjugate compound had a minimum effective concentration of 10 nM, whereas the better mCTX conjugate, i.e. mCTX monoconjugate, had a minimum effective concentration of 1000 nM, which means that the chemotherapeutic conjugate of CTXD5 starts to have the desired therapeutic effect at much lower concentration levels.

### In vitro study on utility of CTXD8 for CAR T cell therapy

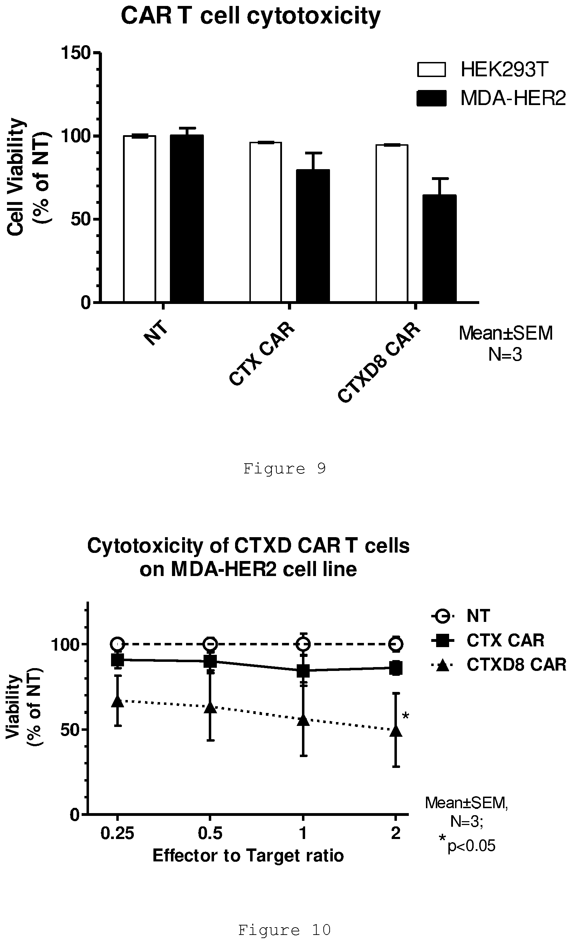

In order to investigate whether CTXD8 or canonical CTX recognition domain redirected effector cells have better potential to induce cytolysis of target cells, we generated retroviral vectors encoding a CTXD8-CAR or CTX-CAR and transduced primary human T lymphocytes that were derived from peripheral blood mononuclear cells (PBMCs) isolated from the blood obtained from three human donors. We found that both CTXD8- and CTX-CARs were stably expressed on T cells. The mean expression efficiency was 54.4% and 51.2% for CTXD8- and CTX-CAR T cells. To investigate the effector functions and cytotoxic potential of the CTXD8- and CTX-redirected CAR T cells we used a HER2+ trastuzumab resistant breast cancer-derived cell line (MDA-HER2) as target, which exhibits high MMP-2 expression. We also investigated cytotoxicity on HEK293T cell line as known CTX-CAR insensitive controls [WO 2017/066481 A1]. To assess their effector functions and cytotoxic activity, the CTXD8- and CTX-expressing CAR T ("effector") cells were cocultured with MDA-HER2 cells at different effector to target cell ratios. We investigated their effector function by determining the production of interferon gamma (IFNγ) cytokine. CTXD8- and CTX-derived CAR T cells produced significant amount of IFNγ. No cytokine production was observed in unmodified T cells (NT) or in the absence of the target antigen (HEK293T cell line). The viability of CTXD8- or CTX-CAR effector-treated tumor cells was presented as percentage of mean viability of tumor cells normalized to the mean viability measured in cocultures with non-transduced ("NT") lymphocytes. The protocols of the investigation are described in Examples 10 and 11. The results shown in Figure 9 and Figure 10 indicated that both CTX- and CTXD8-CARs induced prominent cytotoxic effects (reduced viability) that were dependent on the effector to target ratio. There was no cytotoxicity in cocultures with NT control T cells. However, neither of the CTXD8- or CTX-reprogrammed CAR T lymphocytes exerted cytotoxic effects on HEK293T cells. We concluded that CTXD8-CAR T cells induced greater cytotoxicity than CTX-CAR T cells. The effect of CTXD8-CAR T cells was statistically significant (two-way ANOVA followed by Bonferroni test, Figure 10).

### Concluding on use of the CTXDs of the present invention

In summary, using the designer toxin and phage display technologies, we developed a novel CTXD lead compound (i.e. CTXD1) with substantially higher affinity to MMP-2 protein than CTX and mCTX. With the aid of a novel flow cytometry method suitable for quantitative assessment of the strength of binding of different phage-displayed CTXD proteins to MMP-2 and to other potential target proteins, we established structure-activity relationships around this lead compound. These structure-activity relationship studies delineated a set of CTXD compounds that are even more potent in terms of MMP-2 binding as compared to CTX and mCTX, and also more selective as they do not bind to NRP1. The observations obtained by the investigation of phage-displayed proteins were further confirmed by investigations carried out with two selected example chlorotoxin derivatives, CTXD5 and CTXD8, which were recombinantly produced and used as purified test substances. These investigations were consistent with the phage-display results and indicated 3-4-fold higher MMP-2 affinity and selectivity of CTXD5 and CTXD8. Surprisingly, CTXD5 and CTXD8 proved to be more selective not only against NRP1 but also against other target proteins to which CTX exhibited some degree of binding. Fluorophore- and chemotherapeutic drug-conjugated derivatives of the lead compounds retained the MMP-2 binding affinity of the parent protein. Moreover, the fluorophore labeled CTXD5 and CTXD8 were taken up with much higher intensity than CTX into tumor cells and showed also increased selectivity for tumor cells versus normal cells. Furthermore, the tested chemotherapeutic conjugate of CTXD5 inhibited proliferation of glioblastoma cells with a higher potency than a similar conjugate of mCTX. CAR T cells assembled with CTXD8 or CTX as antigen recognizing domain exerted selective cytotoxic effects on a high MMP-2 expressing breast cancer-derived cell line while non-toxic on a control cell line. The selective cytotoxic effect of CTXD8-CAR cells was greater than that of CTX-CAR cells. These observations altogether lead to the conclusion that compounds of the present invention are useful and better than CTX or mCTX as targeting molecules either alone or as conjugated with other molecules, nano particles, or when applied as target recognizing domains in CAR cell constructs that provide additional features enabling diagnostic, or therapeutic, or combined diagnostic and therapeutic, also called theranostic applications that may be obvious from the prior art related to such postulated utilizations of CTX. The chlorotoxin derivatives of present invention may be starting points of further modifications.

Such utilizations include but are not limited to using the chlorotoxin derivatives of the present invention as diagnostic targeting molecules in various conjugated forms for intravital tumor staining for intra-operative visualisation, or for tumors on skin or mucous membrane surfaces, or for radiological and imaging applications, or for histochemical staining.

### Examples

Hereinafter, the present invention is described in more detail and specifically with reference to the Examples, which however are not intended to limit the present invention.

### Example 1: Recombinant production and purification of rCTX, mCTX, CTXD5 and CTXD8

### Cloning

DNA sequences encoding rCTX, mCTX and CTX derivatives were cloned into a pET-based expression vector that also encoded an N-terminally His-Tagged fusion partner, DsbC (disulfide bond isomerase C). CTX derivatives were amplified in a PCR reaction using a forward primer that contained the recognition site of the BamHI restriction endonuclease and the amino acid sequence of the cleavage site (WELQ) of SplB (serine protease like protein B). The reverse primer comprised two stop codons and the recognition site of XhoI. The PCR product was digested by BamHI and XhoI restriction endonucleases and ligated into the linearized vector, downstream of the DsbC fusion partner protein coding segment. The ligation reaction was transformed into chemically competent DH5α cells (Thermo Fisher Scientific). The DNA sequences of the constructs were determined by Sanger-sequencing.

### Expression

Chemically competent SHuffle T7 Express cells (New England Biolabs) were transformed with the plasmids and streaked onto LB agar, supplemented with ampicillin (100 pg/mL). Next day, a single colony was inoculated into 20 mL LB medium supplemented with ampicillin and was incubated overnight at 37 °C while shaking at 160 rpm. Next day, 1 liter of autoinduction terrific broth supplemented with ampicillin was inoculated with 1 mL of the overnight culture and was grown in Ultra Yield flasks (Thomson Instrument Company) at 30 °C at 160 rpm. After 30 hours of incubation, the bacterial cells were harvested by centrifugation at 7460xg for 10 min. The cell pellet of recombinant CTX was resuspended in 140 mL 50 mM Tris-HCl pH 8.0, 300 mM NaCl, and 0.5% Triton X-100. The cell pellets of recombinant CTX derivatives were resuspended in 140 mL lysis buffer (50 mM Tris-HCl pH 7.5, 300 mM NaCl, 30 mM imidazole, and 0.25% Triton X-100). The samples were stored at -20 °C.

### Purification of rCTX

After thawing, the cells were disrupted by sonication. The cell debris was pelleted by centrifugation at 48400xg for 20 min and the supernatant was loaded by using a peristaltic pump onto a column filled with 17 mL BioRad Nuvia IMAC chromatography resin to capture the 6xHis-tagged DsbC-CTX fusion protein. After washing with 5 column volumes of IMAC washing buffer (50 mM Tris-HCl pH 7.5, 300 mM NaCl and 30 mM imidazole), the sample was eluted from the column with IMAC elution buffer (50 mM Tris-HCl pH 7.5, 300 mM NaCl, and 250 mM imidazole). The eluate was dialyzed against 2 liters of 20 mM Tris-HCl pH 8.0 buffer overnight at 25°C. The dialyzed sample was centrifuged at 20000xg for 10 min, then filtered through a 0.2 µm membrane, and applied to a 5 mL HiTrap Q HP column (GE Healthcare) pre-equilibrated with 20 mM Tris-HCl pH 8.0 buffer (buffer A) connected to an Äkta pure chromatography system. After washing with buffer A, the DsbC-CTX fusion protein was eluted with a 0-50% linear gradient of 20 mM Tris-HCl pH 8.0, 1 M NaCl (buffer B) over 25 minutes at a flow rate of 2 mL/min. Eluted fractions were analysed on a 10% tricine-SDS gel. The DsbC-CTX containing fractions were pooled and supplemented with SplB protease (non-cleavable His-tagged) in a 2:1 fusion protein:protease ratio and with tris(2-carboxyethyl) phosphine (TCEP) at a final concentration of 100 µM. The sample was incubated overnight at 25°C. Next day, the rate of cleavage was determined on a 10% tricine-SDS PAGE gel. The sample was supplemented with NaCl and imidazole to a final concentration of 0.5 M and 15 mM, respectively, and the solution was filtered through a 0.2 µm pore size syringe filter. Next, the sample was applied to 8 ml BioRad Nuvia IMAC resin at 2 ml/min using an Äkta pure chromatography system. The column was washed with 20 mM Tris-HCl pH 8.0, 0.5 M NaCl, and 15 mM imidazole buffer and the flow-through was collected. The 6xHis-DsbC and 6xHis-SplB proteins were eluted from the column with 20 mM Tris-HCl pH 8.0, 0.5 M NaCl, and 250 mM imidazole buffer. The flow-through containing the target (rCTX) protein was concentrated using an Amicon Ultra-15 centrifugal filter unit (MWCO=3000 Da). This filter unit was also used to exchange the washing buffer to 10 mM Tris-HCl pH 8.0, 0.5 M NaCl for the subsequent size exclusion chromatography. A HiLoad 16/600 Superdex 30 pg column (GE Healthcare) connected to an Äkta pure chromatography system was equilibrated with 10 mM Tris-HCl pH 8.0, 0.5 M NaCl buffer. The sample was applied to the column in 1 mL aliquots at 1 mL/min. Then, the sample was eluted with one column volume of buffer at 1 mL/min flow rate and the absorbance was monitored at 280 nm. Fractions containing chlorotoxin were determined by PAGE on a 10% tricine-SDS gel. The Tris-HCl and NaCl content of the chlorotoxin-containing fraction was reduced to less than 0.01 mM and 0.5 mM, respectively, by repeatedly concentrating the sample on an Amicon Ultra-15 centrifugal filter unit (MWCO=3000 Da) and diluting it in USP-tested WFI water. The concentration of the sample was determined by measurement of absorbance at 214 nm, before being subjected to lyophilization. The purity and molecular mass of the protein was determined by subsequent HPLC-UV/MS measurements.

### Purification of mCTX and CTXDs

After thawing, the samples were supplemented with a final concentration of 1 mM phenylmethylsulfonyl fluoride (PMSF, protease inhibitor) and the cells were disrupted by sonication. The cell debris was pelleted by centrifugation at 48400xg for 20 min and the supernatant was loaded by using a peristaltic pump onto a column filled with 25 mL BioRad Nuvia IMAC chromatography resin to capture the 6xHis-tagged DsbC-CTX derivative fusion protein. After washing with 5 column volumes of IMAC washing buffer (50 mM Tris-HCl pH 7.5, 300 mM NaCl and 30 mM imidazole), the sample was eluted from the column with IMAC elution buffer (50 mM Tris-HCl pH 7.5, 300 mM NaCl, and 250 mM imidazole). The eluate was dialyzed using Spectra/Por Dialysis Membrane (MWCO: 12000 Da, Fisher Scientific) overnight against 2 litres of 20 mM HEPES (4-(2-hydroxyethyl)-1-piperazine ethanesulfonic acid) pH 7.5 buffer at 25 °C. After dialysis, the sample was supplemented with SplB protease and TCEP (tris(2-carboxyethyl)phosphine) in a 3:1:1 fusion protein:protease:TCEP molar ratio and was incubated overnight at 25 °C. Next day, the extent of the cleavage was inspected on a 10% tricine-SDS PAGE gel. The digested sample was centrifuged at 20000xg for 10 min, then filtered through a 0.2 µm membrane before being subjected to cation exchange chromatography performed on an Äkta pure system. The sample was loaded onto a 5 mL HiPrep SP HP column (GE Healthcare) pre-equilibrated with washing buffer (20 mM HEPES pH 7.5) and the column was washed with 5 column volumes of washing buffer. Then, the sample was eluted with a 0-50% linear gradient of elution buffer (20 mM HEPES pH 7.5, 1 M NaCl) over 25 minutes at a flow rate of 2 mL/min. Eluted fractions were analysed on an SDS-PAGE gel. Fractions containing the CTX derivatives were pooled and loaded onto a Jupiter 10µ C5 300Å column (Phenomenex) connected to an Agilent 1100 series HPLC system (Agilent Technologies). The mobile phase included HPLC grade water with 0.1% formic acid (buffer A) and acetonitrile supplemented with 0.1% formic acid (buffer B). Proteins were eluted from the column with a 15-45% linear gradient of buffer B over 30 minutes at 2 ml/min. The eluted fractions were analysed on a 10% tricine-SDS gel and fractions containing the CTXD were pooled. The concentration of the sample was determined by measurement of absorbance at 280 nm, before being subjected to lyophilization. The purity and molecular mass of the protein was determined by subsequent HPLC-UV/MS measurements.

In Examples 2 to 4 syntheses of conjugated CTX, rCTX, mCTX, CTXD5 and CTXD8 are presented.

### Example 2: Synthesis of fluorophore labeled CTX, rCTX, mCTX, CTXD5 and CTXD8

### Synthesis of cyanine 5 labeled CTX and CTX variants and derivatives

CTX-Cy5 (monoconjugated) was synthesized using a mixture of CTX (10 mg/mL) dissolved in 0.1 M sodium bicarbonate adjusted to pH 8 with 0.1 N HCl, and Cyanine 5 N-hydroxysuccinimide ester (Cy5-NHS ester, Lumiprobe) dissolved in anhydrous dimethyl formamide (DMF) supplemented with 0.1% (v/v) diisopropylethylamine (DIPEA). Concentration of the dye solution was set, and the two solutions were mixed to yield a molar ratio of 1:0.9 (CTX/dye) and solvent ratio of 1/10 (bicarbonate/DMF). Conjugation was done at room temperature for 1 h. The reacted sample was purified to homogeneity using reverse phase chromatography. The sample was loaded on an XBridge BEH C18 column (3.5 µm 4.6× 50 mm) connected to an Alliance 2795 HPLC system equipped with PDA 996 detector. The mobile phase included HPLC grade water with 0.1% formic acid (buffer A) and acetonitrile (buffer B). CTX-Cy5 was eluted from the column with a 5-70% linear gradient of buffer B from 0.5 min to 4.5 minutes at a flow rate of 2 mL/min by collecting 20-second fractions. The absorbance chromatograms were monitored at 650 nm. Relevant fraction samples were analysed in a mass spectrometer (Waters Acquity SQD; Ionization: ES+/ES-, Source block temp: 150 °C, Desolvation temp: 250 °C, Desolvation Gas:650 L/h, Cone Gas: 80 L/h, Capillary: 3000 V, Cone: 30 V, Extractor: 6 V, Rf Lens: 0.1 V, Scan: 80 to 1000 m/z in 1 sec., Inter-scan delay: 0.1 s) and the fractions containing pure monoconjugated CTX-Cy5 were pooled and the molecular mass and purity of the sample was confirmed by HPLC mass spectrometry. Finally, the resultant sample was lyophilized. Cy5 labeled rCTX, mCTX, CTXD5 and CTXD8 were synthesized by essentially the same process.

### Synthesis of Alexa 488 labeled CTX and CTX variants and derivatives

Alexa 488 labeled CTX (CTX-A488) was also synthesized by a similar process, except that the starting reactive dye compound was Alexa 488 tetrafluorophenyl (TFP) ester.

### Example 3: Synthesis of biotinylated CTXDs

Biotinylated CTXD (monoconjugated) was synthesized using a mixture of CTXD (10 mg/mL) dissolved in 0.1 M sodium bicarbonate adjusted to pH 8 with 0.1 N HCl, and biotin 3-sulfo-N-hydroxysuccinimide ester (Sigma) dissolved in anhydrous dimethyl formamide (DMF) supplemented with 0.1% (v/v) DIPEA. Concentration of the activated biotin solution was set, and the two solutions were mixed to yield a molar ratio of 1:1 (CTX/biotin) and solvent ratio of 1:10 (bicarbonate/DMF). Conjugation was done at room temperature for 1 h. The reacted sample was purified to homogeneity using reverse phase chromatography. The sample was loaded on a Phenomenex Jupiter C5 column (300 Å 4.6× 250 mm) connected to an Alliance 2795 HPLC system equipped with PDA 996 detector. The mobile phase included HPLC grade water with 0.1% formic acid (eluent A) and acetonitrile (eluent B). CTXD-biotin was eluted from the column with a 5-70% linear gradient of eluent B from 5 min to 12 min in 7 minutes at a flow rate of 1.8 mL/min by collecting 27-second fractions. The absorbance chromatograms were monitored at 280 nm. Relevant fraction samples were analysed in a mass spectrometer and the fractions containing pure monoconjugated CTXD-biotin were pooled and the molecular mass and purity of the sample was confirmed by HPLC mass spectrometry. Finally, the resultant sample was lyophilized.

### Example 4: Synthesis of CTXD5-Glutaryl-Val-Cit-PAB-MMAF and mCTX-Glutaryl-Val-Cit-PAB-MMAF

CTX receptor based targeted chemotherapeutics were designed, comprising a CTX derivative as targeting protein, an intracellularly cleaved cathepsin B enzyme sensitive linker (Glutaryl-Valine-Citrullin-P-AminoBenzyloxycarbonyl, briefly G-VC-PAB) and the cytostatic MonoMethyl Auristatin F (MMAF) as payload. For testing, G-VC-PAB-MMAF conjugates were synthesized using either CTXD5 or mCTX as targeting molecules according to the following procedures:

### Step 1: Synthesis of VC-PAB-MMAF

The starting materials were Fmoc-Val-Cit-PAB-PNP purchased from MedChemExpress (Fmoc = fluorenylmethoxycarbonyl protecting group; PNP = paranitrophenyl) and MMAF (MedChemExpress). The starting materials (Fmoc-Val-Cit-PAB-PNP 28 mg and MMAF 27 mg, molar ratio 1.25:1) were dissolved in 3.5 mL DMF containing 5.6 mg 1-hydroxybenzotriazole (HOBt) and 3 µL DIPEA. After a reaction time of 1 h at room temperature 20% (v/v) piperidine was added and let reacting for 20 minutes at room temperature to remove the Fmoc protecting group and the sample was purified on a Sun Fire C18 column (5 µm, 4.6 × 250 mm) connected to a Waters 600 semipreparative reverse phase HPLC system equipped with a 2487 dual wavelength absorbance detector.

The mobile phase included HPLC grade water with 0.1% formic acid (eluent A) and acetonitrile (eluent B). The product was eluted from the column with a 5-70% linear gradient of eluent B from 5 min to 16 min at a flow rate of 13 mL/min. The absorbance chromatograms were monitored at 280 nm. Relevant fraction samples were analysed in a mass spectrometer and the fractions containing purely the target compound (VC-PAB-MMAF) were pooled and lyophilized.

### Step 2: Synthesis of NHS-G-VC-PAB-MMAF

The starting materials for this reaction were the previously synthesized VC-PAB-MMAF and di(N-succinimidyl) glutarate purchased from Sigma. Both starting materials were dissolved in 4-4 mL dimethyl sulfoxide (DMSO), the former supplemented with 3 µL DIPEA. Then the VC-PAB-MMAF solution was added to the disuccinimidyl glutarate solution dropwise in about half an hour and let reacting overnight. Next day, the target compound was purified by HPLC as described above, lyophilized and stored at -20 °C until coupling with the CTXD.

### Step 3: Coupling of G-VC-PAB-MMAF to a CTX derivative

Coupling reactions were performed in identical manner with CTXD5 or mCTX. The coupling reaction was performed using a mixture of CTXD5 (10 mg/mL) dissolved in 0.1 M sodium bicarbonate adjusted to pH 8 with 0.1 N HCl, and NHS-G-VC-PAB-MMAF dissolved in anhydrous dimethyl formamide (DMF) supplemented with 0.1% (v/v) DIPEA. Concentration of the linker-payload solution was set, and the two solutions were mixed to yield a molar ratio of 1:2 (CTXD/linker-payload) and solvent ratio of 1:10 (bicarbonate/DMF). Conjugation was done at room temperature for 1 h. The reacted sample was purified to homogeneity using reverse phase chromatography as described above for Cy5 conjugation reactions. Since the NHS ester linker-payload molecule can conjugate primarily with the primary amine of the only lysine residue in the monolysine CTX derivatives but secondarily also to the N terminal amine, both monoconjugate (e.g. CTXD5-G-VC-PAB-MMAF) and biconjugate (CTXD5(-G-VC-PAB-MMAF)₂) derivatives formed. Therefore, both the monoconjugate and the biconjugate compounds were collected separately in the purification process and submitted for biological investigations after lyophilization.

In Examples 5 to 9 several testing protocols are presented.

### Example 5: Co-bead test

The Co-bead test was used for measurement of binding intensities of phage-displayed or purified CTXD ligand proteins to various target proteins. The test is based on the principle of anchoring various his-tagged target proteins (receptors) to immobilized cobalt-coated His-Tag Isolation & Pulldown Dynabeads (ThermoFisher Scientific, Catalog number: 10104D), then binding a fluorophore labeled ligand to the target protein and measuring of the bead-bound fluorescence intensity by flow cytometry.

### Co-bead preparation

Volumes of 0.5 µL of beads (specified bead diameter 1 pm) were pipetted in each reaction tube, and three rounds of washing were performed. Each round of washing comprises (re)suspending the beads in 2 mL Tris Buffered Saline solution (TBS, from 20x TBS of ThermoFisher Scientific, Catalog number: 28360) containing 0.05% (v/v) Tween-20 (washing solution hereafter abbreviated as TT), placing the tubes in a magnetic concentrator (DynaMag-5; ThermoFisher Scientific, Catalog number: 12303D) and discarding the supernatant after 10 minutes. Then the beads were incubated overnight at 4 °C in 50 µL target protein solution. In the studies reported in this application we applied all target proteins at identical molar concentration: 0.16 µM, which is equivalent with 10 ug/mL in the case of active MMP-2 (MW 62 kDa). The target protein solutions were dissolved or diluted in TBS. Negative control beads were incubated simultaneously with TBS containing no target protein. The used target proteins were the following (with NCBI accession number [segment amino acid sequence; his-tag position: C- or N-terminal; supplier; and catalog number# listed in parentheses): MMP2 (NP_004521.1 [110-660]; N-; ProSpec, #ENZ-769), NRP1 (NP_001019799.1 [1-644]; C-; Sino Biological; #10011-H08H); MMP-9 (NP_004985.2 [20-701]; C-; ProSpec; #ENZ-1091), TIMP-2 (NP_003246.1 [27-220]; N-; ProSpec; #ENZ-646), MMP-14 (NP_004986 [24-524]; C-;ThermoFisher; #RP77533), Anx2 (NP_001002857.1 [1-339]; N-; ProsPec; #PRO-777), αᵥβ₃ integrin (AAA52589.1 & NP_002196.4 heterodimer; both with C-terminal his-tag; Native Antigen Company; #REC31719-100), CLC3 (NP_001820.2 [1-818]; C-; Creative Biomart; custom made by recombinant expression in *E. coli*), human serum albumin (NP_000468.1 [25-609]; C-; Abcam; #ab217817). Following target protein incubation, the beads were washed three times and then blocked by incubation with 100 µL Casein Blocking Solution (Sigma, #B6429) for 1 hour at room temperature, followed by three rounds of washing. The subsequent process is different for phage-displayed proteins, and for purified and labeled protein test substances.

### Phage-display exposure and staining

In the case of testing phage-displayed protein (CTX and CTXD) ligands, the prepared beads were resuspended in the tested monoclonal phage solution and incubated for 1 hour at room temperature. The phages were dissolved in a phage and antibody incubation buffer, TBS containing 0.5% bovine serum albumin and 0.05% (v/v) Tween-20 (hereafter abbreviated as TBT). After phage exposure, the beads were washed three times, then incubated for 30 minutes at 4 °C in 50 µL TBT containing mouse monoclonal anti-M13 antibody (Sino Biological, #11973-MM05) at a dilution of 1:200. Then, the beads were washed 3 times and incubated for 30 minutes at 4 °C in 50 µL TBT containing Cy5-labeled goat antimouse secondary antibody diluted at 1:1000. After three rounds of final washing the beads were resuspended in 2 mL TT and were ready for flow cytometry measurement.

### Fluorophore labeled test substance exposure

For testing fluorophore labeled test substances (e.g. CTX-Cy5 or CTXD-Cy5), the target protein coated and casein blocked beads were resuspended in 50 µL test solution containing the specified concentration of the labeled test substance and incubated for 1 hour at room temperature. The test solutions were prepared, for example, by diluting with TT a stock solution of 100 µM test substance dissolved in water containing 10-14% (v/v) dimethyl sulfoxide (DMSO). After incubation with the test solution, the beads were washed three times, resuspended in 2 mL TT and were ready for flow cytometry measurement.

### Measurement of bead-bound fluorescent intensities by flow cytometry

Flow cytometric analysis was performed with a flow cytometer (MACSQuant Analyzer 10 of Miltenyi Biotec) using the 635 nm red laser for excitation, and R1 channel for fluorescent intensity measurement in pulse area mode. The aspiration volume was 200 µL per sample, with a setup for gentle mixing and 10000 events collected per sample. The flow rate was set slow, with a maximum number of events of 500 per second. An inclusive elliptic gate was applied around the main bead population, which was set based on a forward scatter *versus* side scatter dot plot obtained with negative control beads so that events related to doublets and multiplets were excluded from the analysis. The gating was kept constant throughout all experiments with the same type of beads. For quantitative characterization of binding the median fluorescent intensity (MFI) of gated events was used. The results were expressed in terms MFI or relative fluorescent intensity (RFI) which latter is defined as the ratio of MFI of a sample per MFI of a simultaneously processed relevant negative control sample. The negative control sample was subjected to all preparatory, blocking and staining procedures, except that the beads were not coated with the target protein. For assessment of the intensity (i.e. number / amount of bead-bound phages / protein) of binding, delta MFI or delta RFI values were used, which mean increases (difference) relative to the values of negative control samples.

During assessment of binding intensity of CTXD displaying phages (e.g. results presented in Table I and Table II) a CTX expressing phage solution was also tested in the same experiment at identical phage concentration (in the range of 1.08 to 4.08 × 10¹⁴ phage particles/mL) and the relative MMP-2 binding intensity (delta RFI) for CTXD was normalized (divided) with delta RFI for CTX. The relative MMP-2 binding intensity values presented in Table I were averages of N experiments. N was at least 3 for mCTX and the claimed compounds (Compounds 3-18).

### Example 6: Magnabead test

The Magnabead test is based on the principle of anchoring target proteins via target protein specific rabbit IgG antibody to MagnaBind Goat Anti-Rabbit IgG Beads (ThermoFisher, Scientific, Catalog number: 21356), i.e. microbeads factory-coated with goat anti-rabbit antibodies, and binding a fluorophore labeled ligand to the target protein and measuring of the bead-bound fluorescence intensity by flow cytometry. The Magnabead test was used for characterization of concentration-binding relationships of fluorophore labeled CTX, mCTX and CTXD ligand proteins to MMP-2 and NRP-1 target proteins, thereby characterizing their relative binding affinities by measurement of half-maximal effective concentration (EC₅₀ values). The method is useful also for characterizing relative affinities of unlabeled CTXDs and conjugated CTX derivatives via measurement of displacement (inhibition) of binding of fluorophore labeled CTX from its receptor on MMP-2 or NRP1 target proteins. Thus, half-maximal inhibitory concentrations (IC₅₀ values) were measured to compare affinities of various CTX, mCTX and CTXD derivatives.

### Magnabead preparation

Volumes of 1 µL of beads (specified bead diameter 1-4 pm) were pipetted in each reaction tube, and three rounds of washing were performed. Each round of washing comprises (re)suspending the beads in 2 mL Dulbecco's Phosphate Buffered Saline (DPBS, Sigma #D8662), placing the tubes in a magnetic concentrator (DynaMag-5; ThermoFisher Scientific, Catalog number: 12303D) and discarding the supernatant after 10 minutes. Then the beads were incubated at 4 °C for 1 hour in 50 µL DPBS containing a rabbit antibody against the tested target protein, diluted at 1:100. The used anti-MMP-2 antibody was a polyclonal antibody (ThermoFisher Scientific, Catalog number: PA1-16667) which was raised against a synthetic sequence at amino acid positions 504-518 in the 660 amino acid long human MMP-2 protein (NP_004521.1). The MMP-2 protein can be either the mature active enzyme (e.g. ProspecBio #Enz-100 or Sigma #SRP3118) or the proenzyme (e.g. ProspecBio #Enz-769 or Sigma #SRP6270) with very similar CTX binding results. The used anti-NRP1 antibody is a polyclonal antibody (ThermoFisher Scientific, Catalog number: PA5-26079) which was raised against a synthetic sequence at amino acid positions 722-750 in the 923 amino acid long human NRP1 protein (NP_003864.5). The NRP1 protein anchored to the beads by the antibody was recombinantly produced as a single, glycosylated polypeptide chain containing 843 amino acids (22-856) of the 923 amino acid long sequence (ProsPec, Catalog number: Cyt-1059). After the second antibody coating, the beads were washed three times and incubated at room temperature for 1 hour in 50 µL DPBS containing the selected target protein (e.g. MMP-2 or NRP1) at a concentration of 0.16 µM. In the case of Magnabead test there was no need to apply any blocking incubation to eliminate nonspecific binding.

### Test substance exposures

For testing fluorophore labeled test substances (e.g. CTX-Cy5 or CTXD-Cy5 or CTX-A488), the target protein coated beads were resuspended in a test solution containing the specified concentration of the labeled test substance and incubated for 1 hour at room temperature. The test solutions were prepared, for example, by diluting with DPBS a stock solution of 100 µM test substance dissolved in water containing 10-14% (v/v) dimethyl sulfoxide (DMSO). After incubation with the test solution, the beads were washed three times, resuspended in 2 mL DPBS and were ready for flow cytometry measurement.

For testing unlabeled test substances, the target protein coated beads were resuspended in 50 µL test solution containing the specified concentration of the unlabeled test substance dissolved in DPBS and incubated for 1 hour at room temperature. Then 50 µL DPBS solution containing 2 µM labeled CTX (CTX-Cy5 or CTX-A488) and the specified concentration of the unlabeled test substance was added to incubate the beads in 1 µM labeled CTX (displaced indicator ligand) and the specified concentration of the unlabeled test substance (displacing ligand). The beads were incubated for additional 1 hour at room temperature, then washed three times, resuspended in 2 mL DPBS and were ready for flow cytometry measurement.

### Measurement of bead-bound fluorescent intensities by flow cytometry

Flow cytometric analysis was performed with a flow cytometer (MACSQuant Analyzer 10 of Miltenyi Biotec) using the 635 nm red laser for excitation, and R1 channel for fluorescent intensity measurement of Cy5 labeled ligand binding, or the 488 nm blue laser for excitation, and B1 channel for fluorescent intensity measurement of A488 labeled ligand binding in pulse area mode. The aspiration volume was 200 µL per sample, with a setup for gentle mixing and 10000 events collected per sample. The flow rate was set slow, with a maximum number of events of 500 per second. An inclusive elliptic gate was applied around the main bead population, which was set based on a forward scatter *versus* side scatter dot plot obtained with negative control beads so that events related to doublets and multiplets were excluded from the analysis. The gating was kept constant throughout all experiments with the same type of beads.

For quantitative characterization of binding of labeled ligands, MFI of gated events was used. The results were expressed in terms of delta RFI (RFI minus one) values. The relative affinities of labeled test substances were assessed by plotting delta RFI values against log concentrations and determining half maximal effective concentrations (EC₅₀) by sigmoidal curve fitting (Boltzmann function).

In the displacement studies, the results were converted to percent inhibitions (displacement %) of labeled ligand binding, and relative affinities of unlabeled test substances were assessed by plotting percent displacements against log concentrations and determining half maximal inhibitory concentrations (IC₅₀) by sigmoidal curve fitting.

### Example 7: Preparation of phages for phage-displayed ligand peptide binding measurements

Phage display test solutions were prepared according to routine phage display protocols [e.g. Tonikian 2007]. Phosphorylated and annealed oligonucleotides encoding the expressed peptides were ligated into a pAS62 phagemid vector [US8716437B2, column 32, lines 48 to 49] at a vector/insert ratio of 1:3. The phagemid with insert encoded the displayed protein extended at its C-terminal with a linker peptide sequence (GSASSA) fused to the P3 coat protein. Electrocompetent bacterial cells (*E. coli* SS320) were transformed with the ligation products and then streaked onto ampicillin (100 pg/mL) containing lysogeny broth agar (LB/Amp) plates and incubated for 16 hours at 37 °C. Under these conditions only the phagemid bearing cells survive and form colonies. A few clones were sequenced and a single colony with confirmed target sequence was inoculated into 3 mL 2YT/Amp broth and incubated at 37 °C till the culture reached mid-log phase. The culture was infected with M13KO7 helper phage (phage/cell ratio 10:1) and was incubated for 30 min at 37 °C. The 3 mL culture was transferred into 200 mL 2YT/Amp/Kan broth and incubated overnight. The overnight cell culture (200 mL) was pelleted by centrifugation at 8000xg for 10 min in 400 mL centrifuge tubes at 4 °C. The supernatant was transferred into a fresh 400 mL centrifuge tube containing 40 mL PEG/NaCl (200 g/L PEG-8000, 146.1 g/L NaCl), then was mixed thoroughly and incubated at room temperature for 20 min. The precipitated phages were pelleted by centrifugation at 18000xg for 15 min at room temperature. After discarding the supernatant, the tubes were centrifuged again at 1000 rpm for 1 min with the pellet facing the tangential side to collect and remove the residual fluid. Phages were solubilized in 4 mL phage re-suspension buffer (TBT), and all insoluble materials were removed by centrifugation at 18000xg for 10 min at 4 °C. The supernatant was transferred into four 1.5 mL centrifuge tubes (4x1 mL) and precipitated again with 200-200 µL PEG/NaCl and was incubated for an additional 5 min before centrifuged again at 12000xg. The pellets were resuspended in low volumes (150-300 µL) of phage re-suspension buffer and combined into a single phage solution. The particle concentrations of phage solutions were determined spectrophotometrically using a NanoDrop One C (Thermo Scientific) spectrophotometer based on the linear relationship between absorbance (OD₂₆₈) and phage titer in the absorbance range of 0.5-10. The phage particle concentration was assessed according to the formula: (OD₂₆₈-OD₃₂₀)×5×10¹² particles/mL [Tonikian 2007]; and set to target concentration by diluting with TBT.

### Example 8: Analysis of cell staining intensity of fluorophore labeled test substances by flow cytometry

Cells were maintained at 37 °C in a humidified atmosphere containing 5% CO₂ in recommended media supplemented with 10% Fetal Bovine Serum (FBS) and Antibiotic Antimycotic Solution (Ab/Am, Sigma #A5955) except if otherwise stated. The used cells (source #catalog number and culture medium in parentheses) were the following: U251 MG human glioblastoma (Sigma #09063001, RPMI-1640), U87 MG human glioblastoma (Sigma #89081402, DMEM high glucose), PANC-1 human pancreas carcinoma (Sigma #87092802, DMEM high glucose + Glutamax), A375 human melanoma (Sigma #88113005, DMEM high glucose + Glutamax), SK-Br-3 human breast cancer (AddexBio #C0006007, DMEM low glucose), TE-671 human rhabdomyosarcoma (Sigma #85111502, DMEM high glucose), HDFa human dermal fibroblasts (Sigma #106-05A, Fibroblast Growth Medium without any other supplements), HUVEC human endothelial cells (ThermoFisher #C0035C, Medium 200 supplemented only with Large Vessel Endothelial Supplement), neonatal rat astrocytes (ThermoFisher Scientific #N7745100, DMEM high glucose + Glutamax, supplemented with 15% FBS).

After removal of the culture medium, cells were detached by applying TrypLE Express cell-dissociation enzyme solution for 5 to 10 min, then the enzyme was inactivated by addition of culture medium with FBS. The cells were washed three times by centrifugation, supernatant discarding and resuspension in DPBS. After the third washing, cell density of the suspension was determined from a sample of 20 µL by a Countess II Automated Cell Counter (ThermoFisher Scientific), and the fulfilment of the criterion of viability >95% was also assessed by counterstaining with 0.4% Trypan Blue. The cell density was set between 1-1.5 million cells per mL by dilution with DPBS. The cell suspension was dispensed as 100 µL samples into 5-mL flow cytometry tubes and the labeled test substance (e.g. stock solution of 100 µM diluted as needed) or equivalent vehicle (e.g. 10% DMSO solution) was added in volumes of 1-3 µL. The tubes were incubated at 37 °C in a humidified atmosphere containing 5% CO₂ for 45 minutes. The test tubes were then placed in a chill-rack and the cells were incubated for 15 minutes at 4 °C with 2.5 µM Po-Pro-1 added to each tube to stain for non-viable cells. Then, the cells were washed three times and resuspended in cold DPBS (2 mL/tube) and were kept in the chill-rack until the flow cytometry testing, performed as soon as possible, usually within one hour.

Flow cytometric analysis was performed with a flow cytometer (MACSQuant Analyzer 10 of Miltenyi Biotec) using the 635 nm red laser for excitation, and R1 channel for fluorescent intensity measurement of Cy5 labeled ligand uptake, and the 405 nm violet laser for excitation, and V1 channel for Po-Pro-1 fluorescence detection. Events from both channels were recorded in pulse area mode. The aspiration volume was 200 µL per sample, with a setup for gentle mixing and 10000 events collected per sample. The flow rate was set slow, with a maximum number of events of 500 per second.

To analyse each set of experiments, gates were first defined on linear mode FSC *versus* SSC scatter plots obtained with unstained control cells, to include in the analysis only single cells with the size and granularity typical of the cell-line. Non-viable cells displaying high Po-Pro-1 signal intensity were also excluded from the analysis. The established gate settings were kept constant throughout all experiments with the same type of cells. For quantitative characterization of staining intensities, median fluorescence intensity (MFI) of gated events was used. The results were expressed in terms MFI or relative fluorescent intensity (RFI) which latter is defined as the ratio of MFI of a sample per MFI of a simultaneously processed unstained vehicle control sample. For assessment of cellular uptake, delta MFI or delta RFI values were used, which mean increases (difference) relative to the value of simultaneous unstained control sample. Most of the experiments were performed in duplicate format.

### Example 9: Measurement of cytostatic potencies of test substances

The cells were grown in a monolayer and were incubated at 37 °C in a humidified atmosphere containing 5% CO2 until they reached 75-90% confluence level. After detaching by TrypLE Express, cells were seeded in 96-well plates at 1,500 cells/well in 100 µL per well of cell culture medium supplemented with 10% FBS. Following an approximate 24 h incubation period, the whole amount of medium in each well was replaced with 90 µl fresh medium, to which 30 µL of test substance-containing medium was added to yield various test concentrations from 3.16 nM to 3.16 µM in 3.16-fold steps. Then the cells were incubated for an additional 72 h. The viability of cells after treatment with test substances were determined using a Cell counting Kit-8 (CCK-8) viability assay performed according to the manufacturer's instructions. Briefly, CCK-8 solution was added to cells to constitute 10% culture volume. The cells were incubated for additional 4 hours at 37 °C, and the formazan dye production was measured by absorbance at 460 nm using a plate reader. All experiments were carried out in six parallel measurements.

For each treatment, a vehicle control group was run simultaneously on the same plate. The results for each treatment were calculated as % of the corresponding vehicle group and plotted against concentrations on a logarithmic scale with sigmoidal curve fitting according to Boltzmann function. Fifty percent inhibitory concentrations (IC₅₀) were determined at the inflection points, i.e. at half-way between vehicle control and maximal inhibition. Minimum effective concentrations were defined as the lowest tested concentration producing greater than twenty percent and statistically significant inhibition. Statistically significant difference from vehicle control was tested with one-way ANOVA followed by Dunnett's test.

### Example 10: Generating CTX-CAR/CTXD-CAR T cells

### Construction of the transducing vector

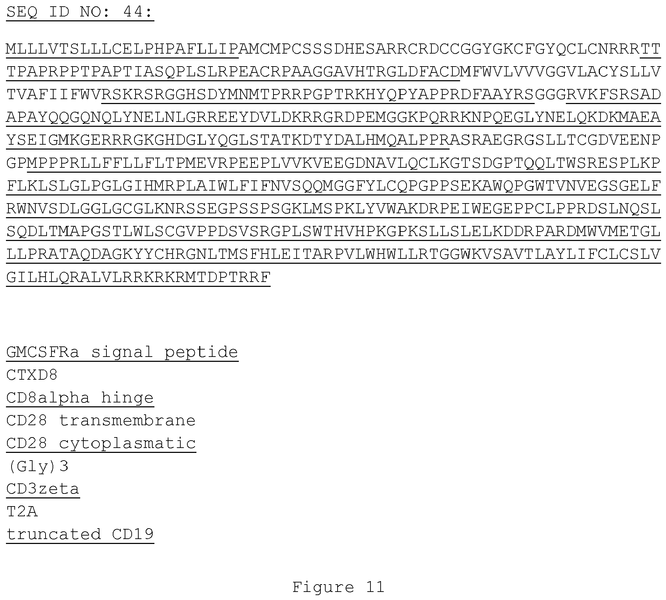

We designed a CTXD8-CAR and a CTX-CAR gene cassette containing cDNA encoding the following protein domain sequences: IgG heavy chain signal peptide (GMCSFRa), CTXD8, or CTX, CD8alpha hinge, a transmembrane region of human CD28 with CD28 intracellular costimulatory endodomains (CD28 transmembrane and CD28 cytoplasmic), (Gly)3 linker, the cytoplasmic region of human CD3 zeta, T2A self-cleaving peptide and truncated CD19. The amino acid sequence of the designed CTXD8-CAR construct is shown in Figure 11 and set forth in SEQ ID NO: 44. Figure 11 depicts the above-mentioned elements of the amino acid sequence, wherein the GMCSFRa signal peptide, the CD8alpha hinge, the CD28 cytoplasmatic, the CD3zeta, and the truncated CD19 are underlined. The functionless truncated CD19 was appended to enable detecting the transduction efficiency. The cassette was obtained as inserted in a pUC57 cloning vector, then cut out and ligated into a modified pMSGV retroviral vector. CTXD8-CAR or CTX-CAR encoding retroviral particles were generated by transient transfection of HEK 293T cells with the CTXD8- and CTX-CAR-encoding modified MSGV retroviral vectors, Peg-Pam-e plasmid containing the sequence for MoMLV gag-pol, and pMax.RD114 plasmid containing the sequence for RD114, using jetPrime transfection reagent (Polyplus, Illkirch, France). Viral supernatants were collected 72 hours post-transfection and were used on the same day for T cell transduction, or snap-frozen and stored at -80°C until use.

### Propagation and transduction of T lymphocytes

Peripheral blood mononuclear cells (PBMC) were isolated from healthy donors via density gradient separation using Histopaque^{®}-1077 (Sigma-Aldrich (St. Louis, MO, USA). Cells were then plated in 24 well non tissue culture-treated plates precoated with 1 ug/mL anti-CD3e (clone: OKT3; Thermo Fischer, Waltham, MA, USA) and anti-CD28 (R&D Systems, Minneapolis, MN, USA). Culture medium for initial stimulation was RPMI 1640 supplemented with 10% fetal bovine serum and 2 mmol/L GlutaMAX (Thermo Fisher, Waltham, MA). Twenty-four hours later, human interleukin-7 (IL-7; 10 ng/mL) and human interleukin-15 (IL-15; 5 ng/mL) (Miltenyi Biotec, Bergisch Gladbach, Germany) were added to the cultures. The following day, cells were transduced with retroviral particles on 20 ug/mL RetroNectin (Takara Bio, Mountain View, CA)-coated plates in the presence of IL-7 (10 ng/mL) and IL-15 (5 ng/mL). Following a 48-h incubation, cells were used for further experiments.

### Assessment of transduction efficiency

Efficiency of retroviral transduction was determined by flow cytometry detection of the truncated CD19 molecule by using allophycocyanin-conjugated anti-human CD19 antibody (Beckman Coulter, Brea, CA). T cell purity was determined by Alexa Fluor 488-conjugated anti-human CD3 antibody (BD Biosciences, San Jose, CA, USA) staining. All antibodies used for flow cytometric analysis were applied at a 10 ug/mL final concentration for 10 min on ice. Analysis was performed on at least 10,000 cells per sample using a NovoCyte flow cytometer and NovoExpress software (ACEA Biosciences, San Diego, CA, USA).

### Example 11: Investigation of cytotoxic potential of CTX-CAR/CTXD-CAR T cells

The HER2+ trastuzumab resistant breast cancer-derived cell line (MDA HER2) was generated by single cell cloning of the HER2-negative MDA-MB-468 cell line (purchased from American Type Culture Collection (Manassas, VA, USA)) following its transduction with human HER2 gene encoding lentiviral particles. HEK293T negative control cell line was purchased from American Type Culture Collection (Manassas, VA, USA). Firefly luciferase expressing MDA-HER2 and HEK293T cell lines were generated by single cell cloning of MDA-HER2 and HEK293T cell lines following their transduction with a retrovirus encoding eGFP.ffLuc to express an enhanced green fluorescent protein (eGFP) and firefly luciferase (ffLuc) fusion gene.

Cytotoxic activity of CAR T cells against targets was determined by a luciferase-based cytotoxicity assay. MDA-HER2 and HEK293T cells expressing eGFP/ffLuc were plated in 96-well flat-bottom plates at a density of 10⁵ cells/well in duplicates. After 4 h, varying amounts CTXD8- and CTX-derived CAR T cells were added to the tumor cells to assess cytotoxicity at a range of effector-to-target cell ratios. Wells with unmodified effector cells (NT) served as untreated control references. 24 hours later, the media and T cells were removed, and the remaining tumor cells were quantified by their luciferase activity. Luciferase assay kit was used according to the manufacturer's instructions (Promega, Madison, WI, USA) and detection performed by a Synergy HT luminometer (BioTek, Winooski, VE, USA).

### References

### Patent documents

US 20100215575 A1 (TRANSMOLECULAR INC)26 August 2010
US 20100210546 A1 (TRANSMOLECULAR INC) 19 August 2010
WO 2011094671 A2 (THE UAB RESEARCH FOUNDATION) 4 August 2011
WO 2011142858 A2 (FRED HUTCHINSON CANCER RESEARCH CENTER) 17 November 2011
WO 2015042202 A1 (BLAZE BIOSCIENCE INC) 26 March 2015
WO 2017066481 A1 (CITY OF HOPE) 20 April 2017
WO 2017136769 A1 (EISAI R&D MANAGEMENT CO LTD) 10 August 2017 US 8716437 B2 (STEVEN A. GOLDSTEIN, ZOLTAN TAKACS) 13 January 2011

### Non-patent literature

AKCAN, M. et al. Chemical re-engineering of chlorotoxin improves bioconjugation properties for tumor imaging and targeted therapy., J. Med. Chem. 2011. Vol. 54, No. 3, pages 782 to 787.
CHENG, Y. et al. Recent advances in diagnosis and treatment of gliomas using chlorotoxin-based bioconjugates, Am. J. Nucl. Med. Mol. Imaging 2014. Vol. 4, No. 5, pages 385 to 405.
COHEN, G. et al. Chlorotoxin - A Multimodal Imaging Platform for Targeting Glioma Tumors. Toxins (Basel) 2018. Vol. 10, No. 496. pages 1 to 12.
DARDEVET, L. et al. Chlorotoxin: a helpful natural scorpion peptide to diagnose glioma and fight tumor invasion. Toxins. (Basel) 2015. Vol. 7, No. 4, pages 1079 to 1101.
DEBIN, J.A. et al. Purification and characterization of chlorotoxin, a chloride channel ligand from the venom of the scorpion. Am. J. Physiol 1993. Vol. 264, No. 2, pages C361 to C369.
DESHANE, J. et al. Chlorotoxin inhibits glioma cell invasion via matrix metalloproteinase-2. J. Biol. Chem. 2003. Vol. 278, No. 6, pages 4135 to 4144.
DI, L. Strategic approaches to optimizing peptide ADME properties. AAPS J. 2015. Vol. 17, No. 1,134-143.
DINTZIS, S. M. et al. Real-time Visualization of Breast Carcinoma in Pathology Specimens From Patients Receiving Fluorescent Tumor-Marking Agent Tozuleristide. Arch. Pathol. Lab. Med. 2019. Vol. 143, No. 9, pages 1076 to 1083.
DORONINA, S. O. et al. Enhanced activity of monomethylauristatin F through monoclonal antibody delivery: effects of linker technology on efficacy and toxicity. Bioconjug. Chem. 2006. Vol. 17, No. 1, pages 114 to 124.
FUJIWARA, K. et al. Hinge and Transmembrane Domains of Chimeric Antigen Receptor Regulate Receptor Expression and Signaling Threshold. Cells, 2020. Vol. 9, No. 5. 1182.
JEELANI, S. et al. Theranostics: A treasured tailor for tomorrow. J. Pharm. Bioallied. Sci. 2014. Vol. 6, Suppl. 1, pages S6 to S8.
JUNE, C. H. et al. Chimeric Antigen Receptor Therapy. N. Engl. J. Med. 2018. Vol. 379, No. 1, pages 64 to 73.
KESAVAN, K. et al. Annexin A2 is a molecular target for TM601, a peptide with tumor-targeting and anti-angiogenic effects. J. Biol. Chem. 2010. Vol. 285, No. 7, pages 4366 to 4374.
LAMBERT, J. M. et al. Antibody-Drug Conjugates for Cancer Treatment. Annu. Rev. Med. 2018. Vol. 69, pages 191 to 207.
LYONS, S. A. et al. Chlorotoxin, a scorpion-derived peptide, specifically binds to gliomas and tumors of neuroectodermal origin. Glia 2002. Vol. 39, No. 2, pages 162 to 173.
MAMELAK, A.N. et al. Phase I single-dose study of intracavitary-administered iodine-131-TM-601 in adults with recurrent high-grade glioma. J. Clin. Oncol. 2006. Vol. 24, No. 22, pages 3644 to 3650.
MAROFI, F. et al. CAR-NK Cell: A New Paradigm in Tumor Immunotherapy. Front. Oncol. 2021. Vol. 11, 673276.
MCGONIGLE, S. et al. Neuropilin-1 drives tumor-specific uptake of chlorotoxin. Cell. Commun. Signal 2019. Vol. 17, No. 67, pages 1 to 14.
MOORE, S. J. et al. Engineering knottins as novel binding agents. Methods Enzymol. 2012. Vol. 503, pages 223 to 251.
MUKHOPADHYAY, M. Macrophages enter CAR immunotherapy. Nat. Methods. 2020. Vol. 17, No. 6, 561.
OJEDA, P.G. et al. Lysine to arginine mutagenesis of chlorotoxin enhances its cellular uptake. Biopolymers 2017. Vol. 108, No. 5, e2305.
PATIL, C. G. et al. Phase 1 Safety, Pharmacokinetics, and Fluorescence Imaging Study of Tozuleristide (BLZ-100) in Adults With Newly Diagnosed or Recurrent Gliomas. Neurosurgery 2019. Vol. 85, No. 4, pages E641 to E649.
SMITH, G. P. Filamentous fusion phage: novel expression vectors that display cloned antigens on the virion surface. Science 1985. Vol. 228, No. 4705, pages 1315 to 1317.
SOROCEANU, L. et al. Use of chlorotoxin for targeting of primary brain tumors. Cancer Res. 1998.Vol. 58, No. 21, pages 4871 to 4879.
SOROCEANU, L. et al. Modulation of glioma cell migration and invasion using Cl(-) and K(+) ion channel blockers. J. Neurosci. 1999. Vol. 19, No. 14, pages 5942 to 5954.
STROUD, M. R. et al. In vivo bio-imaging using chlorotoxin-based conjugates. Curr. Pharm. Des. 2011. Vol. 17, No. 38, pages 4362 to 4371.
TREVINO, S. R. et al. Amino acid contribution to protein solubility: Asp, Glu, and Ser contribute more favorably than the other hydrophilic amino acids in RNase Sa. J. Mol. Biol. 2007. Vol. 366, No. 2, pages 449 to 460.
TAKACS, Z. et al. A designer ligand specific for Kv1.3 channels from a scorpion neurotoxin-based library. Proc. Natl. Acad. Sci. U. S. A. 2009. Vol. 106, No. 52, pages 22211 to 22216.
TONIKIAN, R. et al. Identifying specificity profiles for peptide recognition modules from phage-displayed peptide libraries. Nat. Protoc. 2007. Vol. 2, No. 6, pages 1368 to 1386.
VEISEH, M. et al. Tumor paint: a chlorotoxin:Cy5.5 bioconjugate for intraoperative visualization of cancer foci. Cancer Res 2007. Vol. 67, No. 14, pages 6882 to 6888.
WIRANOWSKA, M. et al. Clathrin-mediated entry and cellular localization of chlorotoxin in human glioma. Cancer Cell Int. 2011. Vol. 11, No. 27, pages 1 to 13.
YOSEF, G. et al. Targeting the MMP-14/MMP-2/integrin alphavbeta3 axis with multispecific N-TIMP2-based antagonists for cancer therapy. J. Biol. Chem. 2018. Vol. 293, No. 34, pages 13310 to 13326.
ZHANG, H. P. et al. Engineering better chimeric antigen receptor T cells. Exp. Hematol. Oncol. 2020. Vol. 9, No. 1, 34.
ZHONG, X. S. et al. Chimeric antigen receptors combining 4-1BB and CD28 signaling domains augment PI3kinase/AKT/Bcl-XL activation and CD8+ T cell-mediated tumor eradication. Mol. Ther. 2010. Vol. 18, No. 2, pages 413 to 420.

## Claims

1. Chlorotoxin derivative comprising the amino acid sequence of the general sequence
X₀X₁CMPCX_{S1}X_{S2}X_{S3}DHX_{S4}X_{S5}ARRCX₂X₃CCGGYGX₄CFGYQCLCX₅X₆X₇X₈
(SEQ ID NO: 43)
wherein
(i) the N-terminal X₀X₁ cluster is selected from the group consisting of AM, 0M, or 00;
(ii) the solubilityX_{S1}X_{S2}X_{S3}X_{S4}X_{S5} cluster is selected from the group consisting of FTTQT, FTTES, SSSQT, SSSES, FSSQT, FSSES, or FSSQS;
(iii) the internal X₂X₃X₄ cluster is selected from the group consisting of DKR, RDK, KDR, IKY, HKW, DRK, LKQ, KKK; and
(iv) the C-terminal X₅X₆X₇X₈ cluster is selected from the group consisting of N000, R000, NR00, NRG0, NRGY, NRRR, or RRRR;
where 0 denotes a position where no amino acid is present; wherein said chlorotoxin derivative has a relative human MMP-2 binding that is at least 1.62 times higher than the wild-type chlorotoxin of SEQ ID NO: 1.

2. Chlorotoxin derivative according to claim 1 comprising the amino acid sequence of the general sequence
X₀X₁CMPCFTTDHQTARRCX₂X₃CCGGYGX₄CFGYQCLCX₅X₆X₇X₈
(SEQ ID NO: 35)
wherein
(i) the N-terminal X₀X₁ cluster is selected from the group consisting of AM, 0M, or 00;
(ii) the internal X₂X₃X₄ cluster is selected from the group consisting of DKR, RDK, KDR, IKY, HKW, DRK, LKQ, KKK; and
(iii) the C-terminal X₅X₆X₇X₈ cluster is selected from the group consisting of N000, ROOD, NR00, NRG0, or NRGY;
where 0 denotes a position where no amino acid is present.

3. Chlorotoxin derivative according to claim 1 or 2, wherein the internal X₂X₃X₄ cluster is DKR.

4. Chlorotoxin derivative according to claim 1, wherein the chlorotoxin derivative is selected from chlorotoxin derivatives having any of the sequences set forth from SEQ ID NO: 3 to SEQ ID NO: 18 and from SEQ ID NO: 36 to SEQ ID NO: 42.

5. Chlorotoxin derivative according to claim 1, wherein the chlorotoxin derivative has the sequence set forth in SEQ ID NO: 4 or SEQ ID NO: 42.

6. Chlorotoxin derivative according to any of claims 1 to 5, wherein the chlorotoxin derivative is cyclic.

7. Conjugate comprising a chlorotoxin derivative of any of claims 1 to 6 and a prosthetic group.

8. Conjugate of claim 7, wherein the prosthetic group is selected from the following agents:
(i) visualisation agents, preferably selected from fluorescent labels, radiolabels, magnetic resonance imaging labels, and agents enabling indirect labeling by high affinity binding to labeling molecules;
(ii) therapeutic agents, preferably selected from chemotherapeutic agents, and biological therapeutic agents;
(iii) targeting agents, preferably selected from antibodies, polypeptides, polysaccharides, and nucleic acids;
(iv) moieties that increase the circulatory half-life, preferably selected from peg moieties, glycosyl moieties, glycosylpeg moieties and moieties enabling cyclization of the chlorotoxin derivative of any of claims 1 to 6 via a linker extension.

9. Conjugate of any of claims 7 or 8, wherein the conjugate comprises one or more linker(s) and optionally one or more spacer(s) between the chlorotoxin derivative and the prosthetic group.

10. Conjugate of claim 9, wherein the linker is selected from the group of peptides, dimethyl disulfide linker, glutaryl linker, cathepsin-cleavable linkers.

11. Theranostic pair of conjugates comprising a first conjugate and a second conjugate, wherein said first conjugate is a conjugate according to any of claims 7 to 10 and said second conjugate is a conjugate according to any of claims 7 to 10.

12. Theranostic pair of claim 11, wherein
a) the chlorotoxin derivative part of said first conjugate and said second conjugate have the same amino acid sequence and said two conjugates have different prosthetic groups; or
b) the chlorotoxin derivative part of said first conjugate and said second conjugate have different amino acid sequences and said first conjugate and said second conjugate have the same or different prosthetic groups.

13. Kit comprising a conjugate of any claims 7 to 10 and instructions for use.

14. Kit comprising the theranostic pair of claim 11 or 12, and instructions for use.

15. Pharmaceutical composition comprising a conjugate of any of claims 8 to 10, wherein the prosthetic group of the conjugate is a therapeutic agent or a visualisation agent and the pharmaceutical composition further comprises a pharmaceutically acceptable excipient.

16. Conjugate of any of claims 7 to 10 for use in the treatment of cancer.

17. Conjugate of any of claims 7 to 10 for use in the diagnosis of cancer.

18. Conjugate of any of claims 7 to 10, wherein the prosthetic group is a visualisation agent and it is for use in the visualisation of cancerous tissues.

19. Conjugate for use according to claim 16 or 17, wherein the cancer is selected from the group of breast cancers, cervical cancers, colon cancers, ependymomas, Ewing's sarcomas, gangliogliomas, ganglioneuromas, gliomas, glioblastomas, gliosarcomas, medulloblastomas, meningiomas, neuroblastomas, melanomas (primary and metastatic), pancreatic cancers, pheochromocytomas, prostate cancers, Schwannomas, small cell lung carcinomas; more preferred, the cancer is selected from the group of breast cancers, glioblastomas, melanomas and pancreatic cancers.

20. Conjugate for use according to claim 18, wherein the cancerous tissue is selected from the group of breast cancers, cervical cancers, colon cancer, ependymomas, Ewing's sarcomas, gangliogliomas, ganglioneuromas, gliomas, glioblastomas, gliosarcomas, medulloblastomas, meningiomas, neuroblastomas, melanomas (primary and metastatic), pancreatic cancers, pheochromocytomas, prostate cancers, Schwannomas, small cell lung carcinomas; more preferred, the cancerous tissue is selected from the group of breast cancers, glioblastomas, melanomas and pancreatic cancers.

21. Pharmaceutical composition according to claim 15 for use in the treatment of cancer, wherein the prosthetic group of the conjugate is a therapeutic agent suitable for the treatment of the cancer.

22. Pharmaceutical composition for use according to claim 21, wherein the cancer is selected from the group of breast cancers, cervical cancers, colon cancers, ependymomas, Ewing's sarcomas, gangliogliomas, ganglioneuromas, gliomas, glioblastomas, gliosarcomas, medulloblastomas, meningiomas, neuroblastomas, melanomas (primary and metastatic), pancreatic cancers, pheochromocytomas, prostate cancers, Schwannomas, small cell lung carcinomas; more preferred, the cancer is selected from the group of breast cancers, glioblastomas, melanomas and pancreatic cancers.

23. Method for the preparation of conjugates, comprising the steps of
a) providing a chlorotoxin derivative according to any of claims 1 to 6;
b) optionally binding a linker group to the chlorotoxin derivative of step a);
c) binding a prosthetic group to the linker part of the compound formed in step b) or directly to the protein of step a) in the absence of linker.

24. Method for the visualisation of a cancerous tissue, comprising the step of contacting the tissue to be examined with a conjugate of any of claims 7 to 10, wherein the prosthetic group of said conjugate is a visualisation agent suitable for the visualisation of the cancerous tissue.

25. A nucleic acid molecule encoding a chimeric antigen receptor, wherein the chimeric antigen receptor comprises:
a) a chlorotoxin derivative according to any of claims 1 to 6;
b) optionally a spacer region between the chlorotoxin derivative and the transmembrane domain;
c) a transmembrane domain;
d) one or two costimulatory domains;
e) a signaling domain;
wherein the chlorotoxin derivative enables the chimeric antigen receptor, when expressed on the surface of a T cell, to direct the T cell activity to cancerous cells.

26. The nucleic acid molecule according to claim 25, wherein the chlorotoxin derivative is selected from chlorotoxin derivatives having any of the sequences set forth from SEQ ID NO: 3 to SEQ ID NO: 18 and from SEQ ID NO: 36 to SEQ ID NO: 42, preferably SEQ ID NO: 4 or SEQ ID NO: 42.

27. The nucleic acid molecule according to claim 25 or 26, wherein the
i) transmembrane domain selected from the group consisting of a CD4 transmembrane domain or variant thereof, a CD8 transmembrane domain or variant thereof, a CD28 transmembrane domain or a variant thereof, and a CD3 zeta transmembrane domain or a variant thereof;
ii) the one or two costimulatory domains selected from the group consisting of a CD28 costimulatory domain or a variant thereof, a 4-1BB costimulatory domain or a variant thereof and an OX40 costimulatory domain or a variant thereof; and
iii) the signaling domain is the CD3 zeta signaling domain or a variant thereof.

28. A vector comprising the nucleic acid molecule of any of claims 25 to 27.

29. A population of isolated human cells
i) transfected by an RNA or DNA vector comprising an expression cassette comprising the nucleic acid of any of claims 25 to 27, wherein transfection is carried out *in vivo* or *ex vivo*; or
ii) transduced by a viral vector comprising an expression cassette comprising the nucleic acid of any of claims 25 to 27, wherein transduction is carried out *in vivo* or *ex vivo*, wherein the viral vector is preferably a retroviral or lentiviral vector;
and wherein said human cells are selected from the list consisting of autologous human T cells, autologous human CD4+ helper T cells, autologous human CD8+ cytotoxic T cells, a mixture of autologous human CD4+ helper T cells and CD8+ cytotoxic T cells in any proportion, allogeneic human T cells, allogeneic human CD4+ helper T cells, allogeneic human CD8+ cytotoxic T cells, a mixture of allogeneic human CD4+ helper T cells and CD8+ cytotoxic T cells in any proportion, autologous primary human natural killer (NK) cells, allogeneic primary human natural killer (NK) cells, allogeneic cells of an NK-92 cell line, autologous human monocytes, autologous human macrophages, allogeneic human monocytes, allogeneic human macrophages.

30. One or more populations of isolated human cells according to claim 29 for use in the treatment of cancer.

## Patentansprüche

1. Chlorotoxinderivat, umfassend die Aminosäuresequenz der allgemeinen Sequenz
X₀X₁CMPCX_{S1}X_{S2}X_{S3}DHX_{S4}X_{S5}ARRCX₂X₃CCGGYGX₄CFGYQCLCX₅X₆X₇X₈
(SEQ ID NR: 43)
wobei
(i) der N-terminale X₀X₁-Cluster aus der Gruppe bestehend aus AM, 0M oder 00 ausgewählt ist;
(ii) der Löslichkeits-X_{S1}X_{S2}X_{S3}X_{S4}X_{S5}-Cluster aus der Gruppe bestehend aus FTTQT, FTTES, SSSQT, SSSES, FSSQT, FSSES oder FSSQS ausgewählt ist;
(iii) der interne X₂X₃X₄-Cluster aus der Gruppe bestehend aus DKR, RDK, KDR, IKY, HKW, DRK, LKQ, KKK ausgewählt ist; und
(iv) der C-terminale X₅X₆X₇X₈-Cluster aus der Gruppe bestehend aus N000, R000, NR00, NRGO, NRGY, NRRR oder RRRR ausgewählt ist;
wobei 0 eine Position kennzeichnet, an der keine Aminosäure vorhanden ist; wobei das Chlorotoxinderivat eine relative humane MMP-2-Bindung aufweist, die mindestens 1,62 mal höher ist als die des Wildtyp-Chlorotoxins der SEQ ID NR: 1.

2. Chlorotoxinderivat nach Anspruch 1, umfassend die Aminosäuresequenz der allgemeinen Sequenz
X₀X₁CMPCFTTDHQTARRCX₂X₃CCGGYGX₄CFGYQCLCX₅X₆X₇X₈
(SEQ ID NR: 35)
wobei
(i) der N-terminale X₀X₁-Cluster aus der Gruppe bestehend aus AM, 0M oder 00 ausgewählt ist;
(ii) der interne X₂X₃X₄-Cluster aus der Gruppe bestehend aus DKR, RDK, KDR, IKY, HKW, DRK, LKQ, KKK ausgewählt ist; und
(iii) der C-terminale X₅X₆X₇X₈-Cluster aus der Gruppe bestehend aus N000, R000, NR00, NRGO, oder NRGY ausgewählt ist;
wobei 0 eine Position kennzeichnet, an der keine Aminosäure vorhanden ist;

3. Chlorotoxinderivat nach Anspruch 1 oder 2, wobei der interne X₂X₃X₄-Cluster DKR ist.

4. Chlorotoxinderivat nach Anspruch 1, wobei das Chlorotoxinderivat ausgewählt ist aus Chlorotoxinderivaten, die eine der dargelegten Sequenzen von SEQ ID NR: 3 bis SEQ ID NR: 18 und von SEQ ID NR: 36 bis SEQ ID NR: 42 aufweisen.

5. Chlorotoxinderivat nach Anspruch 1, wobei das Chlorotoxinderivat die in SEQ ID NR: 4 oder SEQ ID NR: 42 dargelegten Sequenz aufweist.

6. Chlorotoxinderivat nach einem der Ansprüche 1 bis 5, wobei das Chlorotoxinderivat zyklisch ist.

7. Konjugat, umfassend ein Chlorotoxinderivat nach einem der Ansprüche 1 bis 6 und eine prosthetische Gruppe.

8. Konjugat nach Anspruch 7, wobei die prosthetische Gruppe aus den folgenden Mitteln ausgewählt ist:
(i) Visualisierungsmittel, vorzugsweise ausgewählt aus Fluoreszenzmarkern, Radiomarkern, Markern für die Magnetresonanzbildgebung und Mitteln, die eine indirekte Markierung durch hochaffine Bindung an Markierungsmoleküle ermöglichen;
(ii) therapeutische Mittel, vorzugsweise ausgewählt aus chemotherapeutischen Mitteln und biologischen therapeutischen Mitteln;
(iii) Targeting-Mittel, vorzugsweise ausgewählt aus Antikörpern, Polypeptiden, Polysacchariden und Nukleinsäuren;
(iv) Komponenten, die die zirkulierende Halbwertszeit erhöhen, vorzugsweise ausgewählt aus Peg-Komponenten, Glycosyl-Komponenten, Glycosylpeg-Komponenten und Komponenten, die eine Zyklisierung des Chlorotoxinderivats nach einem der Ansprüche 1 bis 6 über eine Linker-Verlängerung ermöglichen.

9. Konjugat nach einem der Ansprüche 7 oder 8, wobei das Konjugat einen oder mehrere Linker(n) und gegebenenfalls einen oder mehrere Spacer(n) zwischen dem Chlorotoxinderivat und der prosthetischen Gruppe umfasst.

10. Konjugat nach Anspruch 9, wobei der Linker aus der Gruppe aus Peptiden, Dimethyldisulfid-Linkern, Glutaryl-Linkern und kathepsin-spaltbaren Linkern ausgewählt ist.

11. Theranostisches Paar von Konjugaten, umfassend ein erstes Konjugat und ein zweites Konjugat, wobei das erste Konjugat ein Konjugat nach einem der Ansprüche 7 bis 10 ist und das zweite Konjugat ein Konjugat nach einem der Ansprüche 7 bis 10 ist.

12. Theranostisches Paar nach Anspruch 11, wobei
a) der Chlorotoxinderivatteil des ersten Konjugats und des zweiten Konjugats die gleiche Aminosäuresequenz aufweisen und die beiden Konjugate unterschiedliche prosthetische Gruppen aufweisen; oder
b) der Chlorotoxinderivatteil des ersten Konjugats und des zweiten Konjugats unterschiedliche Aminosäuresequenzen aufweisen und das erste Konjugat und das zweite Konjugat die gleichen oder unterschiedliche prosthetische Gruppen aufweisen.

13. Kit umfassend ein Konjugat nach einem der Ansprüche 7 bis 10 und eine Gebrauchsanweisung.

14. Kit umfassend das theranostische Paar nach Anspruch 11 oder 12 und eine Gebrauchsanweisung.

15. Pharmazeutische Zusammensetzung, umfassend ein Konjugat nach einem der Ansprüche 8 bis 10, wobei die prosthetische Gruppe des Konjugats ein therapeutisches Mittel oder ein Visualisierungsmittel ist und die pharmazeutische Zusammensetzung ferner einen pharmazeutisch verträglichen Hilfsstoff umfasst.

16. Konjugat nach einem der Ansprüche 7 bis 10 zur Verwendung bei der Behandlung von Krebs.

17. Konjugat nach einem der Ansprüche 7 bis 10 zur Verwendung bei der Diagnose von Krebs.

18. Konjugat nach einem der Ansprüche 7 bis 10, wobei die prosthetische Gruppe ein Visualisierungsmittel ist und sie zur Verwendung bei der Visualisierung von Krebsgewebe dient.

19. Konjugat zur Verwendung nach Anspruch 16 oder 17, wobei die Krebserkrankung ausgewählt ist aus der Gruppe von Brustkrebs, Gebärmutterhalskrebs, Dickdarmkrebs, Ependymomen, Ewing-Sarkomen, Gangliogliomen, Ganglioneuromen, Gliomen, Glioblastomen, Gliosarkomen, Medulloblastomen, Meningiomen, Neuroblastomen, Melanomen (primär und metastatisch), Bauchspeicheldrüsenkrebs, Phäochromozytomen, Prostatakrebs, Schwannomen, kleinzelligen Lungenkarzinomen; stärker bevorzugt ist die Krebserkrankung ausgewählt aus der Gruppe von Brustkrebs, Glioblastomen, Melanomen und Bauchspeicheldrüsenkrebs.

20. Konjugat zur Verwendung nach Anspruch 18, wobei das Krebsgewebe ausgewählt ist aus der Gruppe von Brustkrebs, Gebärmutterhalskrebs, Dickdarmkrebs, Ependymomen, Ewing-Sarkomen, Gangliogliomen, Ganglioneuromen, Gliomen, Glioblastomen, Gliosarkomen, Medulloblastomen, Meningiomen, Neuroblastomen, Melanomen (primär und metastatisch), Bauchspeicheldrüsenkrebs, Phäochromozytomen, Prostatakrebs, Schwannomen, kleinzelligen Lungenkarzinomen; stärker bevorzugt ist das Krebsgewebe ausgewählt aus der Gruppe von Brustkrebs, Glioblastomen, Melanomen und Bauchspeicheldrüsenkrebs.

21. Pharmazeutische Zusammensetzung nach Anspruch 15 zur Verwendung bei der Behandlung von Krebs, wobei die prosthetische Gruppe des Konjugats ein therapeutisches Mittel ist, das für die Behandlung des Krebses geeignet ist.

22. Pharmazeutische Zusammensetzung zur Verwendung nach Anspruch 21, wobei die Krebserkrankung ausgewählt ist aus der Gruppe von Brustkrebs, Gebärmutterhalskrebs, Dickdarmkrebs, Ependymomen, Ewing-Sarkomen, Gangliogliomen, Ganglioneuromen, Gliomen, Glioblastomen, Gliosarkomen, Medulloblastomen, Meningiomen, Neuroblastomen, Melanomen (primär und metastatisch), Bauchspeicheldrüsenkrebs, Phäochromozytomen, Prostatakrebs, Schwannomen, kleinzelligen Lungenkarzinomen; stärker bevorzugt ist die Krebserkrankung ausgewählt aus der Gruppe von Brustkrebs, Glioblastomen, Melanomen und Bauchspeicheldrüsenkrebs.

23. Verfahren zur Herstellung von Konjugaten, umfassend die folgenden Schritte
a) Bereitstellen eines Chlorotoxinderivats nach einem der Ansprüche 1 bis 6;
b) gegebenenfalls Binden einer Linkergruppe an das Chlorotoxinderivat aus Schritt a),
c) Binden einer prosthetischen Gruppe an den Linker-Teil der in Schritt b) gebildeten Verbindung oder direkt an das Protein aus Schritt a) bei Nichtvorhandensein eines Linkers.

24. Verfahren zur Visualisierung eines Krebsgewebes, umfassend den Schritt des Inkontaktbringens des zu untersuchenden Gewebes mit einem Konjugat nach einem der Ansprüche 7 bis 10, wobei die prosthetische Gruppe des Konjugats ein für die Visualisierung des Krebsgewebes geeignetes Visualisierungsmittel ist.

25. Nukleinsäuremolekül, das einen chimären Antigenrezeptor kodiert, wobei der chimäre Antigenrezeptor Folgendes umfasst:
a) ein Chlorotoxinderivat nach einem der Ansprüche 1 bis 6;
b) gegebenenfalls eine Spacer-Region zwischen dem Chlorotoxinderivat und der Transmembrandomäne;
c) eine Transmembrandomäne;
d) eine oder zwei kostimulatorische Domänen;
e) eine Signalisierungsdomäne;
wobei das Chlorotoxinderivat dem chimären Antigenrezeptor ermöglicht, wenn er auf der Oberfläche einer T-Zelle exprimiert ist, die Aktivität der T-Zelle auf Krebszellen zu richten.

26. Nukleinsäuremolekül nach Anspruch 25, wobei das Chlorotoxinderivat ausgewählt ist aus Chlorotoxinderivaten mit einer der dargelegten Sequenzen von SEQ NR: 3 bis SEQ ID NR: 18 und von SEQ ID NR: 36 bis SEQ ID NR: 42, bevorzugt SEQ ID NR: 4 oder SEQ ID NR: 42 aufweisen.

27. Das Nukleinsäuremolekül nach Anspruch 25 oder 26, wobei
i) die Transmembrandomäne, aus der Gruppe bestehend aus einer CD4-Transmembrandomäne oder einer Variante davon, einer CD8-Transmembrandomäne oder einer Variante davon, einer CD28-Transmembrandomäne oder einer Variante davon und einer CD3-Zeta-Transmembrandomäne oder einer Variante davon ausgewählt ist;
ii) die eine oder zwei kostimulatorische Domäne(n) aus der Gruppe bestehend aus einer CD28 kostimulatorischen Domäne oder einer Variante davon, einer 4-1BB kostimulatorischen Domäne oder einer Variante davon und einer OX40 kostimulatorischen Domäne oder einer Variante davon ausgewählt ist/sind; und
iii) die Signaldomäne die CD3-Zeta-Signaldomäne oder eine Variante davon ist.

28. Ein Vektor umfassend das Nukleinsäuremolekül nach einem der Ansprüche 25 bis 27.

29. Eine Population von isolierten humanen Zellen
i) transfiziert durch einen RNA- oder DNA-Vektor, der eine Expressionskassette umfasst, die die Nukleinsäure nach einem der Ansprüche 25 bis 27 umfasst, wobei die Transfektion *in vivo* oder ex *vivo* durchgeführt wird; oder
ii) transduziert durch einen viralen Vektor, der eine Expressionskassette umfasst, die die Nukleinsäure nach einem der Ansprüche 25 bis 27 umfasst, wobei die Transduktion *in vivo* oder ex *vivo* durchgeführt wird, wobei der virale Vektor vorzugsweise ein retroviraler oder lentiviraler Vektor ist;
und wobei die humanen Zellen ausgewählt sind aus der Liste bestehend aus autologen humanen T-Zellen, autologen humanen CD4+-Helfer-T-Zellen, autologen humanen CD8+-zytotoxischen T-Zellen, einem Gemisch aus autologen humanen CD4+-Helfer-T-Zellen und CD8+-zytotoxischen T-Zellen in einem beliebigen Verhältnis, allogenen humanen T-Zellen, allogenen humanen CD4+-Helfer-T-Zellen, allogenen humanen CD8+-zytotoxischen T-Zellen, einem Gemisch aus allogenen humanen CD4+-Helfer-T-Zellen und CD8+-zytotoxischen T-Zellen in beliebigem Verhältnis, autologen primären humanen natürlichen Killerzellen (NK), allogenen primären humanen natürlichen Killerzellen (NK), allogenen Zellen einer NK-92-Zelllinie, autologen humanen Monozyten, autologen humanen Makrophagen, allogenen humanen Monozyten, allogenen humanen Makrophagen.

30. Eine oder mehrere Populationen isolierter humaner Zellen nach Anspruch 29 zur Verwendung bei der Behandlung von Krebs.

## Revendications

1. Dérivé de chlorotoxine comprenant la séquence d'acides aminés de la séquence générale
X₀X₁CMPCX_{S1}X_{S2}X_{S3}DHX_{S4}X_{S5}ARRCX₂X₃CCGGYGX₄CFGYQCLCX₅X₆X₇X₈
(SEQ ID N° : 43)
dans laquelle
(i) le groupe X₀X₁ N-terminal est choisi dans le groupe constitué par AM, 0M ou 00 ;
(ii) le groupe X_{S1}X_{S2}X_{S3}X_{S4}X_{S5} de solubilité est choisi dans le groupe constitué par FTTQT, FTTES, SSSQT, SSSES, FSSQT, FSSES ou FSSQS ;
(iii) le groupe X₂X₃X₄ interne est choisi dans le groupe constitué par DKR, RDK, KDR, IKY, HKW, DRK, LKQ, KKK ; et
(iv) le groupe X₅X₆X₇X₈ C-terminal est choisi dans le groupe constitué par N000, R000, NR00, NRGO, NRGY, NRRR ou RRRR ;
où 0 désigne une position dans laquelle aucun acide aminé n'est présent ;
ledit dérivé de chlorotoxine présentant une liaison relative à la MMP-2 humaine qui est au moins 1,62 fois supérieure à celle de la chlorotoxine de type sauvage de SEQ ID N° : 1.

2. Dérivé de chlorotoxine selon la revendication 1 comprenant la séquence d'acides aminés de la séquence générale
X₀X₁CMPCFTTDHQTARRCX₂X₃CCGGYGX₄CFGYQCLCX₅X₆X₇X₈
(SEQ ID N° : 35)
dans laquelle
(i) le groupe X₀X₁ N-terminal est choisi dans le groupe constitué par AM, 0M ou 00 ;
(ii) le groupe X₂X₃X₄ interne est choisi dans le groupe constitué par DKR, RDK, KDR, IKY, HKW, DRK, LKQ, KKK ; et
(iii) le groupe X₅X₆X₇X₈ C-terminal est choisi dans le groupe constitué par N000, R000, NR00, NRG0 ou NRGY ;
où 0 désigne une position dans laquelle aucun acide aminé n'est présent.

3. Dérivé de chlorotoxine selon la revendication 1 ou 2, dans lequel le groupe X₂X₃X₄ interne est DKR.

4. Dérivé de chlorotoxine selon la revendication 1, dans lequel le dérivé de chlorotoxine étant choisi parmi les dérivés de chlorotoxine présentant l'une quelconque des séquences décrites de la SEQ ID N° : 3 à la SEQ ID N° : 18 et de la SEQ ID N° : 36 à la SEQ ID N° : 42.

5. Dérivé de chlorotoxine selon la revendication 1, dans lequel le dérivé de chlorotoxine présentant la séquence décrite en SEQ ID N° : 4 ou en SEQ ID N° : 42.

6. Dérivé de chlorotoxine selon l'une quelconque des revendications 1 à 5, dans lequel le dérivé de chlorotoxine étant cyclique.

7. Conjugué comprenant un dérivé de chlorotoxine selon l'une quelconque des revendications 1 à 6 et un groupe prosthétique.

8. Conjugué selon la revendication 7, dans lequel le groupe prosthétique est choisi parmi les agents suivants :
(i) les agents de visualisation, de préférence choisis parmi les marqueurs fluorescents, les radiomarqueurs, les marqueurs d'imagerie par résonance magnétique, et les agents permettant un marquage indirect par liaison de haute affinité à des molécules de marquage ;
(ii) les agents thérapeutiques, de préférence choisis parmi les agents chimiothérapeutiques, et les agents thérapeutiques biologiques ;
(iii) les agents de ciblage, de préférence choisis parmi les anticorps, les polypeptides, les polysaccharides et les acides nucléiques ;
(iv) les moitiés qui augmentent la demi-vie circulante, de préférence choisis parmi les moitiés peg, les moitiés glycosyle, les moitiés glycosylpeg et les moitiés permettant la cyclisation du dérivé de chlorotoxine selon l'une quelconque des revendications 1 à 6 par le biais d'une extension de lieur.

9. Conjugué selon l'une quelconque des revendications 7 ou 8, dans lequel le conjugué comprenant un ou plusieurs lieurs et éventuellement un ou plusieurs espaceurs entre le dérivé de chlorotoxine et le groupe prosthétique.

10. Conjugué selon la revendication 9, dans lequel le lieur est choisi dans le groupe des peptides, d'un lieur de disulfure de diméthyle, d'un lieur de glutaryle, des lieurs clivables par la cathepsine.

11. Paire théranostique de conjugués comprenant un premier conjugué et un second conjugué, dans laquelle ledit premier conjugué est un conjugué selon l'une quelconque des revendications 7 à 10 et ledit second conjugué est un conjugué selon l'une quelconque des revendications 7 à 10.

12. Paire théranostique selon la revendication 11, dans laquelle
a) la partie dérivé de chlorotoxine dudit premier conjugué et celle dudit second conjugué présentent la même séquence d'acides aminés et lesdits deux conjugués présentent des groupes prosthétiques différents ; ou
b) la partie dérivé de chlorotoxine dudit premier conjugué et celle dudit second conjugué présentent des séquences d'acides aminés différentes et ledit premier conjugué et ledit second conjugué présentent des groupes prosthétiques identiques ou différents.

13. Kit comprenant un conjugué selon l'une quelconques des revendications 7 à 10 et des instructions d'utilisation.

14. Kit comprenant la paire théranostique selon la revendication 11 ou 12 et des instructions d'utilisation.

15. Composition pharmaceutique comprenant un conjugué selon l'une quelconque des revendications 8 à 10, dans laquelle le groupe prosthétique du conjugué est un agent thérapeutique ou un agent de visualisation et la composition pharmaceutique comprend en outre un excipient pharmaceutiquement acceptable.

16. Conjugué selon l'une quelconque des revendications 7 à 10 destiné à être utilisé dans le traitement d'un cancer.

17. Conjugué selon l'une quelconque des revendications 7 à 10 destiné à être utilisé dans le diagnostic d'un cancer.

18. Conjugué selon l'une quelconque des revendications 7 à 10, dans lequel le groupe prosthétique est un agent de visualisation et est destiné à être utilisé dans la visualisation de tissus cancéreux.

19. Conjugué destiné à être utilisé selon la revendication 16 ou 17, dans lequel le cancer est choisi dans le groupe des cancers du sein, des cancers du col de l'utérus, des cancers du côlon, des épendymomes, des sarcomes d'Ewing, des gangliogliomes, des ganglioneuromes, des gliomes, des glioblastomes, des gliosarcomes, des médulloblastomes, des méningiomes, des neuroblastomes, des mélanomes (primaires et métastasiques), des cancers du pancréas, des phéochromocytomes, des cancers de la prostate, des schwannomes, des carcinomes pulmonaires à petites cellules ; plus préférentiellement, le cancer est choisi dans le groupe des cancers du sein, des glioblastomes, des mélanomes et des cancers du pancréas.

20. Conjugué destine à être utilisé selon la revendication 18, dans lequel le tissu cancéreux est choisi dans le groupe des cancers du sein, des cancers du col de l'utérus, des cancers du côlon, des épendymomes, des sarcomes d'Ewing, des gangliogliomes, des ganglioneuromes, des gliomes, des glioblastomes, des gliosarcomes, des médulloblastomes, des méningiomes, des neuroblastomes, des mélanomes (primaires et métastasiques), des cancers du pancréas, des phéochromocytomes, des cancers de la prostate, des schwannomes, des carcinomes pulmonaires à petites cellules ; plus préférentiellement, le tissu cancéreux est choisi dans le groupe des cancers du sein, des glioblastomes, des mélanomes et des cancers du pancréas.

21. Composition pharmaceutique selon la revendication 15 destinée à être utilisée dans le traitement d'un cancer, dans laquelle le groupe prosthétique du conjugué est un agent thérapeutique approprié pour le traitement du cancer.

22. Composition pharmaceutique destinée à être utilisée selon la revendication 21, dans laquelle le cancer est choisi dans le groupe des cancers du sein, des cancers du col de l'utérus, des cancers du côlon, des épendymomes, des sarcomes d'Ewing, des gangliogliomes, des ganglioneuromes, des gliomes, des glioblastomes, des gliosarcomes, des médulloblastomes, des méningiomes, des neuroblastomes, des mélanomes (primaires et métastasiques), des cancers du pancréas, des phéochromocytomes, des cancers de la prostate, des schwannomes, des carcinomes pulmonaires à petites cellules ; plus préférentiellement, le cancer est choisi dans le groupe des cancers du sein, des glioblastomes, des mélanomes et des cancers du pancréas.

23. Méthode de préparation de conjugués, comprenant les étapes de
a) fourniture d'un dérivé de chlorotoxine selon l'une quelconque des revendications 1 à 6 ;
b) liaison éventuelle d'un groupe lieur au dérivé de chlorotoxine de l'étape a),
c) liaison d'un groupe prosthétique à la partie lieur du composé formé à l'étape b) ou directement à la protéine de l'étape a) en l'absence de lieur.

24. Méthode de visualisation d'un tissu cancéreux, comprenant l'étape de mise en contact du tissu à examiner avec un conjugué selon l'une quelconque des revendications 7 à 10, dans laquelle le groupe prosthétique dudit conjugué est un agent de visualisation approprié pour la visualisation du tissu cancéreux.

25. Molécule d'acide nucléique codant un récepteur antigénique chimérique, dans laquelle le récepteur antigénique chimérique comprend :
a) un dérivé de chlorotoxine selon l'une quelconque des revendications 1 à 6 ;
b) éventuellement une région espaceur entre le dérivé de chlorotoxine et le domaine transmembranaire ;
c) un domaine transmembranaire ;
d) un ou deux domaines de costimulation ;
e) un domaine de signalisation ;
dans laquelle le dérivé de chlorotoxine permet au récepteur antigénique chimérique, lorsqu'il est exprimé à la surface d'un lymphocyte T, de diriger l'activité du lymphocyte T sur des cellules cancéreuses.

26. Molécule d'acide nucléique selon la revendication 25, dans laquelle le dérivé de chlorotoxine est choisi parmi les dérivés de chlorotoxine présentant l'une quelconque des séquences décrites de la SEQ ID N° : 3 à la SEQ ID N° : 18 et de la SEQ ID N° : 36 à la SEQ ID N° : 42, de préférence la SEQ ID N° : 4 ou la SEQ ID N° : 42.

27. Molécule d'acide nucléique selon la revendication 25 ou 26, dans laquelle
i) le domaine transmembranaire choisi dans le groupe constitué par un domaine transmembranaire de CD4 ou un variant de celui-ci, un domaine transmembranaire de CD8 ou un variant de celui-ci, un domaine transmembranaire de CD28 ou un variant de celui-ci, et un domaine transmembranaire de CD3 zêta ou un variant de celui-ci ;
ii) les un ou deux domaines de costimulation choisis dans le groupe constitué par un domaine de costimulation de CD28 ou un variant de celui-ci, un domaine de costimulation de 4-1BB ou un variant de celui-ci et un domaine de costimulation d'OX40 ou un variant de celui-ci ; et
iii) le domaine de signalisation est le domaine de signalisation de CD3 zêta ou un variant de celui-ci.

28. Vecteur comprenant la molécule d'acide nucléique selon l'une quelconque des revendications 25 à 27.

29. Population de cellules humaines isolées
i) transfectées par un vecteur d'ARN ou d'ADN comprenant une cassette d'expression comprenant l'acide nucléique selon l'une quelconque des revendications 25 à 27, dans laquelle la transfection est effectuée *in vivo* ou ex *vivo* ; ou
ii) transduites par un vecteur viral comprenant une cassette d'expression comprenant l'acide nucléique selon l'une quelconque des revendications 25 à 27, dans laquelle la transduction est effectuée *in vivo* ou ex *vivo,* dans laquelle le vecteur viral est de préférence un vecteur rétroviral ou lentiviral ;
et dans laquelle lesdites cellules humaines sont choisies dans la liste constituée par les lymphocytes T humains autologues, les lymphocytes T auxiliaires CD4+ humains autologues, les lymphocytes T cytotoxiques CD8+ humains autologues, un mélange de lymphocytes T auxiliaires CD4+ et de lymphocytes T cytotoxiques CD8+ humains autologues dans une proportion quelconque, les lymphocytes T humains allogéniques, les lymphocytes T auxiliaires CD4+ humains allogéniques, les lymphocytes T cytotoxiques CD8+ humains allogéniques, un mélange de lymphocytes T auxiliaires CD4+ et de lymphocytes T cytotoxiques CD8+ humains allogéniques dans un proportion quelconque, les lymphocytes tueurs naturels (NK) humains primaires autologues, les lymphocytes tueurs naturels (NK) humains primaires allogéniques, les lymphocytes allogéniques de la lignée cellulaire NK-92, les monocytes humains autologues, les macrophages humains autologues, les monocytes humains allogéniques, les macrophages humains allogéniques.

30. Une ou plusieurs populations de cellules humaines isolées selon la revendication 29 destinées à être utilisées dans le traitement d'un cancer.
